(19) Europäisches Patentamt
European Patent Office
Office européen des brevets

(11) Publication number: **0 106 489**
**B1**

(12) **EUROPEAN PATENT SPECIFICATION**

(45) Date of publication of patent specification: **27.07.88**

(21) Application number: **83305148.5**

(22) Date of filing: **06.09.83**

(51) Int. Cl.[4]: **C 07 D 401/04**, C 07 D 417/04, C 07 D 471/10, C 07 D 487/10, C 07 D 471/04, C 07 D 487/04, C 07 D 498/06, C 07 D 519/00, A 61 K 31/47, A 61 K 31/535, A 61 K 31/55 // (C07D487/10, 209:00, 209:00),(C07D471/04, 221:00, 221:00),(C07D487/04, 209:00, 209:00),(C07D498/06, 265:00, 221:00),(C07D519/00, 487:00, 471:00)

(54) **Antibacterial agents.**

(30) Priority: **09.09.82 US 416406**
**12.08.83 US 522275**

(43) Date of publication of application:
**25.04.84 Bulletin 84/17**

(45) Publication of the grant of the patent:
**27.07.88 Bulletin 88/30**

(84) Designated Contracting States:
**AT BE CH DE FR GB IT LI LU NL SE**

(56) References cited:
**EP-A-0 047 005**
**FR-A-2 138 003**
**FR-A-2 183 895**
**FR-A-2 500 833**
**GB-A- 956 253**
**US-A-3 590 036**
**US-A-3 876 650**
**US-A-4 341 784**

**JOURNAL OF MEDICINAL CHEMISTRY, vol. 23, no. 12, 1980, The American Chemical Society, Washington, US. H. KOGA et al.: "Structure-Activity Relationships of Antibacterial 6,7- and 7,8-Disubstituted 1-Alkyl-1.4-dihydro-4-oxoquinoline-3-carboxylic Acids1", pages 1358-1363**

**The file contains technical information submitted after the application was filed and not included in this specification**

(73) Proprietor: **WARNER-LAMBERT COMPANY**
**201 Tabor Road**
**Morris Plains New Jersey 07950 (US)**

(72) Inventor: **Culbertson, Townley P.**
**2996 Argonne**
**Ann Arbor Michigan 48103 (US)**
Inventor: **Mich, Thomas F.**
**915 Sunset Road**
**Ann Arbor Michigan 48103 (US)**
Inventor: **Domagala, John M.**
**776 Georgetown**
**Canton Michigan 48188 (US)**
Inventor: **Nichols, Jeffrey B.**
**4867 Lake Ridge Drive**
**Ypsilanti Michigan 48197 (US)**

(74) Representative: **Jones, Michael Raymond et al**
**HASELTINE LAKE & CO. Hazlitt House**
**28 Southampton Buildings Chancery Lane**
**London WC2A 1AT (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 106 489 B1

**Description**

BACKGROUND OF THE INVENTION

US Patent 4,341,784 discloses certain substituted 7-(3-amino-1-pyrrolidinyl)-1-ethyl-6-fluoro-1,4-dihydro-4-oxo-1,8-naphthyridine-3-carboxylic acids having the general formula:

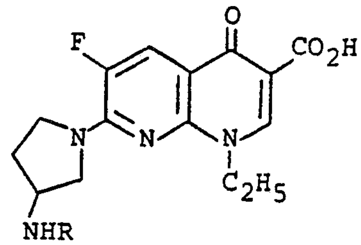

The compounds are stated to have antibacterial activity.

The Journal of Medicinal Chemistry, *23*, 1358 (1980) discloses certain substituted quinoline-3-carboxylic acids having the structural formula

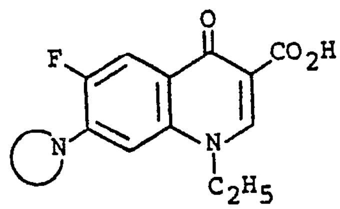

wherein

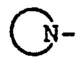

may be pyrrolidinyl. See also US Patent 4,146,719. The compounds are stated to have antibacterial activity.

European Patent Application 81 10 6747, Publication Number 047,005, published March 10, 1982, discloses certain benzoxazine derivatives having the structural formula

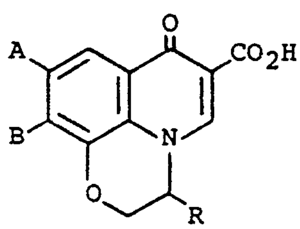

wherein A is halogen and B may be a cyclic amine substituent such as pyrrolidine, or piperidine.

Certain 7-heterocyclic substituted 1,8-naphthyridines are disclosed in Eur. J. Med. Chem. — Chimica Therapeutica, *29*, 27 (1977). US Patents 3,753,993 and 3,907,808 disclose certain 7-pyridylquinolines.

The references teach that these compounds possess antibacterial activity.

A first aspect of the present invention provides a compound having the structural formula I

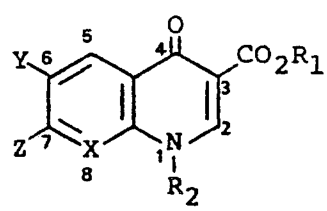

I

wherein Z is

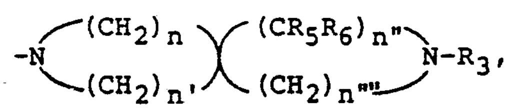

or

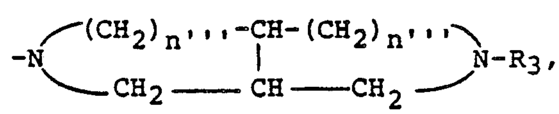

in which

Z′ is

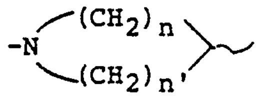 , 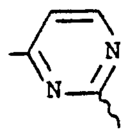 , 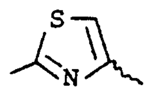 or 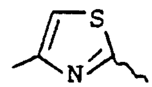 ;

X is CH, CCl, CF, C—OH, CO-alkyl having from one to three carbon atoms, or C—NH-alkyl having from one to three carbon atoms;

Y is hydrogen, fluorine, chlorine, or bromine;

n is 1, 2, 3 or 4;

n′ is 1, 2, 3 or 4 wherein n + n′ is a total of 2, 3, 4 or 5;

n″ is 0, 1 or 2;

n‴ is 1 or 2; and n⁗ is 0, 1 or 2;

$R_1$ is hydrogen, alkyl having from one to six carbon atoms or a cation;

$R_2$ is alkyl having from one to four carbon atoms, vinyl, haloalkyl, or hydroxyalkyl having from two to four carbon atoms, or cycloalkyl having three to six carbon atoms;

$R_3$ is hydrogen, alkyl having from one to four carbon atoms or cycloalkyl having three to six carbon atoms;

$R_4$ is hydrogen, alkyl from one to four carbon atoms, hydroxyalkyl having two to four carbon atoms, trifluoroethyl or $R_7CO$— wherein $R_7$ is alkyl having from one to four carbon atoms, or alkoxy having from one to four carbon atoms;

$R_5$ is hydrogen, or alkyl having from one to three carbon atoms; and

$R_6$ is hydrogen or alkyl having from one to three carbon atoms;

with the proviso that, when X is CH and Z′ is

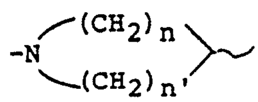

the sum of n + n′ is other than 4;

and the pharmaceutically acceptable acid addition or base salts thereof.

The significance of the symbol ~ is intended only to show point of attachment of the radical to other atoms of the remaining component of the molecule.

The preferred compounds of this invention are those wherein Z′ is

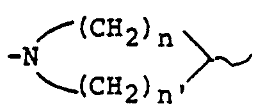 , 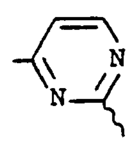 , or 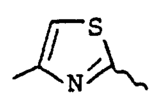 ;

also preferred compounds of this invention are those wherein Z is

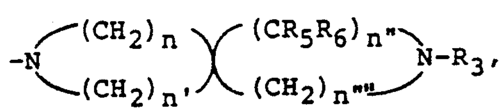

Other preferred compounds of this invention are those wherein Y is fluorine.

Other preferred compounds of this invention are those wherein X is CH or C—F.

Other preferred compounds of this invention are those wherein $R_1$ is hydrogen or a pharmaceutically acceptable base salt such as a metal or amine salt.

Other preferred compounds of this invention are those wherein $R_2$ is ethyl, vinyl, or 2-fluoroethyl.

Other preferred compounds of this invention are those wherein n″ is one, $R_3$ is hydrogen, methyl, or ethyl, $R_4$, $R_5$ and $R_6$ are hydrogen.

The most preferred compounds are those wherein X is CF, Z is

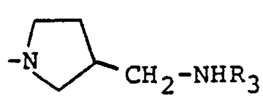 or 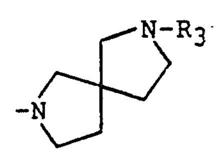

and $R_3$ is hydrogen, methyl or ethyl or a pharmaceutically acceptable acid addition or base salt thereof.

Particularly preferred species of the invention are the compounds having the names:

7-[3-(aminoethyl)-1-pyrrolidinyl]-1-ethyl-6,8-difluoro-1,4-dihydro-4-oxo-3-quinolinecarboxylic acid;

7-[3-(aminoethyl)-1-pyrrolidinyl]-6,8-difluoro-1-(2-fluoroethyl)-1,4-dihydro-4-oxo-3-quinolinecarboxylic acid;

7-[3-(aminomethyl)-1-pyrrolidinyl]-6,8-difluoro-1-ethenyl-1,4-dihydro-4-oxo-3-quinolinecarboxylic acid;

1-ethyl-7-[3-[(ethylamino)methyl]-1-pyrrolidinyl]-6,8-difluoro-1,4-dihydro-4-oxo-3-quinolinecarboxylic acid;

7-[3-[(ethylamino)methyl]-1-pyrrolidinyl]-6,8-difluoro-1-(2-fluoroethyl)-1,4-dihydro-4-oxo-3-quinolinecarboxylic acid;

7-[3-[(ethylamino)methyl-1-pyrrolidinyl]-6,8-difluoro-1-ethenyl-1,4-dihydro-4-oxo-3-quinolinecarboxylic acid;

1-ethyl-7-[3-[[(1-methylethyl)amino]methyl]-1-pyrrolidinyl]-6,8-difluoro-1,4-dihydro-4-oxo-3-quinolinecarboxylic acid;

1-ethyl-6,8-difluoro-1,4-dihydro-7-(7-ethyl-2,7-diazaspiro[4.4]non-2-yl)-4-oxo-3-quinolinecarboxylic acid;

1-ethyl-6,8-difluoro-1,4-dihydro-7-(7-methyl-2,7-diazaspiro[4.4]non-2-yl)-4-oxo-3-quinolinecarboxylic acid;

7-(3-amino-1-pyrrolidinyl)-1-ethyl-6,8-difluoro-1,4-dihydro-4-oxo-3-quinolinecarboxylic acid;

and the pharmaceutically acceptable acid addition or base salts thereof.

A second aspect of the present invention is directed to the following process for preparing compounds of the formula

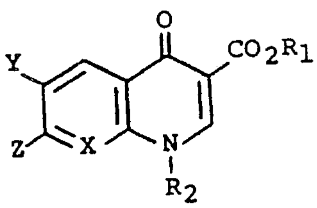

III

wherein $R_1$, $R_2$, X, Y and Z are as defined for formula I, which process comprises reacting a compound having the following structural formulae

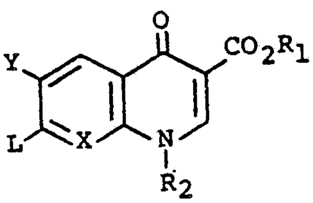

IV

with an amine corresponding to the group Z wherein Z is the compound having the structural formula

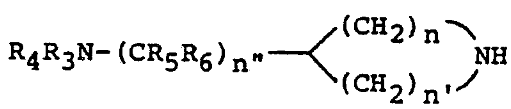

,

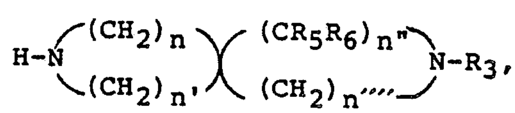

VIa

VIb

or

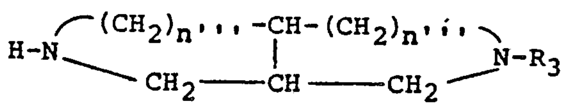

VIc

wherein all of the above terms are as defined in formula I and L is a leaving group which is preferably fluorine or chlorine.

The invention also includes a pharmaceutical composition which comprises an antibacterially effective amount of a compound having structural formula I and the pharmaceuticaly acceptable salts thereof in combination with a pharmaceutically acceptable carrier.

The compounds and compositions of the present invention are useful for treating bacterial infections in a mammal by administering an antibacterially effective amount of the above defined pharmaceutical composition to a mammal in need thereof.

DESCRIPTION OF THE PREFERRED EMBODIMENTS

The compounds of the invention having the structural formula III may be readily prepared by treating a corresponding compound having the structural formula IV with the desired cyclic amine VIa, VIb or VIc. For purposes of this reaction, the alkylamine substituent of Compound VIa, VIb or VIc may, if desired, be protected by a group which renders it substantially inert to the reaction conditions. Thus, for example, protecting groups such as the following may be utilized:

carboxylic acyl groups such as formyl, acetyl, trifluoroacetyl;

alkoxycarbonyl groups such as ethoxycarbonyl, *t*-butoxycarbonyl, β,β,β-trichloroethoxycarbonyl, β-iodoethoxycarbonyl;

aryloxycarbonyl groups such as benzyloxycarbonyl, *p*-methoxybenzyloxycarbonyl, phenoxycarbonyl;

silyl groups such as trimethylsilyl;

and groups such as trityl, tetrahydropyranyl, vinyloxycarbonyl, *o*-nitrophenylsulfenyl, diphenylphosphinyl, *p*-toluenesulfonyl, and benzyl, may all be utilized. The protecting group may be removed, after the reaction between Compound IV and Compound VIa, VIb or VIc if desired, by procedures known to those skilled in the art. For example, the ethoxycarbonyl group may be removed by acid or base hydrolysis and the trityl group may be removed by hydrogenolysis.

The reaction between the compound of structural formula IV and a suitably protected compound of formula VIa, VIb or VIc may be performed with or without a solvent, preferably at elevated temperature for a sufficient time so that the reaction is substantially complete. The reaction is preferably carried out in the presence of an acid acceptor such as an alkali metal or alkaline earth metal carbonate or bicarbonate, a tertiary amine such as triethylamine, pyridine, or picoline. Alternatively an excess of the compound of formula VI may be utilized as the acid acceptor.

Convenient solvents for this reaction are non-reactive solvents such as acetonitrile, tetrahydrofuran, ethanol, chloroform, dimethylsulfoxide, dimethylformamide, pyridine, picoline, water, and the like. Solvent mixtures may also be utilized.

Convenient reaction temperatures are in the range of from about 20° to about 150°C; higher temperatures usually require shorter reaction times.

The removal of the protecting group $R_4$ may be accomplished either before or after isolating the product, III. Alternatively, the protecting group $R_4$ need not be removed.

The starting compounds having structural formulae IV are known in the art. Thus the following compounds are disclosed in the noted reference:

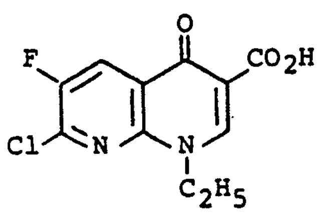

European Patent Application 0027752

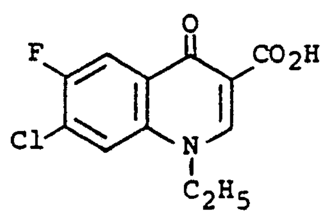

J. Med. Chem., *23,* 1358 (1980)

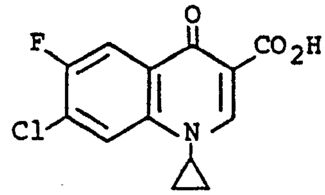

European Patent Application 0078362

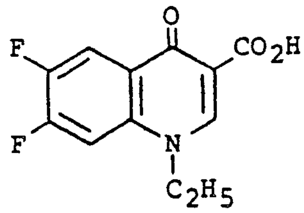

European Patent 0 000 203 (1979)

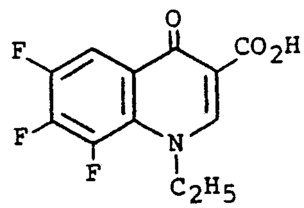

British Patent 2,057,440

The compounds of the invention having structural formula VIa, VIb or VIc are either known compounds or they may be prepared from known starting materials by standard procedures or by variations thereof. For example, 3-pyrrolidinemethanamines having the structural formula D

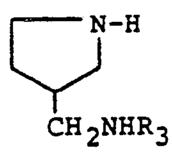

D

may be readily prepared from the known starting material methyl 5-oxo-1-(phenylmethyl)-3-pyrrolidinecarboxylate, A, (J. Org. Chem., *26*, 1519 (1961)] by the following reaction sequence.

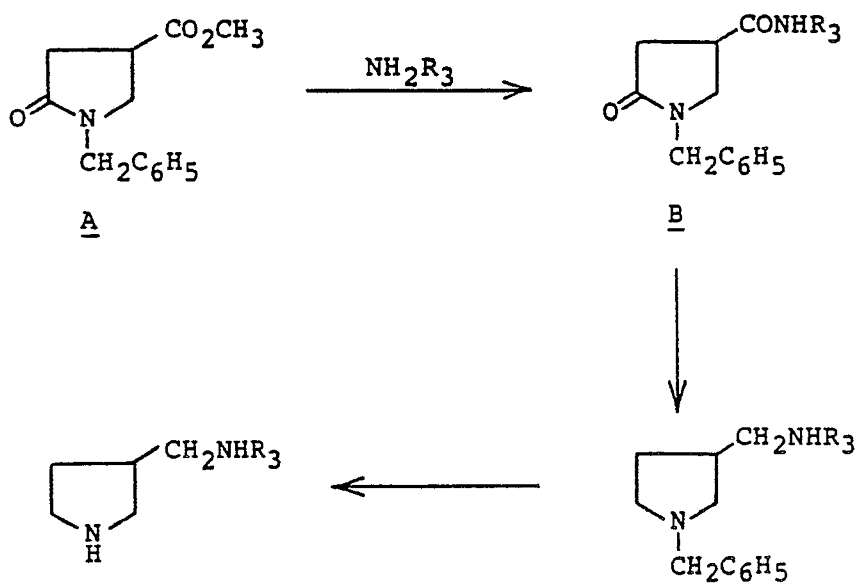


A B C D

The compound wherein $R_3$ is hydrogen, namely 3-pyrrolidinemethanamine, has been reported in J. Org. Chem., *26*, 4955 (1961).

Thus Compound A may be converted to the corresponding amide B by treatment with $R_3NH_2$; for example, a saturated solution of ethylamine in an akanol such as methyl alcohol may be utilized. The diamide B may next be reduced to produce the corresponding diamine C. This reduction may be carried out using lithium aluminum hydride, for example, in a convenient solvent such as tetrahydrofuran. Compound

C may next be debenzylated, for example using hydrogen and 20% palladium on carbon catalyst to produce the diamine D. Alternatively, when R = H in C, the primary amine function may be protected with a group $R_4$ as defined, hereinabove. For example, the primary amine function may be acylated with an acyl halide such as acetyl chloride by well known procedures. The primary amine function of C may also be converted to a carbamate ester such as the ethyl ester by treatment with ethyl chloroformate in the presence of a strong base as 1,8-diazabicyclo[5.4.0]undec-7-ene in a convenient solvent such as methylene chloride. The benzyl group may next be removed, for example as described above for Compound C, thereby producing Compound D where R is $-CO_2Et$, which after conversion to a compound of the type VIa or VIb may be reacted with a compound having the structural formula IV to thereby produce a corresponding compound having the structural formulae I. The $-CO_2Et$ group may be removed by standard procedures.

Likewise spiroamino compounds represented by structural formula VIb may be readily prepared from the known starting material 3-ethoxycarbonyl-5-oxo-3-pyrrolidineacetic acid ethyl ester [J. Org. Chem., 46, 2757 (1981)] by the following reaction sequence.

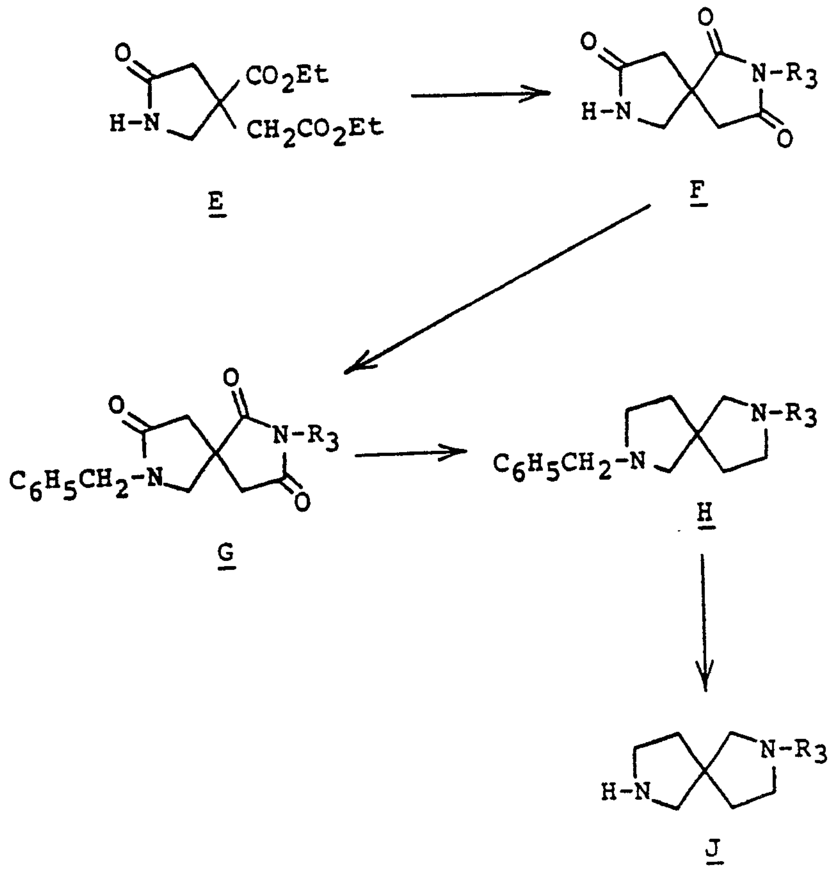

E F G H J

The compound 2,7-diazaspiro[4.4]nonane where $R_3$ is H is described in the above reference. Thus Compound E may be converted to the corresponding amide F by treatment with $R_3NH_2$, for example, methyl amine in water followed by benzylation which may be carried out with sodium hydride and benzyl chloride to give G. Reduction to the diamine H may be accomplished with lithium aluminum hydride. Subsequent debenzylation, for example, with hydrogen and 20% palladium on carbon catalyst produces the diamine J.

A third aspect of the present invention is directed to a process for preparing compounds of the invention of the formula

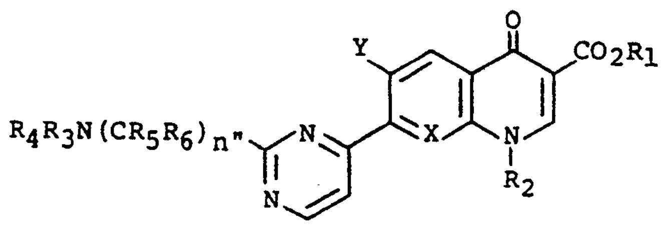

Ib

which comprises reacting a compound having structural formula

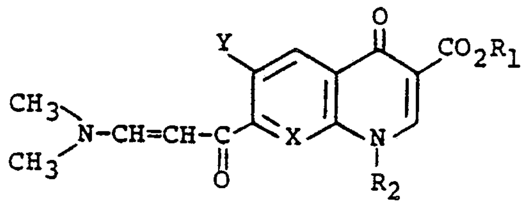

VII

with an amidine having structural formula

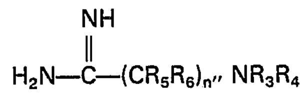

VIII

wherein X, Y, $R_1$—$R_6$, and n″ are defined for formula I.

A fourth aspect of the present invention relates to a process for preparing a compound having structural formula

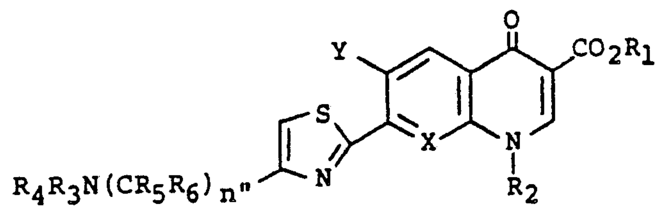

Ic

which comprises: a) reacting a compound having the structural formula

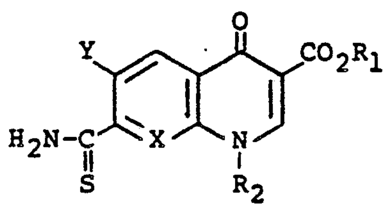

IX

with a dihalo ketone of the formula

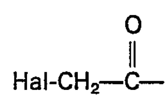

$(CR_5R_6)_{n''}$-Hal wherein X, Y, R, $R_2$, $R_5$ and $R_6$ are defined for formula I; n″ is 1 or 2, and Hal is any convenient halogen, preferably chlorine, to produce the halomethylthiazole;

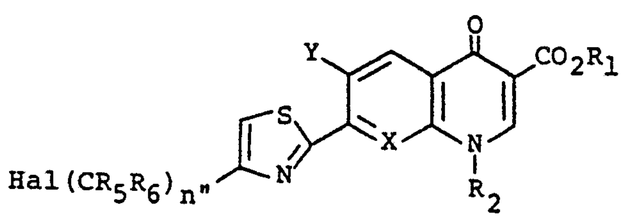

X

b) displacing the halogen atom of X with an amino group of the formula $R_3R_4N$— or with azide ion;

c) reducing the azide group to produce the compound Ib wherein $R_3$ and $R_4$ are hydrogen; and d) optionally alkylating the primary amino function to produce compounds Ic wherein $R_3$ and/or $R_4$ are alkyl of from one to three carbon atoms.

A fifth aspect of the present invention is for preparing compounds of the structural formula

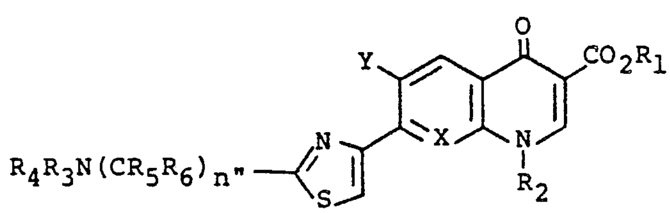

Id

which comprises reacting a compound having the structural formula

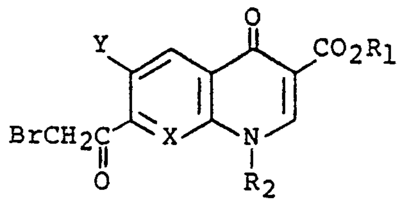

XI

with a thioamide having structural formula XII

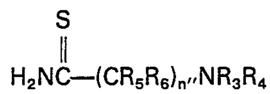

XII

wherein X, Y, $R_1$—$R_6$, and n″ are defined as in formula I.

The compounds of the invention having structural formula I wherein Z is

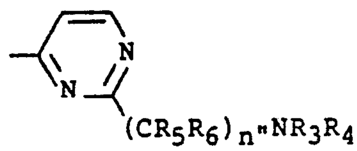

may be prepared from the correspondingly substituted methyl ketone

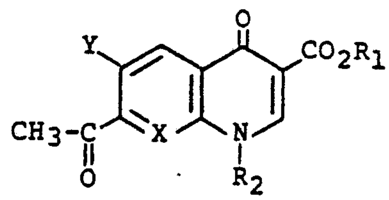

XIII

wherein X, Y, $R_1$—$R_6$, and n″ are defined above.

Thus compound XIII may be treated with *t*-butoxy-bis-dimethylaminomethane to give a compound having the structural formula XIV

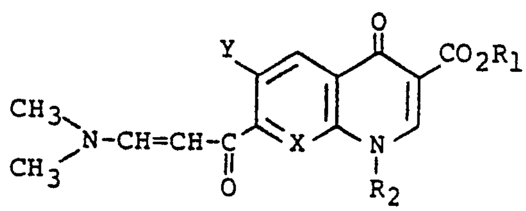

XIV

This reaction may be carried out by mixing the two reactants in a nonreactive solvent such as dimethylformamide at an elevated temperature.

Compound XIV may then be reacted with any of a variety of substitutd amidines having the structural formula

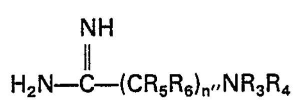

XV

wherein $R_1$—$R_6$ and n″ are defined as in formula I to produce the correspondingly substituted pyrimidines. This reaction may be carried out by mixing the two reactants in an inert solvent such as *t*-butanol in the presence of a base such as potassium *t*-butoxide at elevated temperature. Variations in these reactions, for example to maximize a particular yield is within the skill of the art.

The compounds of the invention having structural formula I wherein Z is

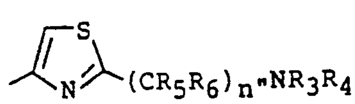

may also be prepared from the correspondingly substituted methyl ketone XIII. Thus, compound XIII is first brominated to produce the α-bromoketone, XVI

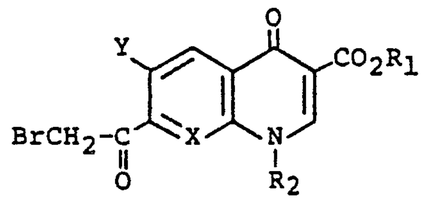

XVI

This reaction may be carried out by treating compound XIII with potassium bromate and hydrobromic acid in a nonreactive solvent such as acetic acid. Compound XVI is then reacted with any of a variety of thioamides having structural formula

$$H_2N\overset{\overset{S}{\|}}{C}—(CR_5R_6)_{n''}NR_3R_4 \qquad \text{XVII}$$

wherein $R_1$—$R_6$ and n'' are defined above to produce the correspondingly substituted 2-(substituted)-thiazol-4-yl compounds. This reaction may be carried out by mixing Compounds XVI and XVII in a non-reactive solvent such as ethanol or dimethylformamide usually at room temperature. Variations in these reactions, for example, to maximize a particular yield is within the skill of the art.

The compounds of the invention having structural formula I wherein Z is

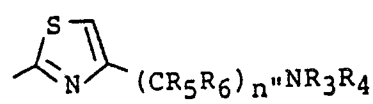

may be prepared from the correspondingly substituted thioamide

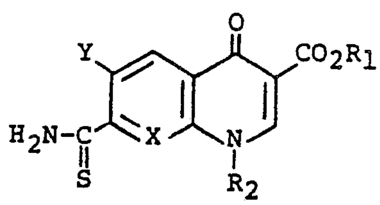

XVIII

by first reacting XVIII with a dihaloketone

$$Hal\text{-}CH_2—\overset{\overset{O}{\|}}{C}—CH_2\text{-}Hal$$

to produce the halomethylthiazole

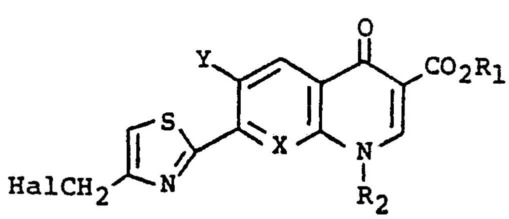

XIX

In the preferred procedure, 1,3-dichloroacetone is mixed with XVIII in a nonreactive solvent such as N,N-dimethylformamide and heated to about 100°C for approximately four hours. The product XIX wherein Hal representes chlorine, may be isolated and purified by standard procedures. This compound is then treated with azide ion, preferably sodium azide, in a convenient nonreactive solvent such as N,N-dimethylformamide by heating at about 100°C for about four hours. The thus produced azido compound

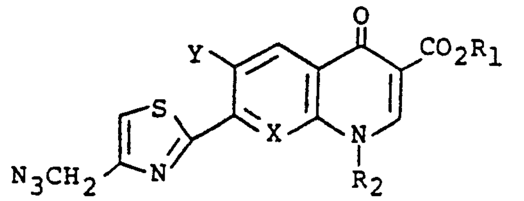

XX

may then be reduced to produce the corresponding primary amine Ic where $R_3$, $R_4$, $R_5$ and $R_6$ are hydrogen. In the preferred procedure, the azide is dissolved in acetic acid and treated with hydrogen gas at atmospheric pressure using a 10% palladium on carbon catalyst. The corresponding secondary and tertiary amines may be produced by reacting XIX with the appropriate amine. When Z is VIa, VIb or VIc in formula I, compounds where $R_2$ is cycloalkyl may be prepared by methods outlined in United States Patent 4,359,578 or by the method described in European Patent Publication Number 00078362.

The compounds of the invention display antibacterial activity when tested by the microtitration dilution method as described in Heifetz, et al, Antimicr. Agents & Chemoth, *6,* 124 (1974).

By use of the above referenced method, the followed minimum inhibitory concentration values (MICs in µg/ml) were obtained for representative compounds of the invention and the prior art compound 7-(3-amino-1-pyrrolidinyl)-1-ethyl-6-fluoro-1,4-dihydro-4-oxo-1,8-naphthyridine-3-carboxylic acid designated as ** in the table.

IN VITRO ANTIBACTERIAL ACTIVITY

Minimal Inhibitory Concentration

MIC (μg/ml)

| Organisms | ** | Compound Ex. 1 | Compound Ex. 2 | Compound Ex. 3 | Compound Ex. 4 | Compound Ex. 4a |
|---|---|---|---|---|---|---|
| Enterobacter cloacae MA 2646 | 0.05 | 1.6 | 1.6 | 0.4 | < 0.1 | 0.2 |
| Escherichia coli Vogel | 0.013 | 0.4 | 0.2 | 0.1 | < 0.1 | 0.2 |
| Klebsiella pneumoniae MGH-2 | 0.05 | 1.6 | 1.6 | 0.8 | 0.4 | 0.8 |
| Proteus rettgeri M 1771 | 0.05 | 3.1 | 6.3 | 1.6 | 0.4 | 0.8 |
| Pseudomonas aeruginosa UI-18 | 0.05 | 0.8 | 1.6 | 3.1 | 0.2 | 0.8 |
| Staphylococcus aureus H 228 | 0.8 | 0.8 | 0.8 | 0.4 | < 0.1 | 0.4 |
| Staphylococcus aureus UC-76 | 0.025 | 0.05 | 0.013 | 0.05 | < 0.1 | ≤ 0.1 |
| Streptococcus faecalis MGH-2 | 1.6 | 0.4 | 1.6 | 0.2 | < 0.1 | < 0.1 |
| Streptococcus pneumoniae SV-1 | 0.4 | 0.2 | 0.4 | 0.2 | < 0.1 | 0.4 |
| Streptococcus pyogenes C-203 | 0.4 | 0.05 | 0.1 | 0.1 | < 0.1 | < 0.1 |

IN VITRO ANTIBACTERIAL ACTIVITY

Minimal Inhibitory Concentration

MIC (μg/ml)

| Organisms | Compound Ex. 5 | Compound Ex. 5a | Compound Ex. 6 | Compound Ex. 6a | Compound Ex. 6b |
|---|---|---|---|---|---|
| Enterobacter cloacae MA 2646 | 0.1 | 0.2 | 0.4 | 3.1 | 0.8 |
| Escherichia coli Vogel | 0.05 | < 0.1 | 0.2 | 1.6 | 0.2 |
| Klebsiella pneumoniae MGH-2 | 0.1 | 0.4 | 0.4 | 6.3 | 0.4 |
| Proteus rettgeri M 1771 | 0.2 | 0.8 | 0.8 | 6.3 | 0.8 |
| Pseudomonas aeruginosa UI-18 | 0.8 | 1.6 | 1.6 | 12.5 | 1.6 |
| Staphylococcus aureus H 228 | 0.013 | 0.4 | 0.4 | < 0.1 | < 0.1 |
| Staphylococcus aureus UC-76 | 0.003 | < 0.1 | < 0.1 | < 0.1 | < 0.1 |
| Streptococcus faecalis MGH-2 | 0.013 | 0.2 | < 0.1 | 0.8 | < 0.1 |
| Streptococcus pneumoniae SV-1 | 0.013 | < 0.1 | < 0.1 | 1.6 | < 0.1 |
| Streptococcus pyogenes C-203 | 0.013 | < 0.1 | < 0.1 | 0.8 | < 0.1 |

IN VITRO ANTIBACTERIAL ACTIVITY

Minimal Inhibitory Concentration

MIC (μg/ml)

| Organisms | Compound Ex. 9 | Compound Ex. 10 | Compound Ex. 11 | Compound Ex. 11a | Compound Ex. 12 |
|---|---|---|---|---|---|
| Enterobacter cloacae MA 2646 | 0.2 | 0.2 | 0.1 | 6.3 | 0.4 |
| Escherichia coli Vogel | < 0.1 | 0.2 | 0.006 | 3.1 | 0.2 |
| Klebsiella pneumoniae MGH-2 | 0.2 | 0.8 | 0.1 | 6.3 | 0.2 |
| Proteus rettgeri M 1771 | 1.6 | 3.1 | 0.8 | 25 | 1.6 |
| Pseudomonas aeruginosa UI-18 | 0.8 | 3.1 | 0.8 | 12.5 | 3.1 |
| Staphylococcus aureus H 228 | < 0.1 | 0.8 | 0.1 | 3.1 | 0.2 |
| Staphylococcus aureus UC-76 | < 0.1 | < 0.1 | 0.006 | 0.4 | 0.1 |
| Streptococcus faecalis MGH-2 | 0.2 | 0.4 | 0.1 | 3.1 | 0.2 |
| Streptococcus pneumoniae SV-1 | 0.2 | 0.4 | 0.4 | 6.3 | 0.8 |
| Streptococcus pyogenes C-203 | < 0.1 | 0.4 | 0.2 | 3.1 | 0.4 |

IN VITRO ANTIBACTERIAL ACTIVITY

Minimal Inhibitory Concentration

MIC (µg/ml)

| Organisms | Compound Ex. 13 | Compound Ex. 14 | Compound Ex. 15 | Compound Ex. 16 | Compound Ex. 17 | Compound Ex. 18 |
|---|---|---|---|---|---|---|
| Enterobacter cloacae MA 2646 | 0.1 | 0.4 | 1.6 | 6.3 | 1.6 | 0.1 |
| Escherichia coli Vogel | 0.05 | 0.2 | 0.2 | 1.6 | 0.8 | 0.013 |
| Klebsiella pneumoniae MGH-2 | 0.05 | 0.4 | 0.4 | 3.1 | 0.8 | 0.1 |
| Proteus rettgeri M 1771 | 0.2 | 0.4 | 1.6 | 12.5 | 3.1 | 0.2 |
| Pseudomonas aeruginosa UI-18 | 1.6 | 6.3 | 12.5 | 100 | 25 | 0.8 |
| Staphylococcus aureus H 228 | 0.1 | 0.4 | 0.05 | 0.8 | 1.6 | 0.1 |
| Staphylococcus aureus UC-76 | 0.013 | 0.1 | 0.025 | 0.2 | 0.2 | 0.025 |
| Streptococcus faecalis MGH-2 | 0.2 | 0.8 | 0.4 | 0.8 | 1.6 | 0.2 |
| Streptococcus pneumoniae SV-1 | 0.4 | 0.8 | 0.4 | 0.4 | 3.1 | 1.6 |
| Streptococcus pyogenes C-203 | 3.1 | 1.6 | 0.8 | 0.4 | 6.3 | 3.1 |

IN VITRO ANTIBACTERIAL ACTIVITY

Minimal Inhibitory Concentration

MIC (µg/ml)

| Organisms | Compound Ex. 18a | Compound Ex. 18b | Compound Ex. 18c | Compound Ex. 18d | Compound Ex. 18e |
|---|---|---|---|---|---|
| Enterobacter cloacae MA 2646 | 0.2 | 0.1 | 0.8 | 0.2 | 0.2 |
| Escherichia coli Vogel | 0.013 | 0.05 | 0.4 | 0.05 | 0.2 |
| Klebsiella pneumoniae MGH-2 | 0.1 | 0.1 | 0.4 | 0.1 | 0.8 |
| Proteus rettgeri M 1771 | 0.8 | 0.4 | 0.4 | 0.2 | 0.8 |
| Pseudomonas aeruginosa UI-18 | 3.1 | 6.3 | 6.3 | 3.1 | 6.3 |
| Staphylococcus aureus H 228 | 0.2 | 0.1 | 0.8 | 0.2 | 0.4 |
| Staphylococcus aureus UC-76 | 0.05 | 0.05 | $< 0.1$ | 0.025 | $< 0.05$ |
| Streptococcus faecalis MGH-2 | 0.8 | 0.4 | 0.4 | 0.2 | 0.4 |
| Streptococcus pneumoniae SV-1 | 3.1 | 1.6 | 6.3 | 3.1 | 1.6 |
| Streptococcus pyogenes C-203 | 3.1 | 1.6 | 3.1 | 1.6 | 3.1 |

IN VITRO ANTIBACTERIAL ACTIVITY

Minimal Inhibitory Concentration

MIC (µg/ml)

| Organisms | Compound Ex. 19 | Compound Ex. 20 | Compound Ex. 20a | Compound Ex. 21 | Compound Ex. 21a | Compound Ex. 22 |
|---|---|---|---|---|---|---|
| Enterobacter cloacae MA 2646 | 0.1 | 0.8 | 0.8 | 3.1 | 3.1 | 0.8 |
| Escherichia coli Vogel | 0.025 | 0.2 | < 0.1 | 0.8 | < 0.1 | 0.2 |
| Klebsiella pneumoniae MGH-2 | 0.2 | 0.8 | 0.4 | 3.1 | 0.4 | 0.8 |
| Proteus rettgeri M 1771 | 0.2 | 6.3 | 1.6 | 3.1 | 6.3 | 1.6 |
| Pseudomonas aeruginosa UI-18 | 3.1 | 50 | 6.3 | 12.5 | 25 | 1.6 |
| Staphylococcus aureus H 228 | 0.4 | 0.4 | 0.8 | < 0.05 | < 0.1 | 1.6 |
| Staphylococcus aureus UC-76 | 0.1 | 0.2 | < 0.1 | < 0.05 | < 0.1 | 0.05 |
| Streptococcus faecalis MGH-2 | 0.4 | 3.1 | 1.6 | 0.2 | < 0.1 | 0.8 |
| Streptococcus pneumoniae SV-1 | 3.1 | 6.3 | 6.3 | 0.8 | 0.8 | 0.2 |
| Streptococcus pyogenes C-203 | 3.1 | 6.3 | 6.3 | 1.6 | 1.6 | 0.2 |

IN VITRO ANTIBACTERIAL ACTIVITY

Minimal Inhibitory Concentration

MIC (μg/ml)

| Organisms | Compound Ex. 23 | Compound Ex. 24 | Compound Ex. 25 | Compound Ex. 25a | Compound Ex. 25b | Compound Ex. 25c |
|---|---|---|---|---|---|---|
| Enterobacter cloacae MA 2646 | 0.8 | 0.8 | 0.2 | 0.4 | 0.4 | 0.8 |
| Escherichia coli Vogel | 0.2 | 0.4 | 0.2 | < 0.1 | 0.05 | 0.2 |
| Klebsiella pneumoniae MGH-2 | 0.8 | 1.6 | 0.4 | 0.2 | 0.2 | 0.4 |
| Proteus rettgeri M 1771 | 0.8 | 1.6 | 0.4 | 0.2 | 0.1 | 0.8 |
| Pseudomonas aeruginosa UI-18 | 6.3 | 12.5 | 1.6 | 3.1 | 0.8 | 1.6 |
| Staphylococcus aureus H 228 | 0.8 | 0.8 | 0.4 | 0.8 | 6.3 | 50 |
| Staphylococcus aureus UC-76 | 0.025 | 0.2 | 0.025 | < 0.1 | 0.4 | 1.6 |
| Streptococcus faecalis MGH-2 | 0.8 | 1.6 | 0.2 | 0.8 | 1.6 | 12.5 |
| Streptococcus pneumoniae SV-1 | 0.8 | 0.8 | 3.1 | 12.5 | 1.6 | 25 |
| Streptococcus pyogenes C-203 | 0.4 | 1.6 | 0.2 | 12.5 | 0.8 | 25 |

The compounds of the invention are capable of forming both pharmaceutically acceptable acid addition and/or base salts. Base salts are formed with metals or amines, such as alkali and alkaline earth metals or organic amines. Examples of metals used as cations are sodium, potassium, magnesium and calcium. Examples of suitable amines are N,N'-dibenzylethylenediamine, chloroprocains, choline, diethanolamine, ethylenediamine, N-methylglucamine, and procaine.

Pharmaceutically acceptable acid addition salts are formed with organic and inorganic acids.

Examples of suitable acids for salt formation are hydrochloric, sulfuric, phosphoric, acetic, citric, oxalic, malonic, salicylic, malic, gluconic, fumaric, succinic, ascorbic, maleic and methanesulfonic. The salts are prepared by contacting the free base form with a sufficient amount of the desired acid to produce either a mono or di, etc. salt in the conventional manner. The free base forms may be regenerated by treating the salt form with a base. For example, dilute solutions of aqueous base may be utilized. Dilute aqueous sodium hydroxide, potassium carbonate, ammonia, and sodium bicarbonate solutions are suitable for this purpose. The free base forms differ from their respective salt forms somewhat in certain physical properties such as solubility in polar solvents, but the salts are otherwise equivalent to their respective free base forms for purposes of the invention. Use of excess base where R' is hydrogen gives the corresponding basic salt.

The compounds of the invention can exist in unsolvated as well as solvated forms, including hydrated forms. In general, the solvated forms, including hydrated forms are equivalent to the unsolvated forms for purposes of the invention.

The alkyl groups contemplated by the invention comprise both straight and branched carbon chains of from one to about three carbon atoms except when specifically stated to be greater than three carbon atoms. Representative of such groups are methyl, ethyl, propyl and isopropyl.

The cycloalkyl groups contemplated by the invention comprise those having three to six carbon atoms such as cyclopropyl, cyclobutyl, cyclopentyl, and cyclohexyl.

The alkoxy groups contemplated by the invention comprise both straight and branched carbon chains of from one to about six carbon atoms unless otherwise specified. Representative of such groups are methoxy, ethoxy, propoxy, *i*-propoxy, *t*-butoxy and hexoxy.

The term haloalkyl, is intended to include halogen substituted straight and branched carbon chains of from two to four carbon atoms. Those skilled in the art will recognize that the halogen substituent may not be present on the α-carbon atom of the chain. Representative of such groups are β-fluoroethyl, β-chloroethyl, β,β-dichloroethyl, β-chloropropyl, β-chloro-2-propyl, γ-iodobutyl, and the like.

The term halogen is intended to include fluorine, chlorine, bromine, and iodine unless otherwise specified.

Certain compounds of the invention may exist in optically active forms. The pure D isomer, pure L isomer as well as mixtures thereof; including the racemic mixtures, are contemplated by the invention. Additional asymmetric carbon atoms may be present in a substituent such as an alkyl group. All such isomers as well as mixtures thereof are intended to be included in the invention.

The compounds of the invention can be prepared and administered in a wide variety of oral and parenteral dosage forms. It will be obvious to those skilled in the art that the following dosage forms may comprise as the active component, either a compound of formula I or a corresponding pharmaceutically acceptable salt of a compound of formula I.

For preparing pharmaceutical compositions from the compounds described by this invention, inert pharmaceutically acceptable carriers can be either solid or liquid. Solid form preparations include powders, tablets, dispersable granules, capsules, cachets, and suppositories. A solid carrier can be one or more substances which may also act as diluents, flavoring agents, solubilizers, lubricants, suspending agents, binders, or tablets disintegrating agents; it can also be an encapsulating material. In powders, the carrier is a finely divided solid which is in admixture with the finely divided active compound. In the tablet the active compound is mixed with carrier having the necessary binding properties in suitable proportions and compacted in the shape and size desired. The powders and tablets preferably contain from 5 or 10 to about 70 percent of the active ingredient. Suitable solid carriers are magnesium carbonate, magnesium stearate, talc, sugar, lactose, pectin, dextrin, starch, gelatin, tragacanth, methyl cellulose, sodium carboxymethyl cellulose, a low melting wax, cocoa butter, and the like. The term "preparation" is intended to include the formulation of the active compound with encapsulating material as carrier providing a capsule in which the active component (with or without other carriers) is surrounded by carrier, which is thus in association with it. Similarly, cachets are included. Tablets, powders, cachets, and capsules can be used as solid dosage forms suitable for oral administration.

Liquid form preparations include solutions suspensions and emulsions. As an example may be mentioned water or water-propylene glycol solutions for parenteral injection. Such solutions are prepared so as to be acceptable to biological systems (isotonicity, pH, etc). Liquid preparations can also be formulated in solution in aqueous polyethylene glycol solution. Aqueous solutions suitable for oral use can be prepared by dissolving the active component in water and adding suitable colorants, flavors, stabilizing, and thickening agents as desired. Aqueous suspension suitable for oral use can be made by dispersing the finely divided active component in water with viscous material, i.e., natural or synthetic gums, resins, methyl cellulose, sodium carboxymethyl cellulose, and other well-known suspending agents.

Preferably, the pharmaceutical preparation is in unit dosage form. In such form, the preparation is

subdivided into unit doses containing appropriate quantities of the active component. The unit dosage form can be a packaged preparation, the package containing discrete quantities of preparation, for example, packeted tablets, capsules, and powders in vials or ampoules. The unit dosage form can also be a capsule, cachet, or tablet itself or it can be the appropriate number of any of these packaged forms.

The quantity of active compound in a unit dose of preparation may be varied or adjusted from 1 mg to 100 mg according to the particular application and the potency of the active ingredient.

In therapeutic use as agents for treating bacterial infections the compounds utilized in the pharmaceutical method of this invention are administered at the initial dosage of about 3 mg to about 40 mg per kilogram daily. A daily dose range of about 6 mg to about 14 mg per kilogram is preferred. The dosages, however, may be varied depending upon the requirements of the patient, the severity of the condition being treated, and the compound being employed. Determination of the proper dosage for a particular situation is within the skill of the art. Generally, treatment is initiated with smaller dosages which are less than the optimum dose of the compound. Thereafter, the dosage is increased by small increments until the optimum effect under the circumstances is reached. For convenience, the total daily dosage may be divided and administered in portions during the day if desired.

The following nonlimiting examples illustrate the inventors' preferred methods for preparing the compounds of the invention.

## Example 1

7-[3-(Aminomethyl)-1-pyrrolidinyl]-1-ethyl-6-fluoro-1,4-dihydro-4-oxo-3-quinolinecarboxylic acid

A mixture of 1.00 g (3.95 mmole) of 6,7-difluoro-1-ethyl-1,4-dihydro-4-oxo-3-quinolinecarboxylic acid, 40 ml acetonitrile and 1.28 g (12.75 mmole) of 3-pyrrolidinemethanamine were stirred at room temperature overnight. The reaction was filtered, the precipitate washed with 10 ml of water, ethanol/ether (1:1) and finally with ether until dry to give 1.13 g of 7-[3-(aminomethyl)-1-pyrrodinyl]-1-ethyl-6-fluoro-1,4-dihydro-4-oxo-3-quinolinecarboxylic acid, mp 234—236°C.

| | | | |
|---|---|---|---|
| Analysis was calculated for $C_{17}H_{20}FN_3O_3\cdot.3H_2O$: | C, 60.27; | H, 6.13; | N, 12.40 |
| Found: | C, 60.63; | H, 5.85; | N, 12.01. |

## Example 2

1-Ethyl-6-fluoro-1,4-dihydro-7-[3-[(methylamino)methyl]-1-pyrrolidinyl]-4-oxo-3-quinolinecarboxylic acid

A mixture of 1.00 g (3.95 mmole) 1-ethyl-6,7-difluoro-1,4-dihydro-4-oxo-3-quinolinecarboxylic acid, 10 ml N,N-dimethylformamide, 75 ml acetonitrile, and 1.35 g (11.85 mmole) of N-methyl-3-pyrrolidinemethanamine were refluxed overnight. The reaction was cooled to room temperature and filtered. The precipitate was washed with water, ethanol/ether (1:3), and finally with ether until dry to give 1.17 g of 1-ethyl-6-fluoro-1,4-dihydro-7-[3-[(methylamino)methyl]-1-pyrrolidinyl]-4-oxo-3-quinolinecarboxylic acid, mp 247—250°C.

| | | | |
|---|---|---|---|
| Analysis for $C_{18}H_{22}FN_3O_3\cdot\frac{1}{2}H_2O$: | C, 60.66; | H, 6.50; | N, 11.79 |
| Found: | C, 60.69; | H, 6.30; | N, 11.82. |

## Example 3

1-Ethyl-7-[3-[(ethylamino)methyl]-1-pyrrolidinyl]-6-fluoro-1,4-dihydro-4-oxo-3-quinolinecarboxylic acid

A mixture of 2.70 g (10.0 mmole) of 7-chloro-1-ethyl-6-fluoro-1,4-dihydro-4-oxo-3-quinolinecarboxylic acid, 60 ml of β-picoline and 3.85 g (30.0 mmole) of N-ethyl-3-pyrrolidinemethanamine were refluxed overnight. Reaction mixture was cooled to room temperature, 100 ml concentrated ammonium hydroxide added, and the solvents removed at reduced pressure. A solution of 200 ml dichloromethane/ether (1:3) was added. The resulting precipitate was filtered, washed with ethanol/ether (1:3) and finally with ether until dry to give 1.87 g of 1-ethyl-7-[3-[(ethylamino)methyl]-1-pyrrolidinyl]-6-fluoro-1,4-dihydro-4-oxo-3-quinolinecarboxylic acid, mp 248—252°C.

| | | | |
|---|---|---|---|
| Analysis calculated for $C_{19}H_{24}FN_3O_3\cdot1.48H_2O$: | C, 58.81; | H, 7.00; | N, 10.83 |
| Found: | C, 58.70; | H, 6.53; | N, 10.85. |

## Example 4

7-[3-(Aminomethyl)-1-pyrrolidinyl]-1-ethyl-6,8-difluoro-1,4-dihydro-4-oxo-3-quinolinecarboxylic acid

A mixture of 0.50 g (1.84 mmole) of 1-ethyl-6,7,8-trifluoro-1,4-dihydro-4-oxo-3-quinolinecarboxylic acid, 5 ml of acetonitrile, 0.28 g (1.84 mmole) of 1,8-diazabicyclo[5.4.0]undec-7-ene and 0.19 g (1.94 mmole) of 3-pyrrolidinemethanamine was refluxed for one hour; then stirred at room temperature overnight. The reaction was filtered and the precipitate washed with ethyl ether to give 0.56 g of 7-[3-(aminomethyl)-1-pyrrolidinyl]-1-ethyl-6,8-difluoro-1,4-dihydro-4-oxo-3-quinolinecarboxylic acid, mp 219—221°C.

The following compounds were also prepared by the above procedure:

7-[3-(aminomethyl)-1-pyrrolidinyl]-6,8-difluoro-1-(2-fluoroethyl)-1,4-dihydro-4-oxo-3-quinolinecarboxylic acid, mp 224—226°C (10a) and 7-[3-(aminomethyl)-1-pyrrolidinyl]-6,8-difluoro-1-ethenyl-1,4-dihydro-4-oxo-3-quinolinecarboxylic acid, mp 204—208°C (10b).

Example 5

1-Ethyl-7-[3-[(ethylamino)methyl]-1-pyrrolidinyl]-6,8-difluoro-1,4-dihydro-4-oxo-3-quinolinecarboxylic acid

A mixture of 22.50 g (83.03 mmole) 1-ethyl-1,4-dihydro-4-oxo-6,7,8-trifluoro-3-quinolinecarboxylic acid, 225 ml acetonitrile, 11.25 g (87.08 mmole) N-ethyl-3-pyrrolidinemethanamine and 12.6 g (83.03 mmole) 1,8-diazabicyclo[5.4.0]undec-7-ene was refluxed 1 hour then was stirred at room temperature overnight. The solid was filtered and washed with ether to give 26.33 g of 1-ethyl-7-[3-[(ethylamino)methyl]-1-pyrrolidinyl]-6,8-difluoro-1,4-dihydro-4-oxo-3-quinolinecarboxylic acid, mp 208—210°C.

Analysis calculated for $C_{19}H_{23}F_2N_3O_3$: C, 60.15; H, 6.11; N, 11.08
Found: C, 59.85; H, 6.17; N, 11.08.

The following compounds were also prepared by the above procedure:

7-[3-[(ethylamino)methyl]-1-pyrrolidinyl]-6,8-difluoro-1-(2-fluoroethyl)-1,4-dihydro-4-oxo-3-quinolinecarboxylic acid, mp 221—223°C (11a), 7-[3-[(ethylamino)methyl]-1-pyrrolidinyl-6,8-difluoro-1-ethenyl-1,4-dihydro-4-oxo-3-quinolinecarboxylic acid, mp 217—220°C (11b), and 1-methyl-7-[3-[(ethylamino)methyl]-1-pyrrolidinyl]-6,8-difluoro-1,4-dihydro-4-oxo-3-quinolinecarboxylic acid, mp 238—240°C (11c).

Example 6

1-Ethyl-6,8-difluoro-1,4-dihydro-7-[3-[[(2-hydroxyethyl)amino]methyl]-1-pyrrolidinyl]-4-oxo-3-quinoline carboxylic acid

A mixture of 0.50 g (1.84 mmole) of 1-ethyl-6,7,8-trifluoro-4-oxo-quinoline-3-carboxylic acid, 5 ml acetonitrile, 0.28 g (1.84 mmole) 1,8-diazabicyclo[5.4.0]undec-7-ene and .28 g (1.94 mmole) of 2-[(3-pyrrolidinylmethyl)amino]ethanol was refluxed one hour and then stirred at room temperature overnight. The reaction was filtered and the precipitate washed with ether until dry to give 0.58 g of 1-ethyl-6,8-difluoro-1,4-dihydro-7-[3-[[(2-hydroxyethyl)amino]methyl]-1-pyrrolidinyl]-4-oxo-3-quinoline carboxylic acid, mp 215—216°C.

Using N - (2,2,2 - trifluoroethyl) - 3 - pyrrolidinemethanamine in the above procedure gave 1 - ethyl - 6,8 - difluoro - 1,4 - dihydro - 4 - oxo - 7 - [3 - [[(2,2,2 - trifluoroethyl)amino]methyl] - 1 - pyrrolidinyl] - 3 - quinolinecarboxylic acid, mp 182—183°C (12a).

Using three equivalents of N-(2-propyl)-3-pyrrolidinemethanamine and no 1,8-diazabicyclo[5.4.0]undec-7-ene in the above procedure gave 1-ethyl-7-[3-[[(1-methylethyl)amino]methyl]-1-pyrrolidinyl]-6,8-difluoro-1,4-dihydro-4-oxo-3-quinolinecarboxylic acid, mp 198—200°C (12b).

Example 7

7-(3-Amino-1-pyrrolidinyl)-1-ethyl-6,8-difluoro-1,4-dihydro-4-oxo-3-quinolinecarboxylic acid

A suspension of 0.81 g (3.0 mmole) of 1-ethyl-1,4-dihydro-6,7,8-trifluoro-4-oxo-3-quinolinecarboxylic acid, 1.28 g (10 mmole) of 3-acetylaminopyrrolidine [United States Patent 4,341,784] and 1.5 g (15 mmole) of triethylamine in 50 ml of acetonitrile was refluxed for four hours. The solvent was removed in vacuo and the residue was dissolved in 50 ml of 6.0 M hydrochloric acid/ethanol (1:1). The mixture was refluxed for four hours and the ethanol was removed in vacuo. The residue was diluted to 100 ml with water and adjusted to pH 7.3 with 1.0*N* sodium hydroxide. After cooling to 5°C, the precipitate was removed by filtration, washed successively with water, ethanol, ether, and dried in vacuo to give 0.6 g of 7-(3-amino-1-pyrrolidinyl)-1-ethyl-6,8-difluoro-1,4-dihydro-4-oxo-3-quinolinecarboxylic acid, mp 240—242°C.

Example 8

A general procedure for the compounds listed below is as follows: A mixture of 1 g of the appropriate halogenated quinoline carboxylic acid, 10 ml of an appropriate solvent (acetonitrile or β-picoline), one equivalent of 1,8-diazabicyclo[5.4.0]unde-7-ene and 1.05 equivalents of the amine are refluxed for a given amount of time, then stirred at room temperature overnight. The product is filtered off and washed with ethyl ether until dry.

The requisite amines may also be prepared according to the methods described in J. Heterocyclic Chem., *20*, 321, 439 (1983).

1-ethyl-6-fluoro-1,4-dihydro-7-(5-methyloctahydropyrrolo-[3,4-*c*]pyrrol-2-yl)-4-oxo-3-quinolinecarboxylic acid, (21c).

1-ethyl-6-fluoro-1,4-dihydro-7-(octahydropyrrolo[3,4-*c*]-pyrrol-2-yl)-4-oxo-3-quinolinecarboxylic acid, (21d).

1-ethyl-6-fluoro-1,4-dihydro-7-(octahydro-1*H*-pyrrolo[3,4-*c*]pyridin-2-yl)-4-oxo-3-quinolinecarboxylic acid, (21e).

1-ethyl-6,8-difluoro-1,4-dihydro-7-(5-methyloctahydropyrrolo[3,4-*c*]pyrrol-2-yl)-4-oxo-3-quinolinecarboxylic acid, mp 213—214°C (21f).

1-ethyl-6,8-difluoro-1,4-dihydro-7-(octahydropyrrolo[3,4-*c*]pyrrol-2-yl)-4-oxo-3-quinolinecarboxylic acid, (21g).

1-ethyl-6,8-difluoro-1,4-dihydro-7-(octahydro-1*H*-pyrrolo[3,4-*c*]pyridin-2-yl)-4-oxo-3-quinolinecarboxylic acid (21h).

Example 9

1-Ethyl-6,8-difluoro-1,4-dihydro-7-(2,7-diazaspiro[4.4]non-2-yl)-4-oxo-3-quinolinecarboxylic acid

A suspension of 0.81 g (3.0 mmol) 1-ethyl-6,7,8-trifluoro-4-oxo-1,4-dihydroquinoline-3-carboxylic acid in 40 ml acetonitrile was treated with 0.80 g (6.3 mmol) 2,7-diazaspiro[4.4]nonane [J. Org. Chem. *46*, 2757 (1981)] and the mixture stirred two days at room temperature, refluxed 1.5 hours, cooled, and filtered to afford 1.17 g of the title compound, mp 234—240°C (dec).

| | | | |
|---|---|---|---|
| Analysis calculated for $C_{19}H_{21}N_3F_2O_3$: | C, 60.47; | H, 5.61; | N, 11.13 |
| Found: | C, 60.17; | H, 5.46; | N, 11.11. |

Example 10

1-Ethyl-6-fluoro-1,4-dihydro-7-(7-methyl-2,7-diazaspiro[4.4]-non-2-yl)-4-oxo-3-quinolinecarboxylic acid

A suspension of 0.65 g (3.05 mmol) 2-methyl-2,7-diazaspiro[4.4]nonane dihydrochloride in 40 ml acetonitrile was treated with 1.33 g (9.0 mmol) 1,8-diazabicyclo[5.4.0]undec-7-ene and 0.76 g (3.0 mmol) 1-ethyl-6,7-difluoro-1,4-dihydro-4-oxo-3-quinolinecarboxylic acid was added. The mixture was refluxed overnight and the hot solution was filtered. After crystallizing at room temperature the product was filtered and washed with acetonitrile to afford 0.72 g of the title compound, mp 239—241°C (dec).

| | | | |
|---|---|---|---|
| Analysis calculated for $C_{20}H_{24}N_3FO_3$: | C, 64.33; | H, 6.48; | N, 11.25 |
| Found: | C, 64.25; | H, 6.50; | N, 11.27. |

Example 11

1-Ethyl-6,8-difluoro-1,4-dihydro-7-(7-methyl-2,7-diazaspiro[4.4]non-2-yl)-4-oxo-3-quinolinecarboxylic acid

A suspension of 0.64 g (3.0 mmol) 2-methyl-2,7-diazaspiro[4.4]nonane dihydrochloride in 40 ml acetonitrile was treated with 1.33 g (9.0 mmol) 1,8-diazabicyclo[5.4.0]undec-7-ene and 0.81 g (3.0 mmol) 1-ethyl-6,7,8-trifluoro-1,4-dihydro-4-oxo-3-quinolinecarboxylic acid was added. The mixture was stirred 45 minutes at room temperature, refluxed 1.5 hours, and stirred at room temperature overnight. The precipitate was filtered and washed with acetonitrile and ether to afford 0.87 g of the title compound, mp 229—231°C (dec).

| | | | |
|---|---|---|---|
| Analysis calculated for $C_{20}H_{23}N_3F_2O_3$: | C, 61.37; | H, 5.92; | N, 10.74 |
| Found: | C, 61.20; | H, 5.88; | N, 10.75. |

Using the above procedure with 2,8-diazaspiro[5.5]undecane dihydrochloride gave 1-ethyl-6,8-difluoro-1,4-dihydro-7-(2,8-diazaspiro[5.5]undec-2-yl)-4-oxo-3-quinolinecarboxylic acid, mp 267—268°C dec. (26a).

Example 12

1-Ethyl-6,8-difluoro-1,4-dihydro-7-(7-ethyl-2,7-diazaspiro[4.4]non-2-yl)-4-oxo-3-quinolinecarboxylic acid

The title compound was prepared according to example 11 by reacting 1-ethyl-6,7,8-trifluoro-4-oxo-1,4-dihydroquinoline-3-carboxylic acid with 2-ethyl-2,7-diazaspiro[4.4]nonane dihydrochloride, mp 199—202°C (dec).

| | | | |
|---|---|---|---|
| Analysis calculated for $C_{21}H_{25}N_3F_2O_3$: | C, 62.21; | H, 6.22; | N, 10.36 |
| Found: | C, 62.24; | H, 6.15; | N, 10.36. |

Example 13

7-[2-(Aminomethyl)-4-thiazolyl]-1-ethyl-6-fluoro-1,4-dihydro-4-oxo-3-quinolinecarboxylic acid

A suspension of 0.59 g (1.54 mmol) ethyl 7-bromoacetyl-1-ethyl-6-fluoro-1,4-dihydro-4-oxo-3-quinolinecarboxylate in 4 ml N,N-dimethylformamide was treated with 0.22 g (1.67 mmol) acetamidothioacetamide [prepared according to J. Am. Chem. Soc. *51*, 1817 (1929)]. After stirring three hours the mixture was treated with 0.22 ml (1.58 mmol) triethylamine, stirred one hour more and poured into 40 ml ice water. The precipitate was filtered, washed with water and dried to afford 0.45 g crude solid. Chromatography on a column of 20 g silica gel with chloroform-methanol (9:1) and crystallization from ethanol gave 0.37 g ethyl 7-[2(acetamidomethyl)-4-thiazolyl]-1-ethyl-6-fluoro-1,4-dihydro-4-oxo-3-quinolinecarboxylate, mp 230—233°C (dec).

A solution of 0.32 g (0.76 mmol) ethyl 7-[2-(acetamidomethyl)-4-thiazolyl]-1-ethyl-6-fluoro-1,4-dihydro-4-oxo-3-quinolinecarboxylate in 3 ml 6N hydrochloric acid was stirred at reflux three hours. The mixture was evaporated to dryness and the resulting solid was suspended in 5 ml water and dissolved by addition of 1N sodium hydroxide to pH 11. After filtration the product was precipitated by addition of 1N hydrochloric acid to pH 6.2, filtered, washed with water and dried to afford 0.22 g 7-[2-aminomethyl)-4-thiazolyl]-1-ethyl-6-fluoro-1,4-dihydro-4-oxo-3-quinolinecarboxylic acid, mp 249—254°C (dec).

Example 14

7-[2-(Methylaminomethyl)-4-thiazolyl]-1-ethyl-6-fluoro-1,4-dihydro-4-oxo-3-quinolinecarboxylic acid

According to example 13, reacting 7-bromoacetyl-1-ethyl-6-fluoro-1,4-dihydro-4-oxo-3-quinolinecarboxylic acid ethyl ester with 2-(N-acetyl-N-methylamino)thioacetamide in ethanol gave 1-ethyl-6-fluoro-7-[2-(N-acetyl-N-methylaminomethyl)-4-thiazolyl]-1,4-dihydro-4-oxo-3-quinolinecarboxylic acid ethyl ester, mp 173—175°C which was then hydrolyzed in refluxing 6N hydrochloric acid to give the title compound, mp 230—234°C (dec).

Analysis calculated for $C_{17}H_{16}N_3FO_3S$: C, 56.50; H, 4.46; N, 11.63
Found: C, 56.26; H, 4.56; N, 11.40.

Example 15

7-[2-Ethylaminomethyl)-4-thiazolyl]-1-ethyl-6-fluoro-1,4-dihydro-4-oxo-3-quinolinecarboxylic acid hydrochloride

According to example 13, reacting 7-bromoacetyl-1-ethyl-6-fluoro-1,4-dihydro-4-oxo-3-quinolinecarboxylic acid ethyl ester with 2-(N-acetyl-N-ethylamino)thioacetamide in ethanol gave 1-ethyl-7-[2-[(N-acetyl-N-ethylamino)methyl]-4-thiazolyl]-6-fluoro-1,4-dihydro-4-oxo-3-quinolinecarboxylic acid ethyl ester, mp 160—161°C which was then hydrolyzed in refluxing 6N hydrochloric acid to afford the title compound, mp 289—300°C (dec).

Analysis calculated for $C_{18}H_{19}N_3ClFO_3S \cdot 0.3H_2O$: C, 51.81; H, 4.73; N, 10.07; Cl, 8.50
Found: C, 51.81; H, 4.79; N, 10.09; CL, 8.49.

Example 16

1-Ethyl-6-fluoro-1,4-dihydro-7-[2-[[(2-hydroxyethyl)amino]methyl]-4-thiazolyl]-4-oxo-3-quinolinecarboxylic acid hydrochloride

According to example 13, by reacting 7 - bromoacetyl - 1 - ethyl - 6 - fluoro - 1,4 - dihydro - 4 - oxo - 3 - quinolinecarboxylic acid ethyl ester with 2 - [N - (2 - acetoxyethyl) - N - acetylamino]thioacetamide in ethanol gave 1 - ethyl - 6 - fluoro - 1,4 - dihydro - 7 - [2 - [[N - (2 - acetoxyethyl) - N - acetylamino]methyl] - 4 - thiazolyl] - 4 - oxo - 3 - quinolinecarboxylic acid ethyl ester, mp 148—152°C, resolidified and melted 164—165°C, which was hydrolyzed with refluxing 6N hydrochloric acid to afford the titled compound, mp 290°C (dec).

Analysis calculated for $C_{18}H_{17}N_3FClO_4S$: C, 50.76; H, 4.02; N, 9.87
Found: C, 50.36; H, 4.50; N, 9.66.

Example 17

7-[2-(1-aminoethyl)-4-thiazolyl]-1-ethyl-6-fluoro-1,4-dihydro-4-oxo-3-quinolinecarboxylic acid

According to example 13, reacting 7-bromoacetyl-1-ethyl-6-fluoro-1,4-dihydro-4-oxo-3-quinolinecarboxylic acid ethyl ester with 2-(acetylamino)thiopropionamide in ethanol gave 7-[2-[1-(N-acetylamino)-ethyl]-4-thiazolyl]-1-ethyl-6-fluoro-1,4-dihydro-4-oxo-3-quinolinecarboxylic acid ethyl ester, mp 236—238°C, which was hydrolyzed with refluxing 6N hydrochloric acid to afford the title compound, mp 246—250°C (dec).

Analysis calculated for $C_{17}H_{16}N_3FO_3S \cdot 0.2H_2O$: C, 55.94; H, 4.53; N, 11.51
Found: C, 55.92; H, 4.51; N, 11.68.

Example 18

7-[2-(aminomethyl)-4-thiazolyl]-1-ethyl-6,8-difluoro-1,4-dihydro-4-oxo-3-quinolinecarboxylic acid

To 800 mg (1.99 mmol) of the 7-bromoacetyl-1-ethyl-6,8-difluoro-1,4-dihydro-4-oxo-3-quinolinecarboxylic acid in a mixture of N,N-dimethylformamide and ethanol was added 446 mg (1.0 eq) of 2-(N-benzyloxycarbonyl)aminothioacetamide. The mixture was stirred for 72 hours at room temperature. The mixture was added to water and ice and the solids were filtered to give 0.92 g of the 1-ethyl-6,8-difluoro-1,4-dihydro-4-oxo-7-[2-[[(phenylmethoxy)carbonyl]aminomethyl]-4-thiazolyl]-3-quinolinecarboxylic acid, mp 185—190°C. Without further purification, the solids were treated with hydrobromic acid in acetic acid overnight and poured into ether:ethyl acetate. Filtration gave a solid that was dissolved in aqueous ammonia at pH 10.8. Concentration of this mixture to one third volume and filtration gave 0.46 g of 7-[2-(aminomethyl)-4-thiazolyl]-1-ethyl-6,8-difluoro-1,4-dihydro-4-oxo-3-quinolinecarboxylic acid, mp 199—203°C.

In similar fashion the following compounds are prepared:

1-Ethyl-6,8-difluoro-1,4-dihydro-7-[2-[(methylamino)methyl]-4-thiazolyl]-4-oxo-3-quinolinecarboxylic acid, mp 172—174°C (18a);

7-[2-[(ethylamino)methyl]-4-thiazolyl]-1-ethyl-6,8-difluoro-1,4-dihydro-4-oxo-3-quinolinecarboxylic acid, mp 155—157°C (18b);

7-[2-(aminomethyl)-4-thiazolyl]-6,8-difluoro-1-(2-fluoroethyl)-1,4-dihydro-4-oxo-3-quinolinecarboxylic acid, mp 216—218°C (18c);

6,8-difluoro-1-(2-fluoroethyl)-1,4-dihydro-7-[2[(methylamino)methyl]-4-thiazolyl]-4-oxo-3-quinolinecarboxylic acid, mp 214—215°C (18d);

7-[2-[(ethylamino)methyl]-4-thiazolyl]-6,8-difluoro-1-(2-fluoroethyl)-1,4-dihydro-4-oxo-3-quinolinecarboxylic acid, mp 197—201°C (18e).

Example 19

7-[2-(Aminomethyl)-4-thiazolyl]-1-ethyl-6-fluoro-1,4-dihydro-8-methylamino-4-oxo-3-quinolinecarboxylic acid

To 500 mg (1.37 mmol) of the 7-[2-(aminomethyl)-4-thiazolyl]-1-ethyl-6,8-difluoro-1,4-dihydro-4-oxo-2-quinolinecarboxylic acid was added 15 ml of 40% aqueous methylamine. The mixture was heated at 50°C

for 72 hours. The solution was taken to pH 6.5 and was filtered to give 350 mg of 7-[2-(aminomethyl)-4-thiazolyl]-1-ethyl-6-fluoro-1,4-dihydro-8-methylamino-4-oxo-3-quinolinecarboxylic acid, mp 194—195°C.

Example 20

1-Ethyl-6-fluoro-1,4-dihydro-8-methoxy-7-[2-[(methylamino)methyl]-4-thiazoly]-4-oxo-3-quinolinecarboxylic acid

To 500 mg (1.32 mmol) of 1-ethyl-6,8-difluoro-1,4-dihydro-7-[2-[(methylamino)methyl]-4-thiazoyl]-4-oxo-3-quinolinecarboxylic acid in 30 ml of dry methanol was added 300 mg (2 eq) of potassium *t*-butoxide. After 24 hours at reflux, the mixture was cooled, diluted with water, brought to pH 6.0, and filtered to give 310 mg of 1-ethyl-6-fluoro-1,4-dihydro-8-methoxy-7-[2-[(methylamino)methyl]-4-thiazoly]-4-oxo-3-quinolinecarboxylic acid, mp 164—166°C.

In a similar manner 7-[2-(aminomethyl)-4-thiazolyl]-1-ethyl-6-fluoro-1,4-dihydro-8-methoxy-4-oxo-3-quinolinecarboxylic acid, mp 172—175°C, was prepared; (37a).

Example 21

1-Ethyl-6-fluoro-1,4-dihydro-8-hydroxy-7-[2-[(methylamino)methyl]-4-thiazolyl]-4-oxo-3-quinolinecarboxylic acid

To 700 mg (1.79 mmol) of the 1-ethyl-6-fluoro-1,4-dihydro-8-methoxy-7-[2-[(methylamino)methyl]-4-thiazolyl]-4-oxo-3-quinolinecarboxylic acid was added 8 ml of hydrobromic acid in acetic acid and the mixture was heated to 70°C. After four hours 8 ml more hydrobromic acid was added and the mixture stirred overnight. The solids were collected, dissolved in aqueous ammonia and concentrated to one fourth volume. Filtration gave 580 mg of 1-ethyl-6-fluoro-1,4-dihydro-8-hydroxy-7-[2-[(methylamino)methyl]-4-thiazolyl]-4-oxo-3-quinolinecarboxylic acid, mp 242—246°C.

In similar manner 7-[2-(aminomethyl)-4-thiazolyl]-1-ethyl-6-fluoro-1,4-dihydro-8-hydroxy-4-oxo-3-quinoline carboxylic acid, mp 275—277°C, was prepared (21a).

Example 22

7-[4-(Aminomethyl)-2-thiazolyl]-1-ethyl-6-fluoro-1,4-dihydro-4-oxo-3-quinolinecarboxylic acid

A solution of 0.16 g (0.5 mmol) ethyl-1-ethyl-6-fluoro-7-thiocarbamoyl-1,4-dihydro-4-oxo-3-quinolinecarboxylate and 0.32 g (2.5 mmol) 1,3-dichloroacetone in 3 ml N,N-dimethylformamide was heated 3.5 hours on a steam bath. Dilution of the cooled reaction mixture with ethyl acetate afforded 0.12 g product which was recrystallized from chloroform:ethyl acetate to give 0.08 g of ethyl 7-[4-(chloromethyl)-2-thiazolyl]-1-ethyl-6-fluoro-1,4-dihydro-4-oxo-3-quinolinecarboxylate, mp 214—215°C (dec).

A mixture of 1.10 g (2.78 mmol) ethyl 7-[4-(chloromethyl)-2-thiazolyl]-1-ethyl-6-fluoro-1,4-dihydro-4-oxo-3-quinolinecarboxylate and 0.50 g (7.6 mmol) sodium azide in 50 ml N,N-dimethylformamide was heated on a steam bath four hours. After evaporation to near dryness 50 ml water was added to afford 1.01 g product was was crystallized from ethanol to give 0.91 g ethyl 7-[4-(azidomethyl)-2-thiazolyl]-1-ethyl-6-fluoro-1,4-dihydro-4-oxo-3-quinolinecarboxylate, mp 192—194°C (dec).

A solution of 0.87 g (2.17 mmol) ethyl 7-[4-(azidomethyl)-2-thiazolyl]-1-ethyl-6-fluoro-1,4-dihydro-4-oxo-3-quinolinecarboxylate in 125 ml acetic acid was stirred with 0.10 g 10% palladium on carbon catalyst and hydrogen gas bubbled through for 1.5 hours. After filtration, evaporation of solvent and trituration with ether gave 0.77 g of ethyl 7-[4-(aminomethyl)-2-thiazolyl]-1-ethyl-6-fluoro-1,4-dihydro-4-oxo-3-quinolinecarboxylate.

A solution of 0.70 g (1.87 mmol) ethyl 7-[4-(aminomethyl)-2-thiazolyl]-1-ethyl-6-fluoro-1,4-dihydro-4-oxo-3-quinolinecarboxylate in 15 ml 6N hydrochloric acid was heated on a steam bath 2.25 hours. After addition of 15 ml water the mixture was cooled to 0°C and filtered. The collected solid was suspended in 8 ml water, dissolved at pH 11 with 2N sodium hydroxide and reprecipitated by addition of 2N hydrochloric acid to pH 6. The product (0.37 g) was twice crystallized from N,N-dimethylformamide to afford 0.19 g of 7-[4-(aminomethyl)-2-thiazolyl]-1-ethyl-6-fluoro-1,4-dichloro-4-oxo-3-quinolinecarboxylic acid, mp 224—226°C (dec).

Example 23

1-Ethyl-6-fluoro-1,4-dihydro-7-[4-[(methylamino)methyl]-2-thiazolyl]-4-oxo-3-quinolinecarboxylic acid

A solution of 0.61 g (1.54 mmol) 7-(4-chloromethyl-2-thiazolyl)-1-ethyl-6-fluoro-1,4-dihydro-4-oxo-3-quinolinecarboxylic acid ethyl ester in 20 ml 6N hydrochloric acid was refluxed 2 hours and evaporated to dryness. The resulting solid was suspended in hot water, filtered and dried to afford 0.48 g crude product, 7-(4-chloro-methyl-2-thiazolyl)-1-ethyl-6-fluoro-1,4-dihydro-4-oxo-3-quinolinecarboxylic acid.

A solution of 0.40 g 7-(4-chloromethyl-2-thiazolyl)-1-ethyl-6-fluoro-1,4-dihydro-4-oxo-3-quinolinecarboxylic acid in 100 ml 40% aqueous methylamine was stirred at room temperature overnight and evaporated to dryness. The resulting solid was crystallized from water to afford 0.33 g of the title compound, mp 216—218°C (dec).

| | | | |
|---|---|---|---|
| Analysis calculated for $C_{17}H_{16}N_3FO_3S \cdot 0.2H_2O$: | C, 55.94; | H, 4.53; | N, 11.51 |
| Found: | C, 55.92; | H, 4.41; | N, 11.18. |

Example 24

7-(2-Amino-4-pyrimidinyl)-1-ethyl-6-fluoro-1,4-dihydro-4-oxo-3-quinolinecarboxylic acid

To 900 mg (2.5 mmol) of the 7-(2'-dimethylaminoethenyl)carbonyl-1-ethyl-6-fluoro-1,4-dihydro-4-oxo-3-quinolinecarboxylic acid ethyl ester in 15 ml of *t*-butanol at 50°C was added 580 mg (2.5 eq) of guanidine hydrochloride that had been previously treated with 1.35 g (5 eq) of potassium *t*-butoxide at 50°C in *t*-butanol for 30 minutes. The final mixture was stirred for 24 hours at 60°C. It was poured into 8% aqueous acetic acid and extracted into chloroform. The chloroform was extracted three times with water. The chloroform was dried magnesium sulfate and concentrated. The residue was purified by column chromatography and gave 295 mg of 7-(2-amino-4-pyrimidinyl)-1-ethyl-6-fluoro-1,4-dihydro-4-oxo-3-quinolinecarboxylic acid, mp 245—247°C.

Example 25

7-[2-(aminomethyl)-4-pyrimidinyl]-1-ethyl-6,8-difluoro-1,4-dihydro-4-oxo-3-quinolinecarboxylic acid

To 1.5 g (4.1 mmol) of the ethyl 7-(2'-dimethylaminoethenyl)carbonyl-1-ethyl-6,8-difluoro-1,4-dihydro-4-oxo-3-quinolinecarboxylate in 35 ml of *t*-butanol was added at 60°C a mixture of 2.49 g 2.5 equivalents) of 2-N-(benzyloxycarbonyl)aminoacetamidine hydrochloride, and 1.15 g (2.5 eq) of potassium t-butoxide in 50 ml of t-butanol. After four hours, 452 mg more of potassium t-butoxide was added. The mixture was stirred overnight at 60°C. It was poured into dilute hydrochloric acid and extracted with dichloromethane. The dichloromethane was concentrated and the residue was purified by column chromatography to give 530 mg of 1-ethyl-6,8-difluoro-1,4-dihydro-4-oxo-7-[2-[[(phenylmethoxy)carbonyl]aminomethyl]-4-pyrimidinyl]-3-quinolinecarboxylic acid as a yellow solid, mp 195—196°C. This material was then treated with hydrobromic acid in acetic acid, for three hours. The mixture was poured into ethylacetate:diethyl ether. The solids were filtered and then dissolved in aqueous ammonia pH 10.8. This mixture was concentrated to one fourth volume and the solids filtered to give 173 mg of 7-[2-(aminomethyl)-4-pyrimidinyl]-1-ethyl-6,8-difluoro-1,4-dihydro-4-oxo-3-quinolinecarboxylic acid, mp 285—289°C.

In a similar fashion the 7-(2-amino-4-pyrimidinyl)-1-ethyl-6,8-difluoro-1,4-dihydro-4-oxo-3-quinolinecarboxylic acid, mp 285—286°C (25a) was prepared using guanidine hydrochloride and ethyl 7-(2'-dimethylaminoethenyl)carbonyl-1-ethyl-6,8-difluoro-1,4-dihydro-4-oxo-3-quinolinecarboxylate.

Also the 7-[2-(aminomethyl)-4-pyrimidinyl]-1-ethyl-6-fluoro-1,4-dihydro-4-oxo-3-quinolinecarboxylic acid, mp 206—208°C (25b), was prepared from 2-N-(benzyloxycarbonyl)aminoacetamidine hydrochloride and ethyl 7-(2'-dimethylaminoethenyl)carbonyl-1-ethyl-6-fluoro-1,4-dihydro-4-oxo-3-quinolinecarboxylate.

Similarly prepared was 7-[2-[(methylamino)methyl]-4-pyrimidinyl]-1-ethyl-6-fluoro-1,4-dihydro-4-oxo-3-quinolinecarboxylic acid, mp 238—239°C (25c) from 2-N-(benzyloxycarbonyl)methylaminoacetamidine hydrochloride and ethyl 7-(2'-dimethylaminoethenyl)carbonyl-1-ethyl-6-fluoro-1,4-dihydro-4-oxo-3-quinolinecarboxylate.

Preparation of Starting Materials

Ethyl 7-acetyl-1-ethyl-6-fluoro-1,4-dihydro-4-oxo-3-quinolinecarboxylate

A solution of 26.4 g (0.17 mole) 3-acetyl-4-fluoroaniline and 34.6 ml (0.35 mole) 1,3-propanedithiol in 2.5 l chloroform was cooled to 5°C. Hydrogen chloride gas was bubbled into the stirred solution for 15 minutes at 5°—10°C, and the reaction was warmed to room temperature with stirring overnight. The solvent was removed in vacuo at 60°C, and the solid residue twice taken into 800 ml toluene and stripped in vacuo at 60°C. The solid material was dissolved in 1.5 l chloroform, washed twice with 200 ml saturated sodium bicarbonate solution, twice with 200 ml water, and dried with magnesium sulfate. Removal of the solvent in vacuo gave a brown oil, containing 4-fluoro-3-(2-methyl-1,3-dithian-2-yl)benzenamine. This oil was dissolved in 1.2 l toluene, and 34.4 ml (0.17 mole) diethyl ethoxymethylenemalonate was added. The toluene was distilled off over two hours, until the head temperature reached 120—125°C. The resulting brown oil, containing diethyl[[[4-fluoro-3-(2-methyl-1,3-dithian-2-yl)phenyl]amino]methylene]propanedioate, was poured directly into 500 ml Dowtherm A® preheated to 250°C. The temperature of the mixture was raised back to 250°C, and heated 15 minutes. After cooling, the mixture was slowly poured into 2 l pentane and stirred vigorously overnight. The solid was collected, washed well with pentane, and dried, giving 40 g of a light brown solid, ethyl 6-fluoro-1,4-dihydro-7-(2-methyl-1,3-dithian-2-yl)-4-oxo-3-quinolinecarboxylate. Without further purification, this material was treated with 75.3 g, (0.55 mole) potassium carbonate and 43.6 ml (0.55 mole) ethyl iodide in 2.7 l N,N-dimethylformamide at 85°C overnight. The solvent was removed in vacuo at 65°C, and the residue dissolved in 2 l chloroform, washed well with water, and dried with magnesium sulfate. Removal of solvent in vacuo gave 48.2 g of a light brown solid, ethyl 1-ethyl-6-fluoro-1,4-dihydro-7-(2-methyl-1,3-dithian-2-yl)-4-oxo-3-quinolinecarboxylate. Without further purification, this material was dissolved in 1 l 80% acetonitrile/water, and added over 30 minutes, at room temperature, under nitrogen, to a well-stirred suspension of 28.9 g (0.13 mole) mercuric oxide and 72.9 g (0.27 mole) mercuric chloride in 2 l 80% acetonitrile/water. The reaction was warmed to reflux for six hours under nitrogen, cooled to room temperature, and filtered through a pad of Celite®. The filter pad was washed with 3 l of 1:1 dichloromethane:hexane. The organic phase of the filtrate was separated, washed twice with 500 ml 5 M ammonium acetate solution, twice with 500 ml water, and dried with magnesium sulfate. The solvent was removed in vacuo, and the solid residue was stirred overnight in 1.5 l diethylether. The solid was collected, washed well with diethyl ether, and dried to give 23.4 g of the title compound, ethyl 7-acetyl-1-ethyl-6-fluoro-1,4-dihydro-4-oxo-3-quinolinecarboxylate, mp 106°—108°C.

Ethyl 7-bromoacetyl-1-ethyl-6-fluoro-1,4-dihydro-4-oxo-3-quinolinecarboxylic acid

A solution of 2.45 g (8.0 mmol) ethyl 7-acetyl-1-ethyl-6-fluoro-1,4-dihydro-4-oxo-3-quinolinecarboxylate in 50 ml acetic acid was treated with 0.46 g potassium bromate, and then 3.8 ml 48% hydrobromic acid was added dropwise over one half hour. The mixture was stirred 24 hours at room temperature and poured into 200 ml ice water. The precipitate was filtered, washed with water and dried to afford 2.87 g ethyl 7-bromoacetyl-1-ethyl-6-fluoro-1,4-dihydro-4-oxo-3-quinolinecarboxylate.

7-(2′-dimethylaminoethenyl)carbonyl-1-ethyl-6-fluoro-1,4-dihydro-4-oxo-3-quinolinecarboxylic acid ethyl ester

To 2.0 (6.5 mmol) of ethyl 7-acetyl-1-ethyl-6-fluoro-1,4-dihydro-4-oxo-3-quinolinecarboxylate in 50 ml of dimethylformamide was added 1.71 ml (1.25 eq) of bis-*t*-butoxydimethylaminomethane. The mixture was heated at 70°C for 18 hours. The mixture was then concentrated and the residue treated with ether and filtered to give 1.95 g of 7-(2′-dimethylaminoethenyl)carbonyl-1-ethyl-6-fluoro-1,4-dihydro-4-oxo-3-quinolinecarboxylic acid ethyl ester, mp 176—179°C.

Ethyl 7-(2′-dimethylaminoethenyl)carbonyl-1-ethyl-6,8-difluoro-1,4-dihydro-4-oxo-3-quinolinecarboxylate

To 5.00 g (15.5 mmol) of the ethyl 7-acetyl-1-ethyl-6,8-difluoro-1,4-dihydro-4-oxo-3-quinolinecarboxylate in 65 ml of dimethylformamide was added 4.27 ml (1.3 eq) of bis *t*-butoxydimethylaminomethane. The mixture was taken to 55°C overnight. The mixture was concentrated and the solids suspended in ethyl ether. Filtration gave 4.88 g of the ethyl 7-(2′-dimethylaminoethenyl)carbonyl-1-ethyl-6,8-difluoro-1,4-dihydro-4-oxo-3-quinolinecarboxylate, mp 175—177°C.

7-cyano-1,4-dihydro-1-ethyl-6-fluoro-3-quinolinecarboxylic acid ethyl ester

To a suspension of 2.78 g (10 mmole) of 7-amino-1,4-dihydro-1-ethyl-6-fluoro-3-quinolinecarboxylic acid ethyl ester in 40 ml of 1N HCl at 8° was added a solution of 0.72 g (10.5 mml) of sodium nitrite and 5 ml of water portionwise keeping the temperature at 8°C. The orange solution was stirred at 5 to 8°C for 0.5 hours. To a solution of 1.07 g (12 mmol) of cuprous cyanide, 2.28 g (35 mmol) of potassium cyanide and 25 ml of water at 45—50°C was added the diazonium solution over 10 minutes. The foaming mixture was heated with stirring at 50—60°C for 1.25 hours, then treated with 10 ml of 29% ammonium hydroxide and stirred at 50°C for 20 minutes. The solution was cooled with ice and the solid collected by filtration. The solid was recrystallized from acetonitrile to give 0.28 g of the title compound, mp 205—207°C. The acetonitrile filtrate was evaporated to dryness and the residue was triturated with ethers to yield an additional 0.63 g of product.

1-Ethyl-6-fluoro-7-thiocarbamoyl-1,4-dihydro-4-oxo-3-quinolinecarboxylic acid ethyl ester

Hydrogen sulfide was passed through a solution of 1.50 g (5.2 mmol) 7-cyano-1-ethyl-6-fluoro-4-oxo-3-quinolinecarboxylic acid ethyl ester in 25 ml pyridine and 1 ml triethylamine for five hours. After stirring overnight in a closed flask, the precipitated solid was filtered, washed with pyridine and ether, and dried to afford 1.28 g of yellowish solid of the title compound, mp 198—199°C (dec).

| | | | | |
|---|---|---|---|---|
| Analysis calculated for $C_{15}H_{15}N_2O_3FS$: | C, 55.88; | H, 4.69; | N, 8.69; | S, 9.95 |
| Found: | C, 55.77; | H, 4.78; | N, 8.43; | S, 10.15. |

7-Acetyl-1-ethyl-6,8-difluoro-1,4-dihydro-4-oxo-3-quinolinecarboxylic acid

*2,6-Difluoroacetophenone*

To 64.19 g (455 mmol) of the 2,6-difluorobenzonitrile in 300 ml of diethyl ester was added in one hour at −78°C, 650 ml of 1.6 M methyl lithium (2.0 eq). The mixture was stirred at −78°C for 2.5 hours, and then treated with 250 ml of 6N hydrochloric acid. The reaction was brought to 5°C and allowed to attain room temperature overnight. The layers were separated, then the water layer washed with dichloromethane. The ether and dichloromethane layers were combined, dried, and concentrated to an oil which was purified by column chromatography on silica gel to give 61 g of 2,6-difluoroacetophenone as a light yellow liquid: IR (liquid film) 1709, 1622 $cm^{-1}$.

*2,6-Difluoro-3-nitroacetophenone*

To 100 ml of concentrated sulfuric acid at 0° was added 17.0 g (109 mmol) of the 2,6-difluoroacetophenone slowly over 20 minutes keeping the temperature at 0—10°C. To this solution, at −5°C, was added a mixture of 20 ml concentrated sulfuric acid and 6.5 ml of 70% nitric acid premixed at 0°C before the addition. The nitrating agent was added at a sufficient rate to keep the reaction temperature at 5°C. The reaction was then stirred for 20 minutes and poured over ice. The mixture was extracted with dichloromethane two times. The dichloromethane was dried and concentrated to an oil which was purified by column chromatography to give 14.8 g of 2,6-difluoro-3-nitroacetophenone as a pale yellow oil: IR (liquid film) 1715, 1620, 1590, 1540, 1350 $cm^{-1}$.

Diethyl 3-acetyl-2,4-difluoroanilinomethylenemalonate

To 18.1 g (90.0 mmol) of the 2,6-difluoro-3-nitroacetophenone was added methanol, Raney Nickel, and hydrogen gas. When the mixture had taken up the theoretical amount of hydrogen, it was filtered into an

excess of diethyl methylenemalonate. The methanol was removed, and the mixture was treated with toluene which was then distilled away to one half volume. The mixture was then concentrated under vacuum and the residue was stirred with ether:pentane to give 24.4 g of the 3-acetyl-2,4-difluoroanilino-methylene malonate, mp 82—84°C.

Ethyl 7-acetyl-6,8-difluoro-1,4-dihydro-4-oxo-3-quinolinecarboxylate

To 380 ml of refluxing Dowtherm A was added 14.4 g (42.2 mmol) of 3-acetyl-2,4-difluoroanilino-methylenemalonate in three portions. The reaction was stirred for 30 minutes. After cooling, it was treated with 500 ml of pentane. The solids were filtered and washed with ether:pentane to give 7.9 g of ethyl 7-acetyl-6,8-difluoro-1,4-dihydro-4-oxo-3-quinolinecarboxylate, mp 267—270°C. This material was used without further purification.

To 22.3 g (76.6 mmol) of the ethyl 7-acetyl-6,8-difluoro-1,4-dihydro-4-oxo-3-quinolinecarboxylate in 900 ml of dimethylformamide was added 25.6 g (2.5 eq) of potassium carbonate and 22 ml (3.6 eq) of ethyl iodide. The mixture was stirred at 45°C overnight. The mixture was concentrated. The residue was dissolved in water and extracted into dichloromethane. The dichloromethane was concentrated and the residual oil was purified by column chromatography on silica gel to give 10.5 g of ethyl 7-acetyl-1-ethyl-6,8-difluoro-1,4-dihydro-4-oxo-3-quinoline carboxylate, mp 129—130°C.

To 400 mg (1.23 mmol) of this material was added 5 ml of 6N hydrochloric acid and the suspension was stirred at 85°C overnight. Filtration gave 310 mg of 7-acetyl-1-ethyl-6,8-difluoro-1,4-dihydro-4-oxo-3-quinolinecarboxylic acid, mp 250—251°C.

1-Ethenyl-6,7,8-trifluoro-1,8-dihydro-4-oxo-3-quinolinecarboxylic acid

In similar fashion, when the 6,7,8-trifluoro-1,4-dihydro-4-oxo-3-quinolinecarboxylic acid ethyl ester is treated with dibromo ethane, the 1-ethenyl-6,7,8-trifluoro-1,4-dihydro-4-oxo-3-quinolinecarboxylic acid ester is obtained, mp 134—135°C. Subsequent hydrolysis with hydrochloric acid gave 1-ethenyl-6,7,8-trifluoro-1,4-dihydro-4-oxo-3-quinolinecarboxylic acid, mp 186—187°C.

7-(Bromoacetyl)-1-ethyl-6,8-difluoro-1,4-dihydro-4-oxo-3-quinolinecarboxylic acid

To 1.12 g (3.80 mmol) of the 7-acetyl-1-ethyl-6,8-difluoro-1,4-dihydro-4-oxo-3-quinolinecarboxylic acid in 30 ml of acetic acid was added 0.18 g of potassium bromate and 1.48 ml of 48% hydrobromic acid. The mixture was stirred at 50°C for 24 hours. The mixture was concentrated to one-half volume and 20 ml of water was added. The solids were filtered to give 1.3 g of the 7-bromoacetyl-1-ethyl-6,8-difluoro-1,4-dihydro-4-oxo-3-quinolinecarboxylic acid, mp 213—215°C.

In similar fashion the 7-(bromoacetyl)-6,8-difluoro-1-(2-fluoroethyl)-1,4-dihydro-4-oxo-3-quinolinecarboxylic acid, mp 173—175°C, and the 10-(bromoacetyl)-9-fluoro-2,3-dihydro-3-methyl-7-oxo-7*H*-pyrido[1,2,3-de]-1,4-benzoxazine-6-carboxylic acid, mp 224—225°C, were prepared.

7-acetyl-6,8-difluoro-1-(2-fluoroethyl)-1,4-dihydro-4-oxo-3-quinolinecarboxylic acid

To 1.62 g (34.0 mmol) of sodium hydride (50% dispersion in oil, pentane washed) in 250 ml of dimethylformamide was added 10.0 g (34 mmol) of the ethyl 7-acetyl-6,8-difluoro-1,4-dihydro-4-oxo-3-quinolinecarboxylate dissolved in 100 ml of dimethylformamide at room temperature. The mixture was stirred for two hours, and 13.0 g (2.4 eq) of 1-bromo-2-fluoroethane was added. The mixture was stirred overnight at 50°C. It was concentrated, and partitioned between water and dichloromethane. The dichloromethane was then concentrated and the residue purified by column chromatography to give 3.75 g of ethyl 7-acetyl-6,8-difluoro-1-(2-fluoroethyl)-1,4-dihydro-4-oxo-3-quinoline carboxylate, mp 155—156°C. This material was hydrolyzed with 2N hydrochloric acid and 2-propanol as co-solvent to give 2.95 g of the 7-acetyl-6,8-difluoro-1-(2-fluoroethyl)-1,4-dihydro-4-oxo-3-quinoline carboxylic acid, mp 215—216°C.

6,7,8-Trifluoro-1-(2-fluoroethyl)-1,4-dihydro-4-oxo-3-quinolinecarboxylic acid

In identical fashion, 6,7,8-trifluoro-1,4-dihydro-4-oxo-3-quinoline carboxylic acid ethyl ester was converted to 6,7,8-trifluoro-1-(2-fluoroethyl)-1,4-dihydro-4-oxo-3-quinoline carboxylic acid, mp 207—211°C.

1-[6-fluoro-2-[2-(2-methyl-1,3-dioxalan-2-yl)propoxy]-3-nitrophenyl]ethanone

To 35.45 g (0.230 mole) of 2-hydroxymethyl-2-methyl-1,3-dioxolane 0.78 $H_2O$ in 300 ml tetrahydrofuran at −78°C, was added 100 ml of 2.3 M n-butyl lithium. The solution was warmed to −40°C and added to 46.35 g (0.230 mole) of 2,6-difluoro-3-nitroacetophenone in 200 ml tetrahydrofuran at 0°C. The reaction was stirred 30 minutes then poured into 1000 ml of an ethyl acetate saturated ammonium chloride solution (1:1). The solution was filtered through celite. The layers separated, and the aqueous layer extracted 3 × 500 ml of ethyl acetate. The combined organic extracts were washed with brine, dried over magnesium sulfate and the solvent removed at reduced pressure. The product was chromatographed on silica, using hexane:ether (4:1) to give 41.2 g of 1-[6-fluoro-2-[2-(2-methyl-1,3-dioxalan-2-yl)-propoxy]-3-nitrophenyl]ethanone.

1-(2-acetyl-3-ethanone-1-(2-acetyl-3-fluoro-6-nitrophenoxy)-2-propanone

2.2 g (7.35 mmole) of 1-[6-fluoro-2-[2-(2-methyl-1,3-dioxalan-2-yl)propoxy]-3-nitrophenyl]ethanone

and 360 ml of water:hydrochloric acid:acetic acid (100:10:250) were stirred overnight. The solvents were removed at reduced pressure, the residue taken up in dichlormethane and washed repeatedly with water. The organic layer was dried over magnesium sulfate and the solvent removed at reduced pressure. The residue was titurated in pentane:$Et_2O$ (3:1) to yield 1.78 g of 1-(2-acetyl-3-fluoro-6-nitrophenoxy)-2-propanone, mp 64—65°C.

N-methyl-5-oxo-1-(phenylmethyl)-3-pyrrolidinecarboxamide

A mixture of 100 g (0.43 mole) of methyl 5-oxo-1-(phenylmethyl)-pyrrolidinecarboxylate [J. Org. Chem., *26,* 1519 (1961)], 500 ml methanol and 100 g (3.2 mole) of methylamine was heated at 100°C in a pressure reactor for 16 hours. The reaction mixture was cooled and the ammonia and methanol were removed under reduced pressure. The residue was taken up in dichloromethane and washed 3 × 100 ml 1N sodium hydroxide. The organic layer was dried over magnesium sulfate and solvent removed at reduced pressure to give 88.3 g of N-methyl-5-oxo-1-(phenylmethyl)-3-pyrrolidinecarboxamide as a white solid, mp 82.5—83.0°C.

| | | | |
|---|---|---|---|
| Analysis calculated for $C_{13}H_{16}N_2O_2$: | C, 67.22; | H, 6.94; | N, 12.06 |
| Found: | C, 66.98; | H, 6.69; | N, 12.02 |

This material was used in the next step.

N-methyl-1-(phenylmethyl)-3-pyrrolidinemethanamine

To a suspension of 37.40 g (1.00 mole) lithium aluminum hydride in 1000 ml tetrahydrofuran, is added a solution of 88.3 g (0.380 mole) of N-methyl-5-oxo-1-(phenylmethyl)-3-pyrrolidinecarboxamide in tetrafuran dropwise under nitrogen. The reaction was then refluxed overnight. The reaction flask was cooled in an ice bath and 37.4 ml of water, 37.4 ml of 15% sodium hydroxide and 112.2 ml of water were added. The precipitated solids were filtered and washed with hot ethanol. The combined filtrates were concentrated, then dissolved in dichloromethane, filtered, dried over magnesium sulfate, and the solvent evaporated under reduced pressure to give 68.68 g of N-methyl-1-(phenylmethyl)-3-pyrrolidinemethanamine as an oil. This material was used without further purification in the step.

N-methyl-3-pyrrolidinemethanamine

A mixture of 67.28 g (0.32 mole) of N-methyl-1-(phenylmethyl)-3-pyrrolidinemethanamine, 3 g of 20% palladium on carbon, and 600 ml of methanol was shaken in an atmosphere of hydrogen at about 4.5 × $10^5$ Pa and at room temperature for 18 hours. Another 3 g of 20% palladium on carbon was added and the hydrogenation continued for 6.5 hours. Another 3.0 g of 20% palladium on charcoal was added and the hydrogenation continued for another 4.5 hours. The catalyst was filtered and the filtrate evaporated under reduced pressure. The residue was distilled under vacuum (72—76°C, 1.39 × $10^3$ Pa (10.5 mm Hg)) to give 8.32 g N-methyl-3-pyrroldinemethanamine.

N-Ethyl-3-pyrrolidinemethanamine

N-Ethyl-5-oxo-1-(phenylmethyl)-3-pyrrolidinecarboxamide

A mixture of 200 g (0.86 mole) of methyl 5-oxo-1-(phenylmethyl)-pyrrolidinecarboxylate [J. Org. Chem., *26,* 1519 (1961)], 1000 ml methanol and 200 g (4.4 mole) of ethylamine was heated at 100°C in a pressure reactor for 17.2 hours. The reaction mixture was cooled and the excess ethylamine and methanol were removed under reduced pressure. The residue was taken up in dichloromethane and washed 3 × 150 ml 1N sodium hydroxide. The organic layer was dried over magnesium sulfate and the solvent removed at reduced pressure to give 104.6 g of N-ethyl-5-oxo-1-(phenylmethyl)-3-pyrrolidinecarboxamide as a white solid, mp 97—99°C.

This material was used in the next step.

N-ethyl-1-(phenylmethyl)-3-pyrrolidinemethanamine

To a suspension of 108.68 g (2.860 mole) lithium aluminum hydride in 800 ml tetrahydrofuran, is added a solution of 194.5 g (0.790 mole) of N-ethyl-5-oxo-1-(phenylmethyl)-3-pyrrolidinecarboxamide in 600 ml tetrahydrofuran dropwise under nitrogen. The reaction was then refluxed four hours. The reaction flask was cooled in an ice bath and 108 ml of water, 108 ml of 15% sodium hydroxide, and 324 ml of water were added. The precipitated solids were filtered and washed with hot ethanol. The combined filtrates were concentrated, then dissolved in dichloromethane, filtered, dried over magnesium sulfate, and the solvent evaporated under reduced pressure to give 151.9 g of N-ethyl-1-(phenylmethyl)-3-pyrrolidinemethanamine as an oil.

This material was used without further purification in the next step.

N-ethyl-3-pyrrolidinemethanamine

A mixture of 151.65 g (0.695 mole) of N-ethyl-1-(phenylmethyl)-3-pyrrolidinemethanamine, 5 g of 20% palladium on carbon, and 1100 ml of ethanol was shaken in an atmosphere of hydrogen at about 4.5 × $10^5$ Pa and at room temperature for 21.6 hours. Another 5 g of 20% palladium on carbon was added and the

hydrogenation continued for 24 hours. The catalyst was filtered and the filtrate evaporated under reduced pressure. The residue was distilled under vacuum (88—91°C, 1.53 × $10^3$ Pa (11.5 mm Hg)) to give 66.0 g N-ethyl-3-pyrrolidinemethanamine.

N-(2,2,2-Trifluoroethyl)-3-pyrrolidinemethanamine

5-Oxo-1-(phenylmethyl)-N-(2,2,2-trifluoroethyl)-3-pyrrolidine carboxamide

A mixture of 21.9 g (0.100 mole) methyl-5-oxo-1-(phenylmethyl)-3-pyrrolidinecarboxylate in 150 ml tetrahydrofuran, was cooled to 0°C in an ice bath under nitrogen and 24.32 g (0.150 mole) carbonyl diimidazole was added. The reaction was stirred at 0°C for 30 minutes, then at room temperature for 30 minutes. A solution of 13.55 g (0.100 mole) of 2,2,2-triflouroethylamine hydrochloride, 15.22 g (0.100 mole) 1,8-diazabicyclo[5.4.0]undec-7-ene and 100 ml tetrahydrofuran was added. The reaction was stirred at room temperature overnight. The solvent was removed at reduced pressure. The residue was taken up in dichloromethane and washed 3 × 150 ml saturated sodium bicarbonate. The organic layer was dried over magnesium sulfate and the solvent removed under reduced pressure. The product was purified by column chromatography on silica with ethyl acetate to give 8.50 g of 5-oxo-1-(phenylmethyl)-N-(2,2,2-trifluoroethyl)-3-pyrrolidinecarboxamide mp 110—112°C.

This material was used in the next step.

1-(Phenylmethyl)-N-(2,2,2-trifluoroethyl)-3-pyrrolidinemethanamine

A mixture of 8.50 g (28.3 mole) of 5-oxo-1-(phenylmethyl)-N-(2,2,2-trifluoroethyl)-3-pyrrolidinemethanamine in 100 ml tetrahydrofuran was added dropwise to 3.22 g (84.9 mmole) of lithium aluminum hydride in 50 ml tetrahyrofuran. The reaction was refluxed two hours, then stirred at room temperature overnight. The reaction was cooled in an ice bath and 3.2 ml of water, 3.2 ml of 15% sodium hydroxide, and 9.6 ml of water were added. The precipitated salts were filtered and washed with hot ethanol. The combined filtrates were concentrated under reduced pressure. The residue was taken up in dichloromethane, filtered, and dried over magnesium sulfate. The solvent was removed at reduced pressure to give 7.15 g of 1-(phenylmethyl-N-(2,2,2-trifluoroethyl)-3-pyrrolidinemethanamine.

This material was used without further purification in the next step.

N-(2,2,2-trifluoroethyl)-3-pyrrolidinemethanamine

A mixture of 7.15 g (26.3 mmole) 1-(phenylmethyl)-N-(2,2,2-trifluoromethyl)-3-pyrrolidinemethanamine 100 ml of methanol and 0.7 g of 20% palladium on carbon was shaken in an atmosphere of hydrogen at about 4.5 × $10^5$ Pa and at room temperature for 24 hours. The catalyst was filtered and the filtrate evaporated under reduced pressure. The residue was distilled under vacuum (63—65°C, 0.37 × $10^3$ Pa (2.8 mmHg)) to give 2.55 g of N-(2,2,2-trifluoroethyl)-3-pyrrolidinemethanamine.

N-Propyl--3-pyrrolidinemethanamine

5-Oxo-1-(phenylmethyl)-N-propyl-3-pyrrolidinecarboxamide

To a solution of 10.96 g (50 mmole) of 5-oxo-1-(phenylmethyl)-3-pyrrolidinecarboxylic acid in 150 ml of acetonitrile was added 9.73 g (60 mmole) of 1,1′-carbonyldiimdazole. The reaction was heated to 60°C for one hour, cooled to room temperature and treated with 4.13 g (70 mmole) of n-propylamine. After stirring for two hours, the solvent was removed in vacuo and the residue partitioned between ether and water. The organic layer was washed with water, 1N hydrochloric acid, dried over magnesium sulfate, filtered, and evaporated in vacuo to give 12.0 g of 5-oxo-1-(phenyl-methyl)-N-propyl-3-pyrrolidinecarboxamide, mp 86—87°C.

1-(Phenylmethyl)-N-propyl-3-pyrrolidinemethanamine

To a suspension of 8.2 g (0.2 mole) of lithium aluminum hydride in 150 ml of dry tetrahydrofuran was added portionwise, 12.0 g (45.6 mmole) of solid 5-oxo-1-(phenylmethyl)-N-propyl-3-pyrrolidinecarboxamide. When the addition was complete, the reaction mixture was stirred at room temperature for 18 hours and then at reflux for two hours. After cooling to room temperature, the mixture was treated dropwise, successively, with 8 ml of water, 8 ml of 15% aqueous sodium hydroxide and 24 ml of water, titrating the final addition to produce a granular precipitate. The solid was removed by filtration, washed with tetrahydrofuran and the filtrate evaporated in vacuo to give 9.6 g of 1-(phenylmethyl)-N-propyl-3-pyrrolidinemethanamine, as a heavy syrup.

This material was used for the next step without further purification.

N-Propyl-3-pyrrolidinemethanamine

A mixture of 14.0 g (60.0 mmole) of 1-(phenylmethyl)-N-propyl-3-pyrrolidinemethanamine, 1.0 g of 20% palladium on carbon and 140 ml of methanol was shaken in an atmosphere of hydrogen at about 4.5 × $10^5$ Pa and room temperature for 24 hours. The catalyst was removed by filtering through Celite®, the filtrate concentrated and distilled in vacuo to give 7.1 g of N-propyl-3-pyrrolidinemethanamine, bp 49—50°C/0.033 × $10^3$ Pa (0.25 mm).

N-Cyclopropyl-3-pyrrolidinemethanamine

5-Oxo-1-(phenylmethyl)-N-cyclopropyl-3-pyrroldinecarboxamide

To a solution of 16.4 g (75 mmole) of 5-oxo-1-(phenylmethyl)-3-pyrroldinecarboxylic acid in 150 ml of acetonitrile was added 13.8 g (85 mmole) of 1,1′-carbonyldiimidazole. The reaction was heated to 60°C for one hour, cooled to room temperature and treated with 4.85 g (85 mmole) of cyclopropylamine. The reaction was stirred at room temperature for 18 hours, the solvent removed in vacuo and the residue partitioned between chloroform and water. The organic layer was washed with water, 1 N hydrochloric acid, dried over magnesium sulfate, filtered, and evaporated in vacuo to give 18.3 g of 5-oxo-1-(phenylmethyl)-N-cyclopropyl-3-pyrrolidinecarboxamide, mp 94—96°C.

1-(Phenylmethyl)-N-cyclopropyl-3-pyrrolidinemethanamine

To a suspension of 8.2 g (0.20 mole) of lithium aluminum hydride in 150 ml of dry tetrahydrofuran was added portionwise 18.0 g (70.0 mmole) of solid 5-oxo-1-(phenylmethyl)-N-cyclopropyl-3-pyrrolidinecarboxamide. When the addition was complete, the reaction mixture was stirred at room temperature for 18 hours and then at reflux for two hours. After cooling to room temperature, the mixture was treated dropwise, successively, with 8 ml of water, 8 ml of 15% aqueous sodium hydroxide and 24 ml of water, titrating the final addition to produce a granular precipitate. The solid was removed by filtration, washed with tetrahydrofuran and the filtrate evaporated in vacuo to give 16.0 g of 1-(phenylmethyl)-N-cyclopropyl-3-pyrrolidinemethanamine, as a heavy oil. This was used for the next step without further purification.

N-Cyclopropyl-3-pyrrolidinemethanamine

A mixture of 13.6 g (59.0 mmol) of 1-(phenylmethyl)-N-cyclopropyl-3-pyrrolidinemethanamine, 0.5 g of 20% palladium on carbon and 140 ml of methanol was shaken in an atmosphere of hydrogen at about 4.5 × $10^5$ Pa and room temeprature for 24 hours. The catalyst was removed by filtering through Celite, the filtrate concentrated and distilled in vacuo to give 6.3 g of N-cyclopropyl-3-pyrrolidinemethanamine, bp 88—90°/ 1.73 × $10^3$ Pa (13 mm).

N-(2-Propyl)-3-pyrrolidinemethanamine

5-Oxo-1-(phenylmethyl-*N*-(2-propyl)-3-pyrrolidinecarboxamide

To a solution of 16.4 g (75.0 mmole) of 5-oxo-1-(phenylmethyl)-3-pyrrolidinecarboxylic acid in 150 ml of acetonitrile was added 13.8 g (85.0 mmole) of 1,1′-carbonyldimidazole. The reaction was heated to 60°C for one hour, cooled to room temperature and treated with 5.0 g (85 mmole) of isopropylamine. The reaction was stirred at room temperature for 18 hours, the solvent removed in vacuo and the residue partitioned between chloroform and water. The organic layer was washed with water, 1N hydrochloric acid, dried over magnesium sulfate and evaporated in vacuo to give 18.6 g of 5-oxo-1-(phenylmethyl)-N-(2-propyl)-3-pyrrolidinecarboxamide, mp 122—124°C.

1-(Phenylmethyl)-N-(2-propyl)-3-pyrrolidinemethanamine

To a suspension of 8.2 g (0.2 mole) of lithium aluminum hydride in 150 ml of dry tetrahydrofuran was added portionwise, 18.3 g (70.0 mmole) of solid 5-oxo-1-(phenylmethyl)-N-(2-propyl)-3-pyrrolidinecarboxamide. When the addition was complete, the reaction mixture was stirred at room temperature for 18 hours and then refluxed for two hours. After cooling to room temperature, the mixture was treated dropwise, successively, with 8 ml of water, 8 ml of 15% aqueous sodium hydroxide and 24 ml of water, titrating the final addition to produce a granular precipitate. The solid was removed by filtration, washed with tetrahydrofuran and the filtrate evaporated in vacuo to give 15.6 g of 1-(phenylmethyl)-N-(2-propyl)-3-pyrrolidinemethanamine as a heavy syrup.

This material was used for the next step without further purification.

N-(2-Propyl)-3-pyrrolidinemethanamine

A mixture of 13.4 g (58.0 mmol) of 1-phenylmethyl-N-(2-propyl)-3-pyrrolidinemethanamine, 1.0 g of 20% palladium on carbon and 130 ml of methanol was shaken in an atmosphere of hydrogen at about 4.5 × $10^5$ Pa and room temperature for 24 hours. The catalyst was removed by filtration through Celite®; the filtrate concentrated and distilled in vacuo to give 6.3 g of N-(2-propyl)-3-pyrrolidinemethanamine, bp 58—60°C/0.46 × $10^3$ Pa (3.5 mm).

2-[(3-pyrrolidinylmethyl)amino]ethanol

N-(2-hydroxyethyl)-5-oxo-1-(phenylmethyl)-3-pyrrolidinecarboxamide

A mixture of 46.7 g (1200 mole) of methyl-5-oxo-1-(phenylmethyl)-3-pyrrolidinecarboxylate (J. Org. Chem., 26, 1519 (1961)], 36.7 g (1600 mole) 2-aminoethanol and 500 ml methanol were refluxed overnight. The reaction was cooled to room temperature and the solvent removed at reduced pressure. The residue was taken up in dichloromethane and extracted 3 × 100 ml 1N sodium hydroxide. The aqueous layer was taken to pH 5, extracted 3 × 150 ml dichloromethane, then taken to pH 8 and again extracted 3 × 150 ml dichloromethane. The aquoeus layer was concentrated at reduced pressure and the resulting slurry stirred in dichloromethane. The salts were filtered off. The combined organic layers were dried over magnesium sulfate, the solvent removed at reduced pressure to yield 47.9 g of N-(2-hydroxyethyl)-5-oxo-1-phenylmethyl)-3-pyrrolidinecarboxamide as an oil. This was used in the next step without further purification.

2-[[[1-(phenylmethyl)-3-pyrrolidinyl]methyl]amino]ethanol

A mixture of 46.66 g (0.178 mole) of N-(2-hydroxyethyl)-5-oxo-2-(phenylmethyl)-3-pyrrolidinecarboxamide in 200 ml of tetrahydrofuran was added dropwise to a slurry of 20.25 g (0.534 mole) of lithium aluminum hydride in 150 ml tetrahydrofuran. The reaction was refluxed three hours, then cooled in an ice bath. The work up consisted of sequential addition of 20 ml water, 20 ml 15% sodium hydroxide then 60 ml water. The reaction was filtered and the precipitate washed with ethanol. The filtrate was concentrated at reduced pressure, the residue taken up in dichloromethane, dried over magnesium sulfate, and the solvent removed at reduced pressure to give 32.31 g of 2-[[[1-(phenylmethyl)-3-pyrrolidinyl]methyl]amine]ethanol as an oil. This material was used in the next step without further purification.

2-[(3-pyrrolidinylmethyl)amino]ethanol

A mixture of 32.32 g of 2-[[[1-(phenylmethyl)-3-pyrroldinyl]-methyl]amino]ethanol, 330 ml of methanol and 3 g of 20% palladium on charcoal was shaken in an atmosphere of hydrogen at about $4.5 \times 10^5$ Pa and at room temperature for 18 hours. The solvents were then removed at reduced pressure. The residue was distilled under vacuum (bp 129—131°C, 1.5 $0.199 \times 10^3$ Pa (mm Hg) to give 11.43 g of 2-[(3-pyrrolidinylmethyl)amino]ethanol.

1,1-Dimethylethyl[1-(diphenylmethyl)-3-azetidinyl]carbamate

A soluton of 12.2 g (51.2 mmole) of 1-(diphenylmethyl)-3-azetidineamine in a mixture of 34 ml of water and 100 ml of *t*-butanol was treated dropwise with 11.4 g (52 mmol) of ditertiarybutyldicarbonate. After the addition was complete, the reaction was heated at 60°C for 1 hour, then at room temperature for 18 hours. The reaction mixture was diluted with water and extracted with chloroform. The chloroform layer was washed with water, dried over magnesium sulfate, filtered, and evaporated in vacuo to give 14.3 g of 1,1-dimethylethyl-1-(diphenylmethyl)-3-azetidinyl]carbamate, mp 148—153°C.

1,1-Dimethylethyl(3-azetidinyl)carbamate

A solution of 14.2 g (42.0 mmole) of 1,1-dimethylethyl-[1-(diphenylmethyl)-3-azetidinyl]carbamate in 100 ml of tetrahydrofuran was shaken with 2 g of 20% palladium on carbon in a hydrogen atmosphere at $4.5 \times 10^5$ Pa for 24 hours. The reaction was filtered through Celite® and the solvent was removed in vacuo. The residue was triturated several times with hexane to give, as the insoluble residue, 6.5 g of 1,1-dimethylethyl-(3-azetidinyl)carbamate, mp 138—140°C.

1-(Diphenylmethyl)-3-azetidinemethanamine

A suspension of 5.7 g (0.15 mole) of lithium aluminum hydride in 200 ml of dry tetrahydrofuran was treated portionwise with 18.6 g (75 mmole) of solid 3-cyano-1-(diphenylmethyl)azetidine. When the addition was complete, the reaction was stirred at room temperature for two hours, refluxed for four hours, and stirred at room temperature for 18 hours. The reaction was decomposed by the successive addition of 6 ml of water, 6 ml of 15% sodium hydroxide, and 18 ml of water, titrating the final water addition to give a granular precipitate. The inorganic precipitate was removed by filtration, washed with tetrahydrofuran and evaporated in vacuo to give 16.9 g of 1-(diphenylmethyl)-3-azetidinemethanamine as a heavy oil.

1,1-Dimethylethyl[[1-(diphenylmethyl)-3-azetidinyl]-methyl]carbamate

To a solution of 12.0 g (47 mmole) of 1-(diphenylmethyl)-3-azetidinemethanamine, 2.08 g (52 mmole) of sodium hydroxide in 34 ml of water and 100 ml of t-butanol was added dropwise 11.4 g (52 mmole) of ditertiarybutyl-dicarbonate. The reaction was heated at 60°C for one hour, then at room temperature for 18 hours. The reaction was then diluted with water and extracted with chloroform. The organic layer was washed with water, dried over magnesium sulfate, filtered and evaporated in vacuo to give 14.2 g of 1,1-dimethylethyl[[1-(diphenylmethyl)-3-azetidinyl]methyl]carbamate.

1,1-Dimethyl(3-azetidinylmethyl)carbamate

A solution of 13.7 g (38.9 mmole) of 1,1-dimethylethyl[[1-diphenylmethyl)-3-azetidinyl]methyl]carbamate in 150 ml of tetrahydrofuran was shaken with 2 g of 20% palladium on carbon

in a hydrogen atmosphere 4.5 × $10^5$ Pa for 24 hours. The reaction was filtered through Celite® and the solvent removed in vacuo. The residue was triturated several times with hexane to give, as an insoluble residue, 6.8 g of 1,1-dimethyl(3-azetidinylmethyl)carbamate as a viscous oil. This was used without further purification.

1-(Diphenylmethyl)-N-methyl-3-azetidinecarboxamide

To a solution of 7.5 g (28 mmole) of 1-(diphenylmethyl)azetidine-3-carboxylic acid in 75 ml of acetonitrile was added 6.0 g (37 mmole) of 1,1'-carbonyldiimidazole. The reaction was heated at 60°C for two hours and successively treated with 3.11 g (30.8 mmole) of triethylamine and 2.08 g (30.8 mmole) of methylamine hydrochloride. The reaction was stirred at 60°C for an additional hour, the solvent evaporated in vacuo, and the residue dissolved in chloroform. After washing with water and drying over magnesium sulfate, the chloroform layer was evaporated in vacuo to give 9.0 g of 1-(diphenylmethyl)-N-methyl-3-azetidinecarboxamide, mp 103—107°C.

1-(Diphenylmethyl)-N-methyl-3-azetidinemethanamine

To a suspension of 3.2 g (85 mmole) of lithium aluminum hydride in 50 ml of dry tetrahydrofuran was added dropwise, a solution of 8.5 g (28 mmole) of 1-diphenylmethyl-N-methyl-3-azetidinecarboxamide in 50 ml of dry tetrahydrofuran. After the addition was complete, the reaction was refluxed for two hours, cooled to room temperature, and decomposed by the successive addition of 3.4 ml of water, 3.4 ml of 15% aqueous sodium hydroxide and 10.2 ml of water, titrating the final water addition to give a granular precipitate. The inorganic precipitate was removed by filtration, washed with tetrahydrofuran, and evaporated in vacuo. The residue was dissolved in chloroform, dried over magnesium sulfate, filtered, and evaporated in vacuo to give 7.0 g of 1-(diphenylmethyl-N-methyl-3-azetidinemethanamine as a heavy syrup. This was used without further purification.

N-Methyl-3-azetidininemethanamine

A solution of 6.7 g (25 mmole) of 1-diphenylmethyth-N-methyl-3-azetidinemethanamine in 100 ml of methanol was shaken with 2.0 g of 20% palladium on carbon in a hydrogen atmosphere at 4.5 × $10^5$ Pa for 18 hours. The reaction was filtered through Celite® and the solvent removed in vacuo. The residue was triturated several times with hexane to give, as the insoluble residue, 2.3 g of N-methyl-3-azetidinemethanamine as a heavy syrup. This was used without further purification.

1-(Diphenylmethyl)-N-ethyl-3-azetidinecarboxamide

To a solution of 7.5 g (28 mmole) of 1-(diphenylmethyl)azetidine-3-carboxylic acid in 75 ml of acetonitrile was added 6.0 g (37 mmole) of 1,1'-carbonyldiimidazole. The reaction was heated at 60°C for two hours and successively treated with 3.1 g (30.8 mmole) of triethylamine and 2.52 g (30.8 mmole) of ethylamine hydrochloride. The reaction was stirred at 60°C for an additional hour, the solvent evaporated in vacuo and the residue dissolved in chloroform. After washing with water and drying over magnesium sulfate, the chloroform layer was evaporated in vacuo to give 9.4 g of 1-(diphenylmethyl)-N-ethyl-3-azetidinecarboxamide, mp 91—93°C.

1-(Diphenylmethyl)-N-ethyl-3-azetidinemethanamine

To a suspension of 3.2 g (85 mmole) of lithium aluminum hydride in 50 ml of dry tetrahydrofuran was added dropwise, a solution of 8.5 g (28.0 mmole) of 1-(diphenylmethyl)-N-ethyl-3-azetidinecarboxamide in 50 ml of dry tetrahydrofuran. After the addition was complete, the reaction was refluxed for two hours, cooled to room temperature, and decomposed by the successive addition of 3.4 ml of water, 3.4 ml of 15% aqueous sodium hydroxide and 10.2 ml of water, titrating the final water addition to give a granular precipitate. The inorganic precipitate was removed by filtration, washed with tetrahydrofuran and evaporated in vacuo to give 6.7 g of 1-(diphenylmethyl)-N-ethyl-3-azetidinemethanamine as a heavy syrup. This was used without further purification.

N-Ethyl-3-azetidinemethanamine

A solution of 6.4 g (23 mmole) of 1-(diphenylmethyl)-N-ethyl-3-azetidinemethanamine in 100 ml of methanol was shaken with 2.0 g of 20% palladium on carbon in a hydrogen atmosphere at 4.5 × $10^5$ Pa for 18 hours. The reaction was filtered through Celite® and the solvent removed in vacuo. The residue was triturated several times with hexane to give, as the insoluble residue, 1.6 g of N-ethyl-3-azetidinemethanamine as a heavy syrup. This was used without further purification.

2-Methyl-2,7-diazaspiro[4.4]nonane-1,3,8-trione

A solution of 20.3 g (0.084 mole) 3-ethoxycarbonyl-5-oxo-3-pyrrolidineacetic acid ethyl ester [J. Org. Chem. *46,* 2757 (1981)] in 40 ml of 40% aqueous methylamine was stirred at room temperature overnight, then placed in an oil bath and gradually heated to 220°C over 30 minutes allowing volatiles to distil from the open flask. The crude product was crystallized from ethanol to afford 12.56 g of the title compound, mp 201—204°C.

Analysis calculated for $C_8H_{10}N_2O_3$: C, 52.74; H, 5.53; N, 15.38.

Found: C, 52.87; H, 5.60; N, 15.25.

7-Benzyl-2-methyl-2,7-diazaspiro[4.4]nonane-1,3,8-trione

A solution of 1.82 g (10 mmole) 2-methyl-2,7-diazaspiro[4.4]nonane-1,3,8-trione in 20 ml N,N-dimethylformamide was added gradually under a nitrogen atmosphere to 0.050 g (10.4 mmole) of 50% oil suspension of sodium hydride which had been previously washed twice with toluene and covered with 10 ml N,N-dimethylformamide. After stirring one hour there was added 1.40 g (11 mmol) of benzyl chloride and stirring was continued overnight at room temperature. After concentrating to a small volume in vacuo, the residue was diluted with 40 ml water and extracted twice with dichloromethane. The combined organic phase was washed with water, dried over magnesium sulfate, and evaporated to give a solid. Crystallization from toluene:hexane to afford 1.74 g of the title compound, mp 157—158°C.

Analysis calculated for $C_{15}H_{16}N_2O_3$: C, 66.16; H, 5.92; N, 10.27.
Found: C, 66.45; H, 5.79; N, 10.09.

2-Benzyl-7-methyl-2,7-diazaspiro[4.4]nonane Dihydrochloride

A solution of 1.36 g (5.0 mmol) 7-benzyl-2-methyl-2,7-diazaspiro[4.4]nonane-1,3,8-trione in 50 ml tetrahydrofuran was added dropwise to a suspension of 0.95 g (25 mmol) lithium aluminum hydride in 30 ml tetrahydrofuran. The mixture was stirred overnight at room temperature, refluxed one hour, cooled, and treated dropwise with 0.95 ml water, 0.95 ml 15% sodium hydroxide solution and 2.8 ml water. After removal of the inorganic solids by filtration, the filtrate was concentrated in vacuo to give a syrup which was dissolved in isopropanol and treated with excess 6N hydrogen chloride in isopropanol. Crystallization afforded 0.97 g of the title compound, mp 233—234°C.

Analysis calculated for $C_{15}H_{24}N_2Cl_2$: C, 59.40; H, 7.98; N, 9.24; Cl, 23.38.
Found: C, 59.37; H, 7.98; N, 9.03; Cl, 23.09.

2-Methyl-2,7-diazaspiro[4.4]nonane Dihydrochloride

A solution of 7-benzyl-2-methyl-2,7-diazaspiro[4.4]nonane dihydrochloride in 150 ml methanol with 1.0 g 20% palladium on carbon catalyst was hydrogenated at 4.5 × $10^5$ Pa for two days. After filtration, the filtrate was concentrated to a thick syrup which crystallized on addition of acetonitrile to give 11.50 g of the title compound, softened at 164°C and melted at 168—170°C.

Analysis calculated for $C_8H_{18}N_2Cl_2$: C, 45.08; H, 8.51; N, 13.15; Cl, 33.27;
Found: C, 45.24; H, 8.77; N, 13.18; Cl, 33.26.

2-Ethyl-2,7-diazaspiro[4.4]nonane-1,3,8-trione

A suspension of 24.33 g (0.100 mmole) 3-ethoxycarbonyl-5-oxo-3-pyrrolidineacetic acid ethyl ester in an excess of 2N sodium hydroxide, was stirred three hours at room temperature, acidified with dilute hydrochloric acid, and evaporated to dryness in vacuo. The product, 3-carboxy-5-oxo-3-pyrrolidineacetic acid, was taken up in isopropyl alcohol, separated from insoluble sodium chloride by fitration, concentrated to a syrup and dissolved in 100 ml 70% ethylamine. The solution was gradually heated in an oil bath up to 230°C allowing volatiles to distil and then maintained at 230—240°C for ten minutes. After cooling, the product was crystallized from isopropyl alcohol to afford 10.12 g of the title compound, mp. 168—169°C.

Analysis calculated for $C_9H_{12}N_2O_3$: C, 55.09; H, 6.17; N, 14.28;
Found: C, 55.03; H, 5.84; N, 14.01.

2-Ethyl-7-benzyl-2-7-diazaspiro[4.4]nonane-1,3,8-trione

A suspension of sodium hydride (2.20 g of 60% oil suspension (0.055 mole) washed with toluene) in 50 ml N,N-dimethylformamide was treated gradually with a solution of 10.0 g (0.051 mole) 2-ethyl-2,7-diazaspiro[4.4]nonane-1,3,8-trione in 100 ml N,N-dimethylformamide. After stirring 15 minutes, there was added dropwise 6.4 ml (0.055 mole) benzyl chloride and the mixture was stirred overnight, concentrated in vacuo and shaken with water-methylene chloride. The organic layer was dried, evaporated, and the product crystallized from toluene-hexane to afford 11.11 g of the title compound, mp 125—126.5°C.

Analysis calculated for $C_{16}H_{18}N_2O_3$: C, 67.11; H, 6.34; N, 9.79.
Found: C, 67.41; H, 6.33; N, 9.79.

2-Benzyl-7-ethyl-2,7-diazaspiro[4.4]nonane Dihydrochloride

A solution of 11.00 g (0.0385 mole) 2-ethyl-7-benzyl-2,7-diazaspiro[4.4]nonane-1,3,8-trione in 100 ml tetrahydrofuran was added dropwise to a suspension of 6.00 g (0.158 mole) lithium aluminium hydride in 250 ml tetrahydrofuran. After stirring overnight, the mixture was refluxed one hour, cooled, and treated dropwise with 6 ml water, 6 ml 15% sodium hydroxide, and 18 ml water. Inorganic solids were separated by filtration and the filtrate was concentrated, taken up in ether, dried with magnesium sulfate, and reevaporated. The resulting syrup was dissolved in isopropyl alcohol and treated with excess hydrogen chloride in isopropyl alcohol to afford 9.63 g of the title compound, mp 196—198°C (dec).

Analysis calculated for $C_{16}H_{26}N_2Cl_2$: C, 60.56; H, 8.26; N, 8.83; Cl, 22.35.
Found: C, 60.51; H, 8.08; N, 8.69; Cl, 22.26.

2-Ethyl-2,7-diazaspiro[4.4]nonane Dihydrochloride

A solution of 9.50 g (0.030 mole) 2-benzyl-7-ethyl-2,7-diazaspiro[4.4]nonane dihydrochloride in 100 ml methanol was hydrogenated with 1.0 g 20% palladium on carbon catalyst at $4.5 \times 10^5$ Pa for 22 hours. After filtration, the solution was concentrated to a syrup and crystallized from acetonitrile to afford 6.66 g of the title compound, mp 168—172°C.

| | | | | |
|---|---|---|---|---|
| Analysis calculated for $C_9H_{20}N_2Cl_2$: | C, 47.58; | H, 8.86; | N, 12.33; | Cl, 31.21. |
| Found: | C, 47.70; | H, 8.58; | N, 12.39; | Cl, 30.92. |

**Claims for the Contracting States: BE CH DE FR GB IT LI LU NL SE**

1. A compound of the formula

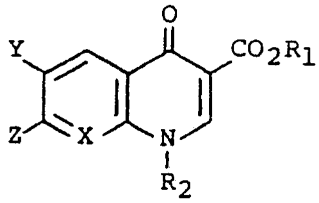

III

wherein

Z is

$$-Z'-(CR_5R_6)_{n'''}NR_3R_4,$$

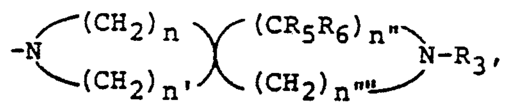 or 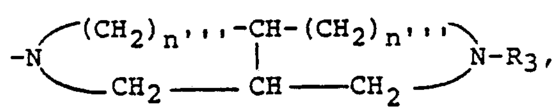

in which

Z' is

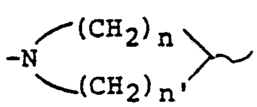 , 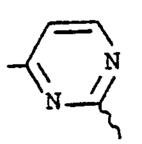 , 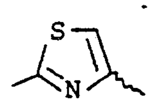 or 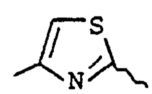 ;

X is CH, CCl, CF, C—OH, CO-alkyl having from one to three carbon atoms, or C—NH-alkyl having from one to three carbon atoms;

Y is hydrogen, fluorine, chlorine, or bromine;

n is 1, 2, 3 or 4;

n' is 1, 2, 3 or 4 wherein n+n' is a total of 2, 3, 4 or 5;

n'' is 0, 1, or 2;

n''' is 1 or 2; and n'''' is 0, 1 or 2;

$R_1$ is hydrogen, alkyl having from one to six carbon atoms or a cation;

$R_2$ is alkyl having from one to four carbon atoms, vinyl, haloalkyl, or hydroxyalkyl having from two to four carbon atoms or cycloalkyl having three to six carbon atoms;

$R_3$ is hydrogen, alkyl having from one to four carbon atoms or cycloalkyl having three to six carbon atoms;

$R_4$ is hydrogen, alkyl from one to four carbon atoms, hydroxyalkyl having two to four carbon atoms, trifluoroethyl, or $R_7CO$— wherein $R_7$ is alkyl having from one to four carbon atoms or alkoxy having from one to four carbon atoms; and

$R_5$ and $R_6$ are independently hydrogen or alkyl having from one to three carbon atoms;

with the proviso that, when X is CH and Z' is

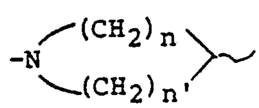

the sum of n+n' is other than 4;

and the pharmaceutically acceptable acid addition or base salts thereof.

2. A compound as claimed in claim 1, wherein Y is fluorine.

3. A compound as claimed in claim 1 or 2, wherein $R_2$ is ethyl, vinyl, or 2-fluoroethyl.

4. A compound as claimed in claim 1, 2 or 3, wherein X is CH or CF.

5. A compound as claimed in any preceding claim, wherein $R_1$ is hydrogen or a pharmaceutically acceptable acid addition or base salt thereof.

6. A compound as claimed in any preceding claim, wherein Z' is

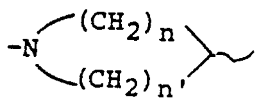 or 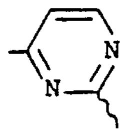 , or 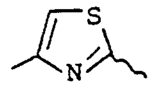 ;

or wherein Z is

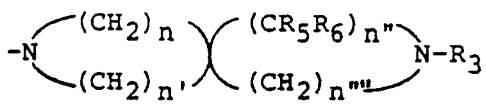,

7. A compound as claimed in claim 6, wherein Z is

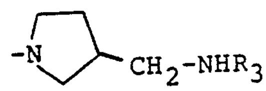

X is C—F and $R_3$ is hydrogen, methyl or ethyl.

8. A compound as claimed in claim 6, wherein Z is

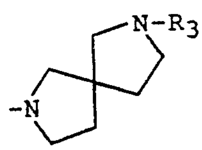

X is C—F and $R_3$ is hydrogen, methyl, or ethyl.

9. A compound as claimed in claim 7 or 8, being

7-[3-(aminoethyl)-1-pyrrolidinyl]-1-ethyl-6,8-difluoro-1,4-dihydro-4-oxo-3-quinolinecarboxylic acid, or

7-[3-(aminoethyl)-1-pyrrolidinyl]-6,8-difluoro-1-(2-fluoroethyl)-1,4-dihydro-4-oxo-3-quinolinecarboxylic acid, or

7-[3-(aminomethyl)-1-pyrrolidinyl]-6,8-difluoro-1-ethenyl-1,4-dihydro-4-oxo-3-quinolinecarboxylic acid, or

1-ethyl-7-[3-[(ethylamino)methyl]-1-pyrrolidinyl]-6,8-difluoro-1,4-dihydro-4-oxo-3-quinolinecarboxylic acid, or

7-[3-[(ethylamino)methyl]-1-pyrrolidinyl]-6,8-difluoro-1-(2-fluoroethyl)-1,4-dihydro-4-oxo-3-quinolinecarboxylic acid, or

7-[3-[(ethylamino)methyl-1-pyrrolidinyl]-6,8-difluoro-1-ethenyl-1,4-dihydro-4-oxo-3-quinolinecarboxylic acid, or

1-ethyl-7-[3-[[(1-methylethyl)amino]methyl]-1-pyrrolidinyl]-6,8-difluoro-1,4-dihydro-4-oxo-3-quinolinecarboxylic acid, or

1-ethyl-6,8-difluoro-1,4-dihydro-7-(7-methyl-2,7-diazaspiro[4.4]non-2-yl)-4-oxo-3-quinolinecarboxylic acid,

10. A process for the preparation of a compound as claimed in claim 1 and of the formula

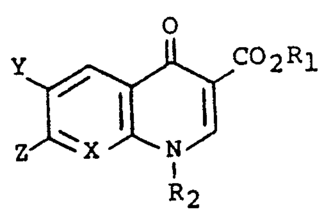

III

wherein Z is

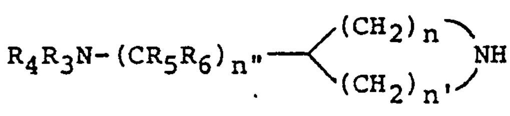
,
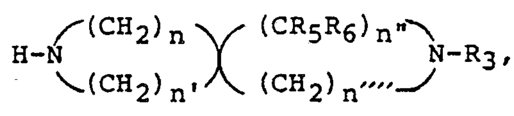

VIa VIb

or
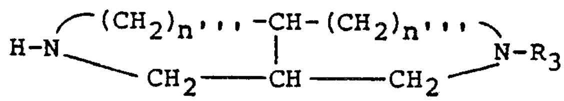

VIc

which comprises reacting a compound of the formula

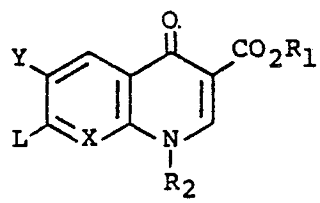
IV

wherein L is a leaving group and the other symbols are as defined in claim 1, with an amine corresponding to the group Z defined above, and, if desired, converting the resulting product to a pharmaceutically acceptable acid addition or base salt thereof by known methods.

11. A process for the preparation of a compound as claimed in claim 1 and of the formula

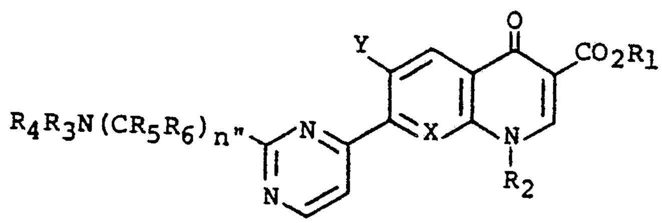
Ib

which comprises reacting a compound of the formula

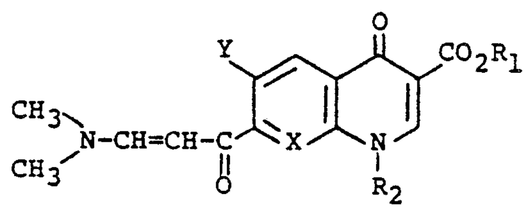
VII

with an amidine of the formula

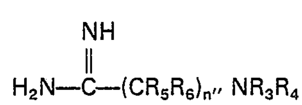

and, if desired, converting the resulting product to a pharmaceutically acceptable acid addition or base salt thereof by known methods.

12. A process for the preparation of a compound as claimed in claim 1 and of the formula

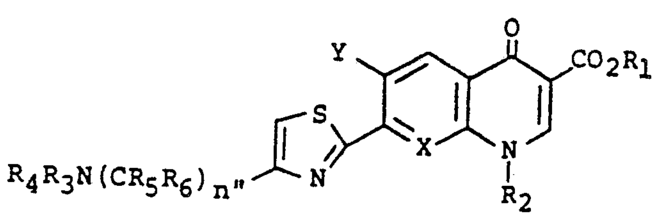
Ic

which comprises:

a) reacting a compound having the formula

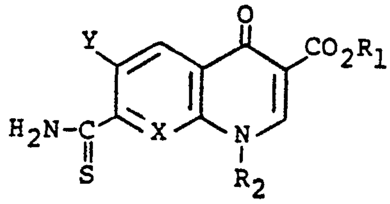

IX

with a compound of the formula

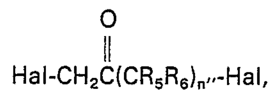

wherein n'' is 1 or 2 and Hal is halogen;

b) displacing the halogen atom of the resulting compound of the formula

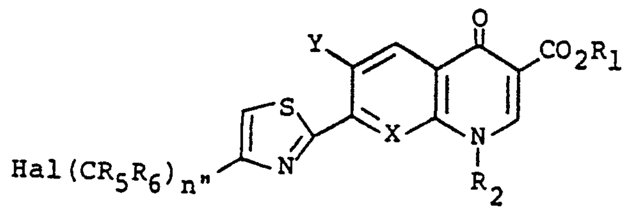

X

with an amino group of the formula $R_3R_4N—$ or with azide ion;

c) reducing the resulting azide group to a compound wherein $R_3$ and $R_4$ are hydrogen, and, if desired, alkylating the amino group with an alkyl halide in which alkyl has one to three carbon atoms, and, if desired, converting the resulting product to a pharmaceutically acceptable acid addition or base salt thereof by known methods.

13. A process for the preparation of a compound as claimed in claim 1 and of the formula

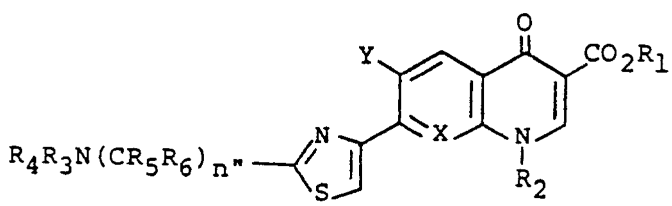

Id

which comprises reacting a compound of the formula

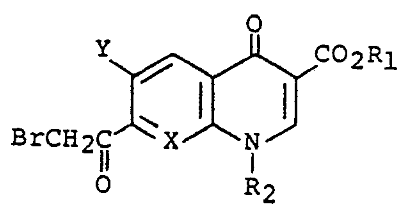

XI

with a thioamide for the formula

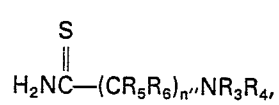

and, if desired, converting the resulting product to a pharmaceutically acceptable acid addition or base salt thereof by known methods.

14. A pharmaceutical composition comprising an anti-bacterially effective amount of a compound as claimed in any of claims 1 to 9 together with a pharmaceutically acceptable carrier.

**Claims for the Contracting State: AT**

1. A process for the preparation of a compound of the formula

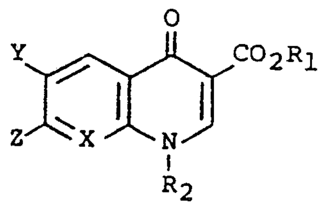

III

wherein
Z is

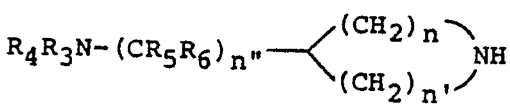
,
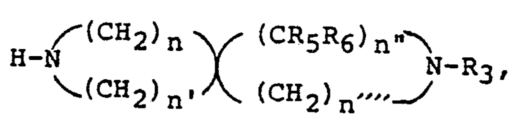

VIa VIb

or
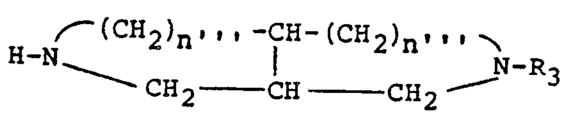

VIc

X is CH, CCl, CF, C—OH, CO-alkyl having from one to three carbon atoms, or C—NH-alkyl having from one to three carbon atoms;
Y is hydrogen, fluorine, chlorine, or bromine;
n is 1, 2, 3 or 4;
n′ is 1, 2, 3 or 4 wherein n+n′ is a total of 2, 3, 4 or 5;
n″ is 0, 1, or 2;
n‴ is 1 or 2; and n⁗ is 0, 1 or 2;
$R_1$ is hydrogen, alkyl having from one to six carbon atoms or a cation;
$R_2$ is alkyl having from one to four carbon atoms, vinyl, haloalkyl, or hydroxyalkyl having from two to four carbon atoms or cycloalkyl having three to six carbon atoms;
$R_3$ is hydrogen, alkyl having from one to four carbon atoms or cycloalkyl having three to six carbon atoms;
$R_4$ is hydrogen, alkyl from one to four carbon atoms, hydroxyalkyl having two to four carbon atoms, trifluoroethyl, or $R_7CO$— wherein $R_7$ is alkyl having from one to four carbon atoms or alkoxy having from one to four carbon atoms; and
$R_5$ and $R_6$ are independently hydrogen or alkyl having from one to three carbon atoms;
with the proviso that, when X is CH and Z is

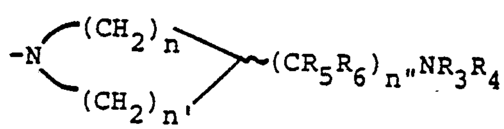
,

the sum of n+n′ is other than 4;
which comprises reacting a compound of the formula

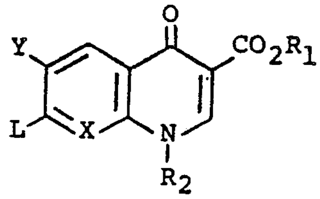

IV

wherein L is a leaving group and the other symbols are as defined in claim 1, with an amine corresponding to the group Z defined above, and, if desired, converting the resulting product to a pharmaceutically acceptable acid addition or base salt thereof by known methods.

2. A process for the preparation of a compound of the formula

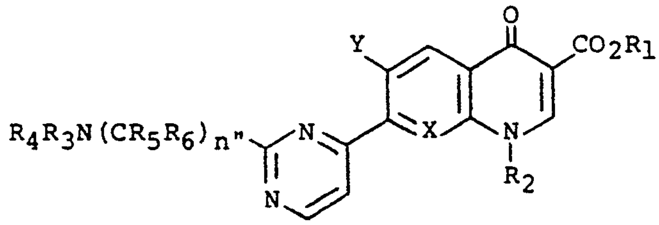

Ib

which comprises reacting a compound of the formula

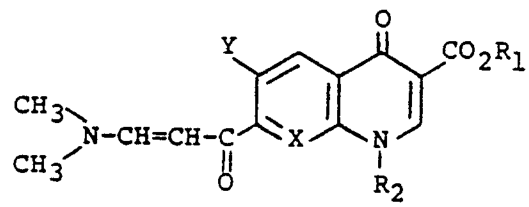

VII

with an amidine of the formula

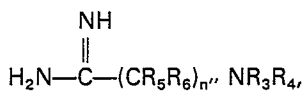

and, if desired, converting the resulting product to a pharmaceutically acceptable acid addition or base salt thereof by known methods, wherein $R_1$ to $R_6$, X, Y and n'' are as defined in claim 1.

3. A process for the preparation of a compound of the formula

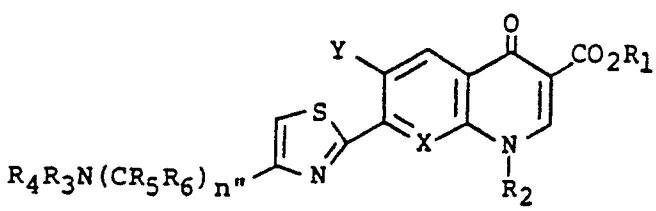

Ic

which comprises:

a) reacting a compound having the formula

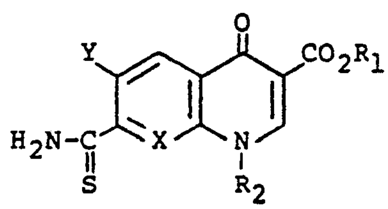

IX

with a compound of the formula

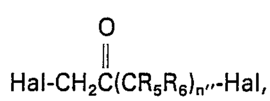

wherein n'' is 1 or 2 and Hal is halogen;

b) displacing the halogen atom of the resulting compound of the formula

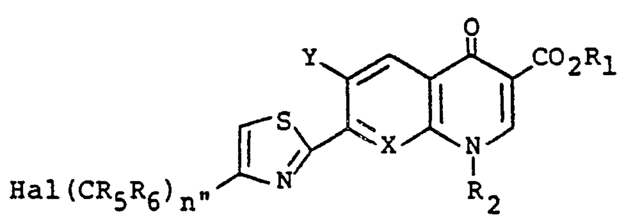

X

with an amino group of the formula $R_3R_4N$— or with azide ion;

c) reducing the resulting azide group to a compound wherein $R_3$ and $R_4$ are hydrogen, and, if desired, alkylating the amino group with an alkyl halide in which alkyl has one to three carbon atoms, and, if desired, converting the resulting product to a pharmaceutically acceptable acid addition or base salt thereof by known methods, wherein $R_1$ to $R_6$, X, Y and n'' are as defined in claim 1.

4. A process for the preparation of a compound of the formula

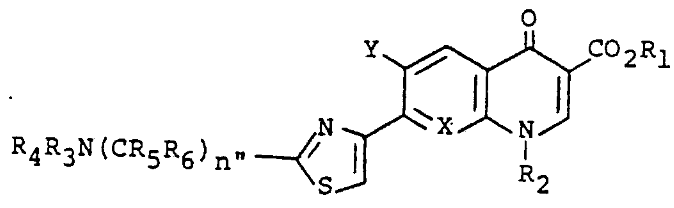

Id

which comprises reacting a compound of the formula

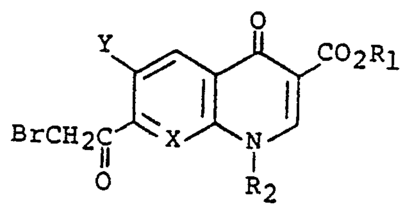

XI

with a thioamide for the formula

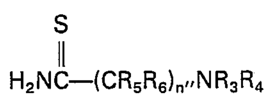

and if desired, converting the resulting product to a pharmaceutically acceptable acid addition or base salt thereof by known methods, wherein $R_1$ to $R_6$, X, Y and n'' are as defined in claim 1.

5. A process as claimed in any preceding claim, wherein Y is fluorine.

6. A process as claimed in any of claims 1 to 5, wherein $R_2$ is ethyl, vinyl, or 2-fluoroethyl.

7. A process as claimed in any preceding claim wherein X is CH or CF.

8. A process as claimed in any preceding claim, wherein $R_1$ is hydrogen or a pharmaceutically acceptable acid addition or base salt thereof.

9. A process as claimed in claim 1, wherein Z is

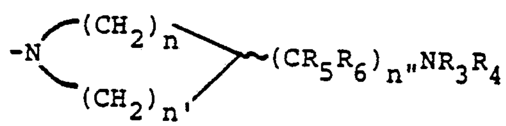 or 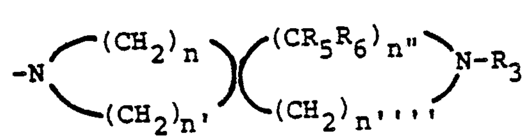

10. A process as claimed in claim 9, wherein Z is

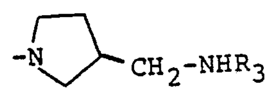

X is C—F and $R_3$ is hydrogen, methyl or ethyl.

11. A process as claimed in claim 9, wherein Z is

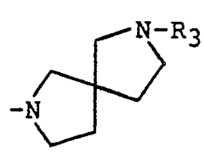 ;

X is C—F and $R_3$ is hydrogen, methyl, or ethyl.

12. A process as claimed in claim 10 or 11, for producing

7-[3-(aminomethyl)-1-pyrrolidinyl]-1-ethyl-6,8-difluoro-1,4-dihydro-4-oxo-3-quinoline carboxylic acid, or

7-[3-(aminomethyl)-1-pyrrolidinyl]-6,8-difluoro-1-(2-fluoroethyl)-1,4-dihydro-4-oxo-3-quinolinecarboxylic acid, or

7-[3-(aminomethyl)-1-pyrrolidinyl]-6,8-difluoro-1-ethenyl-1,4-dihydro-4-oxo-3-quinolinecarboxylic acid, or

1-ethyl-7-[3-(ethylamino)methyl]-1-pyrrolidinyl]-6,8-difluoro-1,4-dihydro-4-oxo-3-quinolinecarboxylic acid, or

7-[3-[(ethylamino)methyl]-1-pyrrolidinyl]-6,8-difluoro-1-(2-fluoroethyl)-1,4-dihydro-4-oxo-3-quinolinecarboxylic acid, or

7-[3-[(ethylamino)methyl-1-pyrrolidinyl]-6,8-difluoro-1-ethenyl-1,4-dihydro-4-oxo-3-quinolinecarboxylic acid, or

1-ethyl-7-[3-[[(1-methylethyl)amino]methyl]-1-pyrrolidinyl]-6,8-difluoro-1,4-dihydro-4-oxo-3-quinolinecarboxylic acid, or

1-ethyl-6,8-difluoro-1,4-dihydro-7-(7-methyl-2,7-diazaspiro[4.4]non-2-yl)-4-oxo-3-quinolinecarboxylic acid.

13. A process for preparing a pharmaceutical composition, which comprises incorporating an antibacterially effective amount of a compound prepared by a process as claimed in any preceding claim into a pharmaceutically acceptable carrier.

**Patentansprüche für die Vertragsstaaten: BE CH DE FR GB IT LI LU NL SE**

1. Verbindung der Formel

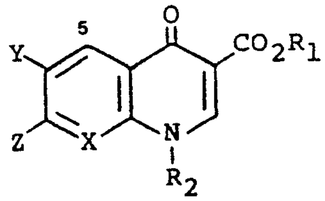

I

worin Z für

$$-Z'\sim(CR_5R_6)_{n'''}NR_3R_4,$$

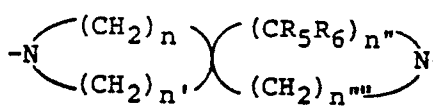

oder

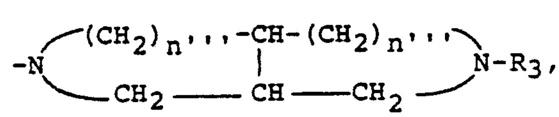

worin Z' für

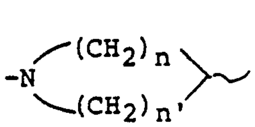

,

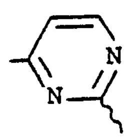

,

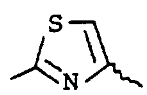

oder

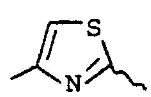

steht;

X für CH, CCl, CF, C—OH, CO-Alkyl mit 1 bis 3 Kohlenstoffatomen oder C—NH-Alkyl mit 1 bis 3 Kohlenstoffatomen steht;

Y Wasserstoff, Fluor, Chlor oder Brom ist;

n für 1, 2, 3 oder 4 steht;

n' für 1, 2, 3 oder 4, worin n + n' eine Summe von 2, 3, 4 oder 5 ist, steht;

n'' für 0, 1 oder 2 steht;

n''' und n'''' 1 oder 2 darstellt; für 0, 1 oder 2 steht;

$R_1$ Wasserstoff, Alkyl mit 1 bis 6 Kohlenstoffatomen oder ein Kation repräsentiert;

$R_2$ Alkyl mit 1 bis 4 Kohlenstoffatomen, vinyl, Halogenalkyl oder Hydroxyalkyl mit 2 bis 4 Kohlenstoffatomen oder Cycloalkyl mit 3 bis 6 Kohlenstoffatomen bedeutet;

$R_3$ Wasserstoff, Alkyl mit 1 bis 4 Kohlenstoffatomen oder Cycloalkyl mit 3 bis 6 Kohlenstoffatomen darstellt;

$R_4$ Wasserstoff, Alkyl mit 1 bis 4 Kohlenstoffatomen, Hydroxyalkyl mit 2 bis 4 Kohlenstoffatomen, Trifluoräthyl oder $R_7CO$—, worin $R_7$ Alkyl mit 1 bis 4 Kohlenstoffatomen oder Alkoxy mit 1 bis 4 Kohlenstoffatomen ist, bedeutet; und

$R_5$ und $R_6$ unabhängig voneinander Wasserstoff oder Alkyl mit 1 bis 3 Kohlenstoffatomen darstellen; mit der Maßgabe, daß wenn X CH ist, und Z für

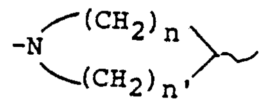

steht, die Summe von n + n' eine andere Zahl als 4 ist; und die pharmazeutisch akzeptablen Säureadditions- oder Basensalze derselben.

2. Verbindung nach Anspruch 1, worin Y für Fluor steht.

3. Verbindung nach Anspruch 1 oder 2, worin $R_2$ für Äthyl, Vinyl oder 2-Fluoräthyl steht.

4. Verbindung nach Anspruch 1, 2 oder 3, worin X für CH oder CF steht.

5. Verbindung nach irgendeinem der vorhergehenden Ansprüche, worin $R_1$ Wasserstoff oder ein pharmazeutisch akzeptables Säureadditions- oder Basensalz davon ist.

6. Verbindung nach irgendeinem der vorhergehenden Ansprüche, worin Z'

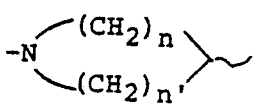 oder 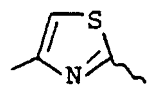 oder 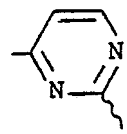 ,

ist, oder worin Z

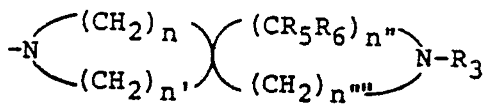

bedeutet.

7. Verbindung nach Anspruch 6, worin Z für

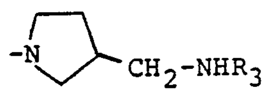

steht, X für C—F steht, und $R_3$ Wasserstoff, Methyl oder Äthyl bedeutet.

8. Verbindung nach Anspruch 6, worin Z für

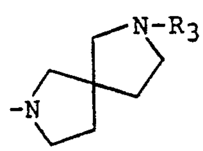

steht, X für C—F steht, und $R_3$ Wasserstoff, Methyl oder Äthyl darstellt.

9. Verbindung nach Anspruch 7 oder 8 mit dem Namen:

7-[3-(Aminomethyl)-1-pyrrolidinyl]-1-äthyl-6,8-difluor-1,4-dihydro-4-oxo-3-chinolincarbonsäure oder

7-[3-(Aminomethyl)-1-pyrrolidinyl]-6,8-difluor-1-(2-fluoräthyl)-1,4-dihydro-4-oxo-3-chinolin-carbonsäure oder

7-[3-(Aminomethyl)-1-pyrrolidinyl]-6,8-difluor-1-äthenyl-1,4-dihydro-4-oxo-3-chinolin-carbonsäure oder

1-Äthyl-7-[3-(äthylamino)-methyl]-1-pyrrolidinyl]-6,8-difluor-1,4-dihydro-4-oxo-3-chinolincarbonsäure oder

7-[3-[(Äthylamino)-methyl]-1-pyrrolidinyl]-6,8-difluor-1-(2-fluoroäthyl)-1,4-dihydro-4-oxo-3-chinolin-carbonsäure oder

7-[3-[(Äthylamino)-methyl]-1-pyrrolidinyl]-6,8-difluor-1-äthenyl-1,4-dihydro-4-oxo-3-chinolin-carbonsäure oder

1-Äthyl-7-[3-[[(1-methyläthyl)-amino]-methyl]-1-pyrrolidinyl-6,8-difluor-1,4-dihydro-4-oxo-3-chinolin-carbonsäure oder.

1-Äthyl-6,8-difluor-1,4-dihydro-7-(7-methyl-2,7-diazaspiro[4.4]non-2-yl)-4-oxo-3-chinolincarbonsäure.

10. Verfahren zur Herstellung einer Verbindung nach Anspruch 1 und mit der Formel

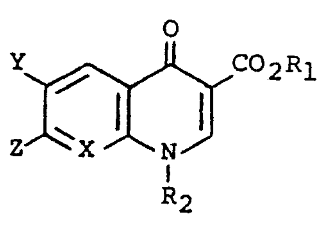

III

worin Z

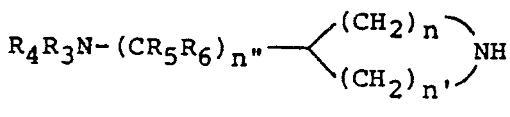

,

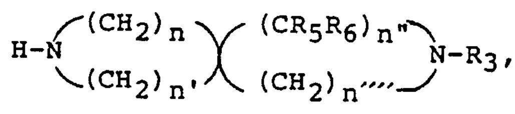

VIa VIb

or

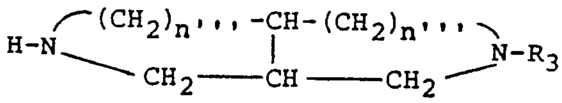

VIc

bedeutet, welches die Umsetzung einer Verbindung der Formel

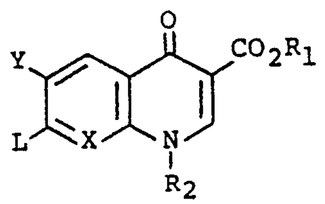

IV

worin L eine austretende Gruppe ist, und die anderen Symbole wie im Anspruch 1 definiert sind, mit einem Amin, welches der oben definierten Gruppe Z entspricht, und gewünschtenfalls die Überführung des resultierenden Produkts in ein pharmazeutisch akzeptables Säureadditions- oder Basensalz davon mittels bekannter Verfahren umfaßt.

11. Verfahren zur Herstellung einer Verbindung nach Anspruch 1 und mit der Formel

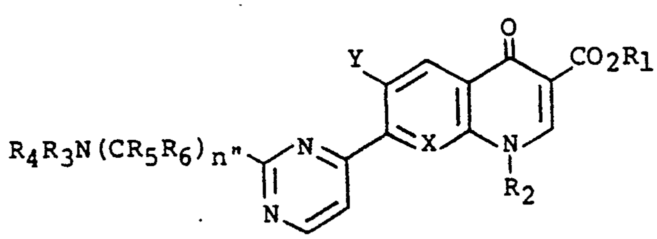

Ib

welches die Umsetzung einer Verbindung der Formel

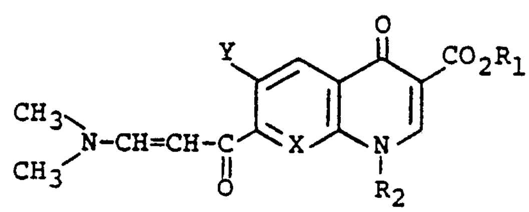

VII

mit einem Amidin der Formel

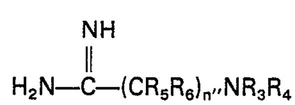

und gewünschtenfalls die Überführung des resultierenden Produkts in ein pharmazeutisch akzeptables Säureadditions- oder Basensalz davon mittels bekannter Verfahren umfaßt.

12. Verfahren zur Herstellung einer Verbindung nach Anspruch 1 und mit der Formel

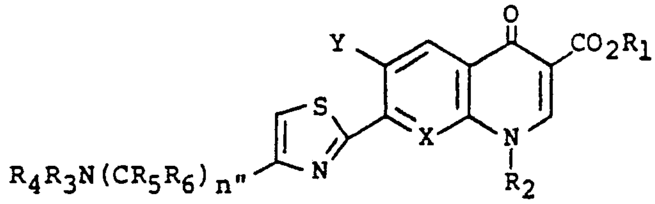

Ic

welches umfaßt:

a) Umsetzung einer Verbindung der Formel

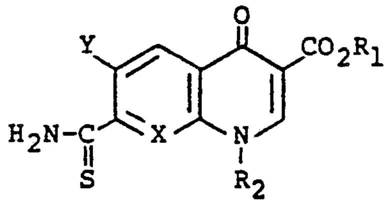

IX

mit einer Verbindung der Formel

$$\mathrm{Hal{-}CH_2\overset{O}{\overset{\|}{C}}(CR_5R_6)_{n''}{-}Hal},$$

worin n'' 1 oder 2 ist, und Hal für Halogen steht;

b) Substituieren des Halogenatoms der resultierenden Verbindung der Formel

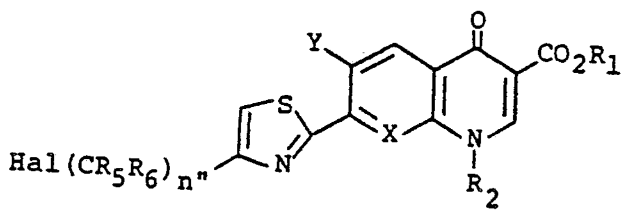

X

mit einer Aminogruppe der Formel $R_3R_4N$— oder mit einem Azidion;

c) Reduzieren der resultierenden Azidgruppe zu einer Verbindung, worin $R_3$ und $R_4$ Wasserstoff sind, und gewünschtenfalls Alkylieren der Aminogruppe mit einem Alkylhalogenid, in welchem Alkyl 1 bis 3 Kohlenstoffatome hat, und gewünschtenfalls Überführung des resultierenden Produkts in ein pharmazeutisch akzeptables Säureadditions- oder Basensalz davon mittels bekannter Verfahren.

13. Verfahren zur Herstellung einer Verbindung nach Anspruch 1 und mit der Formel

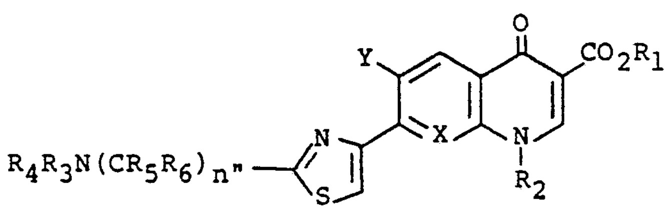

Id

welches die Reaktion einer Verbindung der Formel

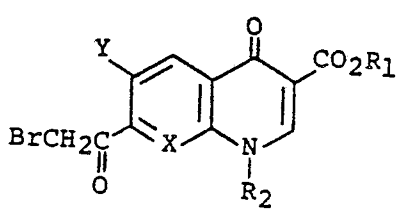

XI

mit einem Thioamid der Formel

$$\mathrm{H_2N\overset{S}{\overset{\|}{C}}{-}(CR_5R_6)_{n''}NR_3R_4}$$

und gewünschtenfalls Überführung des resultierenden Produkts in ein pharmazeutisch akzeptables Säureadditions- oder Basensalz davon mittels bekannter Verfahren umfaßt.

14. Pharmazeutische Zusammensetzung, welche eine antibakteriell wirksame Menge einer Verbindung nach irgendeinem der Ansprüche 1 bis 9 zusammen mit einem pharmazeutisch akzeptablen Träger umfaßt.

**Patentansprüche für den Vertragsstaat: AT**

1. Verfahren zur Herstellung einer Verbindung der Formel

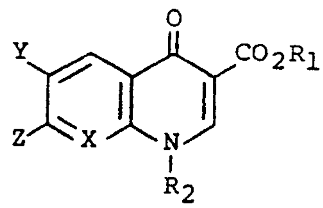

III

worin Z' für

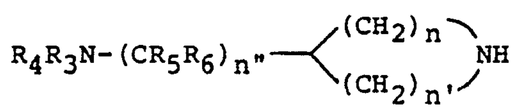

,

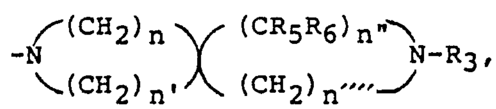

VIa VIb

oder

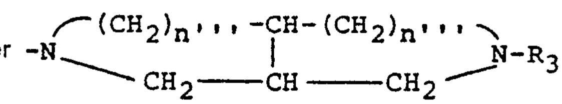

VIc

steht, X für CH, CCl, CF, C—OH, CO-Alkyl mit 1 bis 3 Kohlenstoffatomen oder C—NH-Alkyl mit 1 bis 3 Kohlenstoffatomen steht;

Y Wasserstoff, Fluor, Chlor oder Brom ist;

n für 1, 2, 3 oder 4 steht;

n' für 1, 2, 3 oder 4, worin n + n' eine Summe von 2, 3, 4 oder 5 ist, steht;

n'' für 0, 1 oder 2 steht;

n''' 1 oder 2 darstellt;

und n'''' für 0, 1 oder 2 steht;

$R_1$ Wasserstoff, Alkyl mit 1 bis 6 Kohlenstoffatomen oder ein Kation repräsentiert;

$R_2$ Alkyl mit 1 bis 4 Kohlenstoffatomen, Vinyl, Halogenalkyl oder Hydroxyalkyl mit 2 bis 4 Kohlenstoffatomen oder Cycloalkyl mit 3 bis 6 Kohlenstoffatomen bedeutet;

$R_3$ Wasserstoff, Alkyl mit 1 bis 4 Kohlenstoffatomen oder Cycloalkyl mit 3 bis 6 Kohlenstoffatomen repräsentiert;

$R_4$ Wasserstoff, Alkyl mit 1 bis 4 Kohlenstoffatomen, Hydroxyalkyl mit 2 bis 4 Kohlenstoffatomen, Trifluoräthyl oder $R_7CO$—, worin $R_7$ Alkyl mit 1 bis 4 Kohlenstoffatomen oder Alkoxy mit 1 bis 4 Kohlenstoffatomen ist, bedeutet;

$R_5$ und $R_6$ unabhängig voneinander Wasserstoff oder Alkyl mit 1 bis 3 Kohlenstoffatomen sind;

mit der Maßgabe, daß, wenn X für CH steht, und Z

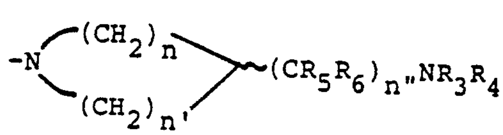

ist, die Summe von n + n' eine andere Zahl als 4 ist;

und der pharmazeutisch akzeptablen Säureadditions- oder Basensalze davon; welches die Umsetzung einer Verbindung der Formel

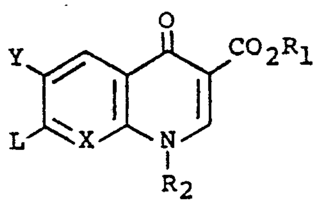

IV

worin L eine austretende Gruppe ist, und die anderen Symbole wie im Anspruch 1 definiert sind, mit einem Amin, welches der oben definierten Gruppe Z entspricht, und gewünschtenfalls die Überführung des resultierenden Produkts in ein pharmazeutisch akzeptables Säureadditions- oder Basensalz davon mittels bekannter Methoden umfaßt.

2. Verfahren zur Herstellung einer Verbindung der Formel

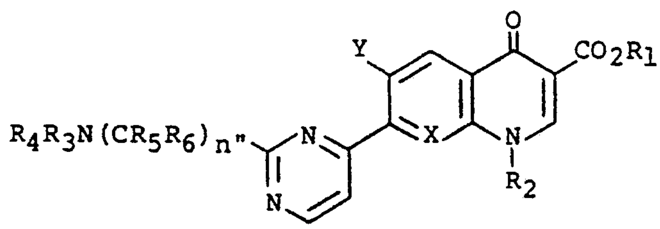

Ib

welches die Reaktion einer Verbindung der Formel

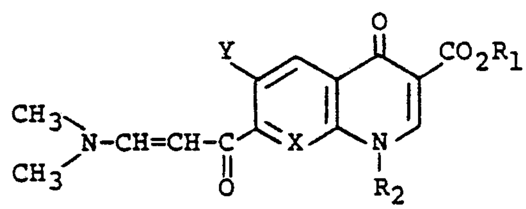

VII

mit einem Amidin der Formel

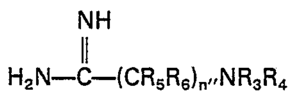

und gewünschtenfalls Überführen des resultierenden Produkts in ein pharmazeutisch akzeptables Säureadditions- oder Basensalz davon mittels bekannter Methoden, worin $R_1$ bis $R_6$, X, Y und N'' wie im Anspruch 1 definiert sind.

3. Verfahren zur Herstellung einer Verbindung der Formel

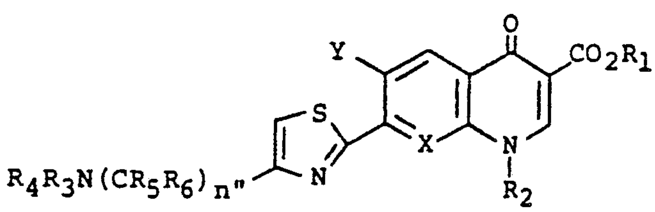

Ic

welches umfaßt:

a) Reaktion einer Verbindung der Formel

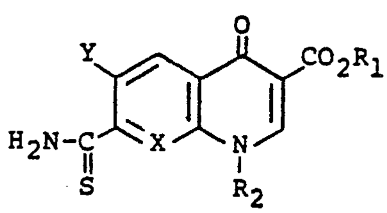

IX

mit einer Verbindung der Formel

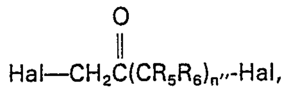

worin n'' 1 oder 2 bedeutet, und Hal für Halogen steht;

b) Substitution des Halogenatoms der resultierenden Verbindung der Formel

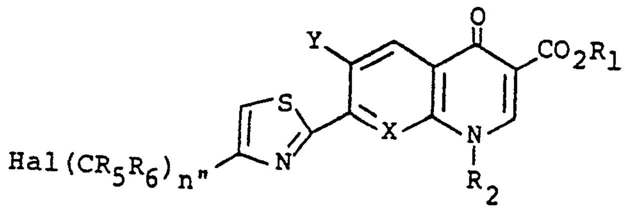

X

mit einer Aminogruppe der Formel $R_3R_4N$— oder mit einem Azidion;

c) Reduktion der resultierenden Azidgruppe zu einer Verbindung, worin $R_3$ und $R_4$ Wasserstoff sind, und gewünschtenfalls Alkylieren der Aminogruppe mit einem Alkylhalogenid, in welchem Alkyl 1 bis 3 Kohlenstoffatome aufweist, und gewünschtenfalls Überführung des resultierenden Produkts in ein pharmazeutisch akzeptables Säureadditions- oder Basensalz davon mittels bekannter Methoden, worin $R_1$ bis $R_6$, Z, Y und n'' wie im Anspruch 1 definiert sind.

4. Verfahren zur Herstellung einer Verbindung der Formel

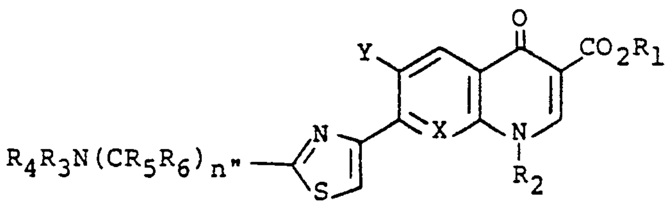

Id

welches die Reaktion einer Verbindung der Formel

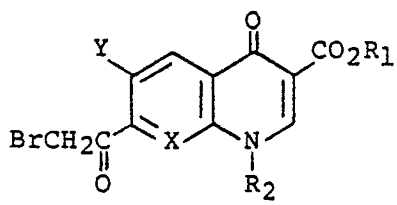

XI

mit einem Thioamid der Formel

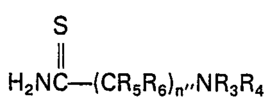

und gewünschtenfalls Überführung des resultierenden Produkts in ein pharmazeutisch akzeptables Säureadditions- oder Basensalz davon mittels bekannter Methoden, worin $R_1$ bis $R_6$, X, Y und n'' wie im Anspruch 1 definiert sind, umfaßt.

5. Verfahren nach irgendeinem vorhergehenden Anspruch, worin Y für Fluor steht.

6. Verfahren nach irgendeinem der Ansprüche 1 bis 5, worin $R_2$ für Äthyl, Vinyl oder 2-Fluoräthyl steht.

7. Verfahren nach irgendeinem vorhergehenden Anspruch, worin X für CH oder CF steht.

8. Verfahren nach irgendeinem vorhergehenden Anspruch, worin $R_1$ Wasserstoff oder ein pharmazeutisch akzeptables Säureadditions- oder Basensalz davon bedeutet.

9. Verfahren nach Anspruch 1, worin Z

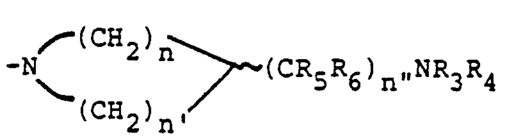

oder

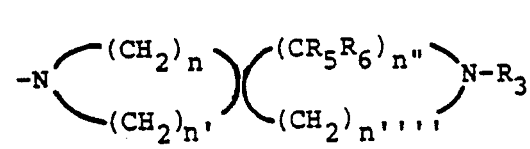

bedeutet.

10. Verbindung nach Anspruch 9, worin Z für

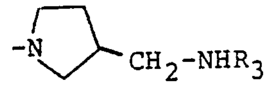

steht, X für C—F steht, und $R_3$ Wasserstoff, Methyl oder Äthyl bedeutet.

11. Verbindung nach Anspruch 9, worin Z

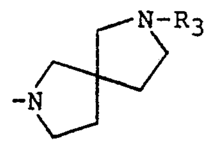

bedeutet; X für C—F steht, und $R_3$ Wasserstoff, Methyl oder Äthyl repräsentiert.

12. Verfahren nach Anspruch 10 oder 11 zur Herstellung von

7-[3-(Aminomethyl)-1-pyrrolidinyl]-1-äthyl-6,8-difluor-1,4-dihydro-4-oxo-3-chinolincarbonsäure oder

7-[3-(Aminomethyl)-1-pyrrolidinyl]-6,8-difluor-1-(2-fluoräthyl)-1,4-dihydro-4-oxo-3-chinolincarbonsäure oder

7-[3-(Aminomethyl)-1-pyrrolidinyl]-6,8-difluor-1-äthenyl-1,4-dihydro-4-oxo-3-chinolin-carbonsäure oder

1-Äthyl-7-[3-(äthylamino)-methyl]-1-pyrrolidinyl]-6,8-difluor-1,4-dihydro-4-oxo-3-chinolincarbonsäure oder

7-[3-[(Äthylamino)-methyl]-1-pyrrolidinyl]-6,8-difluor-1-(2-fluoroäthyl)-1,4-dihydro-4-oxo-3-chinolincarbonsäure oder

7-[3-[(Äthylamino)-methyl]-1-pyrrolidinyl]-6,8-difluor-1-äthenyl-1,4-dihydro-4-oxo-3-chinolincarbonsäure oder

1-Äthyl-7-[3-[[(1-methyläthyl)-amino]-methyl]-1-pyrrolidinyl-6,8-difluor-1,4-dihydro-4-oxo-3-chinolincarbonsäure oder

1-Äthyl-6,8-difluor-1,4-dihydro-7-(7-methyl-2,7-diazaspiro[4.4]non-2-yl)-4-oxo-3-chinolincarbonsäure.

13. Verfahren zur Herstellung einer pharmazeutischen Zusammensetzung, welches die Inkorporierung einer antibakteriell wirksamen Menge einer Verbindung, welche mittels eines Verfahrens nach irgendeinem vorhergehenden Anspruch hergestellt wurde, in einen pharmazeutisch akzeptablen Träger umfaßt.

**Revendications pour les Etats contractants: BE CH DE FR GB IT LI LU NL SE**

1. Un composé de formule:

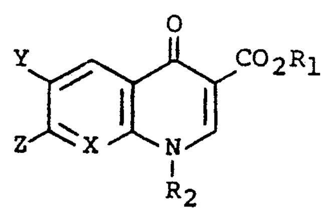

III

dans laquelle:

Z est

$$-Z'\sim(CR_5R_6)_{n''}NR_3R_4,$$

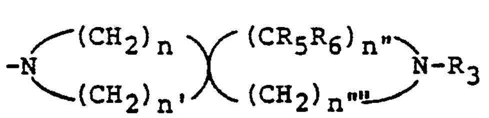

ou

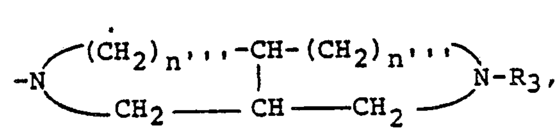

dans lesquels Z' est

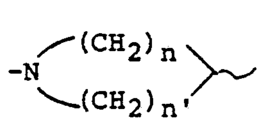

,

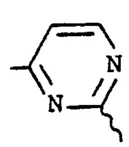

,

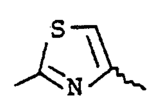

ou

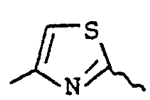

;

X est un groupe CH, CCl, CF, C—OH, CO-alkyle comprenant de 1 à 3 atomes de carbone, ou C—NH-alkyle présentant de 1 à 3 atomes de carbone;

Y est un atome d'hydrogène, de fluor, de chlore ou de brome;
n est égal à 1, 2, 3 ou 4;
n' est égal à 1, 2, 3 ou 4, étant entendu que n + n' égal un total de 3, 4 ou 5;
n'' est 0, 1 ou 2;
n''' est 1 ou 2; et
n'''' est 0, 1 ou 2;
$R_1$ est un atome d'hydrogène, un radical alkyle comprenant de 1 à 6 atomes de carbone ou un cation;
$R_2$ est un radical alkyle comprenant de 1 à 4 atomes de carbone, un radical vinyle, haloalkyle ou hydroxyalkyle comprenant de 2 à 4 atomes de carbone, ou cycloalkyle comprenant de 3 à 6 atomes de carbone;
$R_3$ est un atome d'hydrogène, un radical alkyle comprenant de 1 à 4 atomes de carbone ou cycloalkyle comprenant de 3 à 6 atomes de carbone;
$R_4$ est un atome d'hydrogène ou un radical alkyle comprenant de 1 à 4 atomes de carbone; hydroxyalkyle comprenant de 2 à 4 atomes de carbone, trifluoroéthyle ou $R_7CO$— dans lequel $R_7$ est un radical alkyle comprenant de 1 à 4 atomes de carbone ou alkoxy comprenant de 1 à 4 atomes de carbone;
$R_5$ et $R_6$, indépendamment l'un de l'autre, sot un atome d'hydrogène ou un radical alkyle comportant de 1 à 3 atomes de carbone;
étant entendu que lorsque X est CH et Z' est

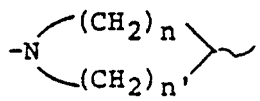

la somme de n + n' est différente de 4;
ainsi que les sels de base ou d'addition d'acides pharmaceutiquement acceptables en dérivant.

2. Un composé ainsi que revendiqué dans la revendication 1, dans lequel Y est un atome de fluor.

3. Un composé ainsi que revendiqué dans la revendication 1 ou 2, dans lequel $R_2$ est un radical éthyle, vinyle ou 2-fluoroéthyle.

4. Un composé ainsi que revendiqué dans la revendication 1, 2 ou 3, dans lequel X est CH ou CF.

5. Un composé ainsi que revendiqué dans l'une quelconque des revendications précédentes, dans lequel $R_1$ est un atome d'hydrogène ou qui consiste en un sel de base ou d'addition d'acide pharmaceutiquement acceptable en dérivant.

6. Un composé ainsi que revendiqué dans l'une quelconque des revendications précédentes, dans lequel Z' est

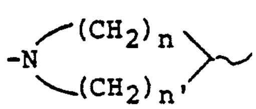 ou 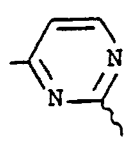 , ou 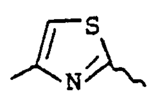 ;

ou dans lequel Z est

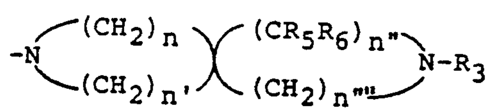

7. Un composé ainsi que revendiqué dans la revendication 6, dans lequel Z est

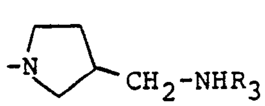

X étant C—F et $R_3$ est un atome d'hydrogène, un radical méthyle ou éthyle.

8. Un composé ainsi que revendiqué dans la revendication 6, dans lequel Z est

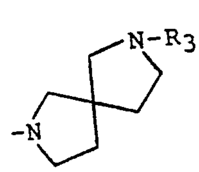

X est C—F et $R_3$ est un atome d'hydrogène, un radical méthyle ou éthyle.

9. Un composé ainsi que revendiqué dans les revendications 7 ou 8, consistant en:

acide 7-[3-(aminométhyl)-1-pyrrolidinyl]-1-éthyl-6,8-difluoro-1,4-dihydro-4-oxo-3-quinolinecarboxylique, ou

acide 7-[3-(aminométhyl)-1-pyrrolidinyl]-6,8-difluoro-1-(2-fluoroéthyl)-1,4-dihydro-4-oxo-3-quinolinecarboxylique, ou

acide 7-[3-(aminométhyl)-1-pyrrolidinyl]-6,8-difluoro-1-éthényl-1,4-dihydro-4-oxo-3-quinolinecarboxylique, ou

acide 1-éthyl-7-[3-[(éthylamino)méthyl]-1-pyrrolidinyl]-6,8-difluoro-1,4-dihydro-4-oxo-3-quinolinecarboxylique, ou

acide 7-[3-[(éthylamino)méthyl]-1-pyrrolidinyl]-6,8-difluoro-1-(2-fluoroéthyl)-1,4-dihydro-4-oxo-3-quinolinecarboxylique, ou

acide 7-[3-[(éthylamino)méthyl]-1-pyrrolidinyl]-6,8-difluoro-1-éthényl-1,4-dihydro-4-oxo-3-quinolinecarboxylique, ou

acide 1-éthyl-7-[3-[[(1-méthyléthyl)amino]méthyl]-1-pyrrolidinyl]-6,8-difluoro-1,4-dihydro-4-oxo-3-quinolinecarboxylique, ou

acide 1-éthyl-6,8-difluoro-1,4-dihydro-7-(7-méthyl-2,7-diazaspiro[4,4]non-2-yl)-4-oxo-3-quinolinecarboxylique.

10. Un procédé de préparation d'un composé ainsi que revendiqué dans la revendication 1, présentant la formule:

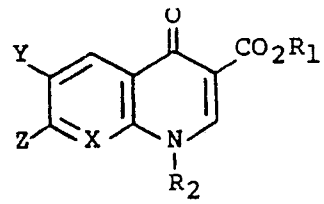

III

dans laquelle:

Z est

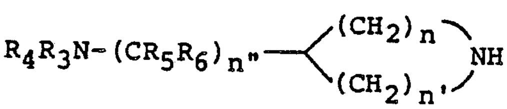

,

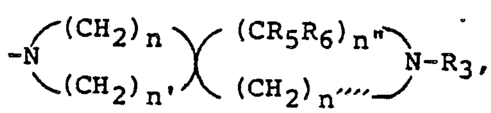

VIa

VIb

ou

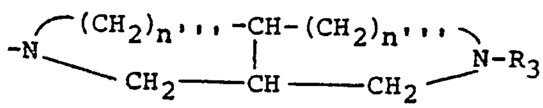

VIc

qui consiste à faire réagir un composé de formule:

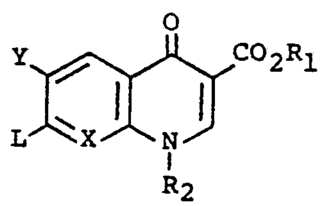

IV

dans laquelle L est un groupe sécant et les autres symboles sont tels que définis dans la revendication 1, avec une amine correspondant au groupe Z défini ci-dessus et, le cas échéant, à convertir le produit résultant en un sel de base ou d'addition d'acide pharmaceutiquement acceptable en dérivant par des méthodes connues.

11. Un procédé de préparation d'un composé ainsi que revendiqué dans la revendication 1 et présentant la formule:

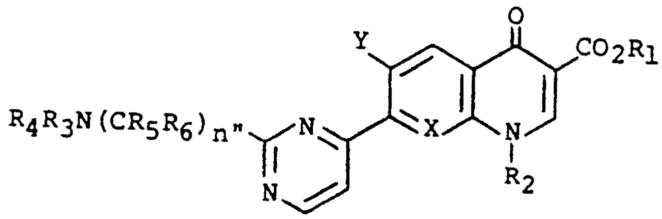

Ib

qui consiste à faire réagir un composé de formule:

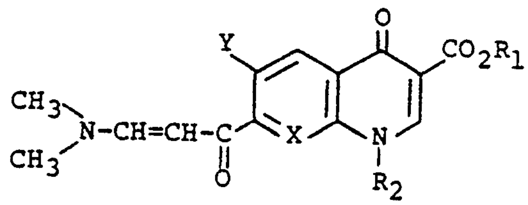

VII

avec une amidine de formule:

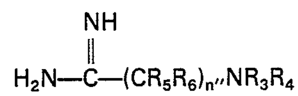

et, le cas échéant, à convertir le produit résultant en un sel de base ou d'addition d'acide pharmaceutiquement acceptable en dérivant par des méthodes connues.

12. Un procédé de préparation d'un composé ainsi que revendiqué dans la revendication 1 et présentant la formule:

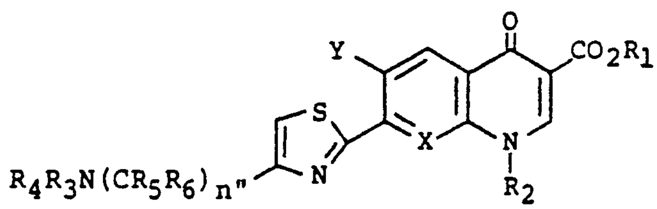

Ic

qui consiste:

a) à faire réagir un composé de formule:

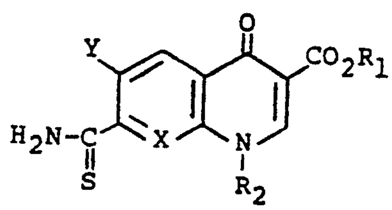

IX

avec un composé de formule:

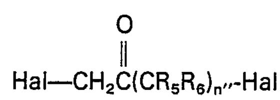

dans laquelle:

n'' est égal à 1 ou 2; et

Hal est un halogène;

b) à déplacer l'atome d'halogène du composé résultant de formule:

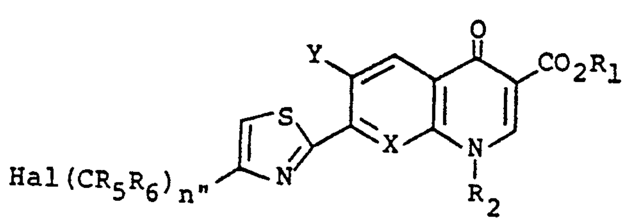

X

par un groupe amino de formule $R_3R_4N$— ou par un ion azide;

c) à réduire le groupe azide résultant en un composé dans lequel $R_3$ et $R_4$ sont des atomes d'hydrogène et, les cas échéant, à alkyler le groupe amino par un halogénure d'alkyle dans lequel le radical alkyle comprend de 1 à 3 atomes de carbone, et éventuellement à convertir le produit résultant en une sel de base ou d'addition d'acide pharmaceutiquement acceptable en dérivant par des méthodes connues.

13. Un procédé de préparation d'un composé ainsi que revendiqué dans la revendiction 1 et présentant la formule:

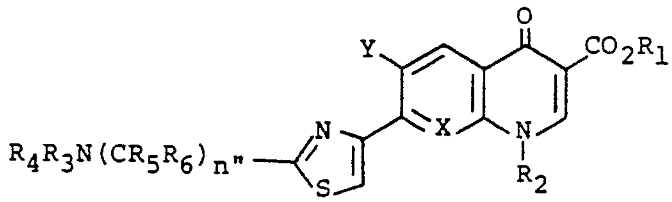

Id

qui consiste à faire réagir un composé de formule:

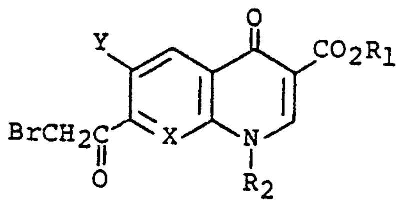

XI

avec un thioamide de formule:

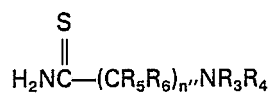

et, le cas échéant, à convertir le produit résultant en un sel de base ou d'addition d'acide pharmaceutiquement acceptable en dérivant par des méthodes connues.

14. Une composition pharmaceutique comprenant une quantité efficace du point de vue antibactérien d'un composé ainsi que revendiqué dans l'une quelconque des revendications 1 à 9 précitées, associé à un support pharmaceutiquement acceptable.

**Revendications pour l'Etat contractant: AT**

1. Un procédé pour la préparation d'un composé de formule:

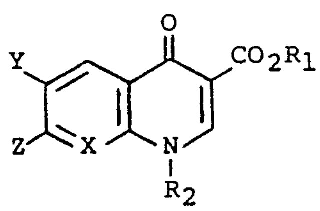

III

dans laquelle:

Z est

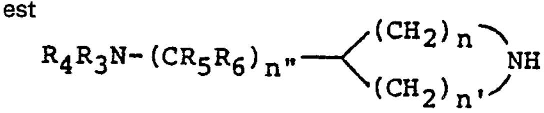

,

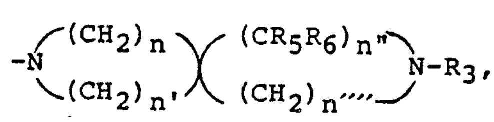

VIa

VIb

ou

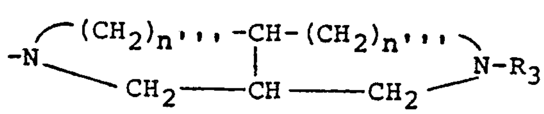

VIc

X est un groupe CH, CCl, CF, C—OH, CO-alkyle comprenant de 1 à 3 atomes de carbone, ou C—NH-alkyle presentant de 1 à 3 atomes de carbone;

Y est un atome d'hydrogène, de fluor, de chlore ou de brome;
n est égal à 1, 2, 3 ou 4;
n' est égal à 1, 2, 3 ou 4, étant entendu que n + n' égal un total de 3, 4 ou 5;
n'' est 0, 1 ou 2;
n''' est 1 ou 2; et
n'''' est 0, 1 ou 2;
$R_1$ est un atome d'hydrogène, un radical alkyle comprenant de 1 à 6 atomes de carbone ou un cation;

$R_2$ est un radical alkyle comprenant de 1 à 4 atomes de carbone, un radical vinyle, haloalkyle ou hydroxyalkyle comprenant de 2 à 4 atomes de carbone, ou cycloalkyle comprenant de 3 à 6 atomes de carbone;

$R_3$ est un atome d'hydrogène, un radical alkyle comprenant de 1 à 4 atomes de carbone ou cycloalkyle comprenant de 3 à 6 atomes de carbone;

$R_4$ est un atome d'hydrogène ou un radical alkyle comprenant de 1 à 4 atomes de carbone; hydroxyalkyle comprenant de 2 à 4 atomes de carbone, trifluoroéthyle ou $R_7CO$— dans lequel $R_7$ est un radical alkyle comprenant de 1 à 4 atomes de carbone ou alkoxy comprenant de 1 à 4 atomes de carbone;

$R_5$ et $R_6$, indépendamment l'un de l'autre, sont un atome d'hydrogène ou un radical alkyle comportant de 1 à 3 atomes de carbone;
étant entendu que lorsque X est CH et Z est

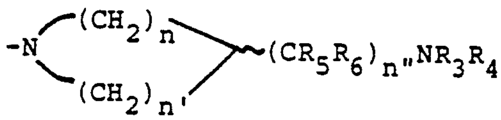

la somme de n + n' est différente de 4;
ainsi que les sels de base ou d'addition d'acides pharmaceutiquement acceptables en dérivant, ce procédé consistant à faire réagir un composé de formule:

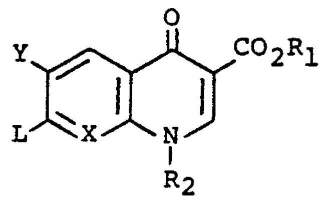

IV

dans laquelle L est un groupe sécant et les autres symboles sont tels que définis dans la revendication 1, avec une amine correspondant au groupe Z défini ci-dessus et, le cas échéant, à convertir le produit résultant en un sel de base ou d'addition d'acide pharmaceutiquement acceptable en dérivant par des méthodes connues.

2. Un procédé pour la préparation d'un composé de formule:

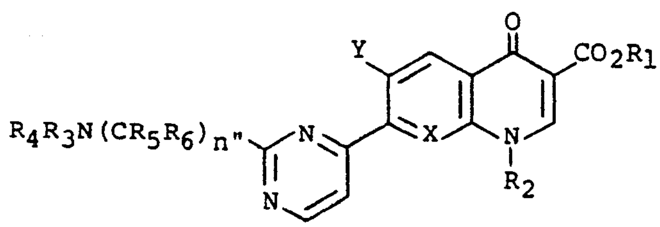

Ib

qui consiste à faire réagir un composé de formule:

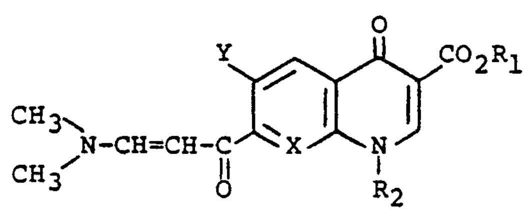

VII

avec une amidine de formule:

$$H_2N—C(=NH)—(CR_5R_6)_{n''}NR_3R_4$$

et, le cas échéant, à convertir le produit résultant en un sel de base ou d'addition d'acide pharmaceutiquement acceptable en dérivant par des méthodes connues, dans lesquels $R_1$ à $R_6$, X, Y et n'' sont tels que définsi ci-dessus.

3. Un procédé de préparation d'un composé de formule:

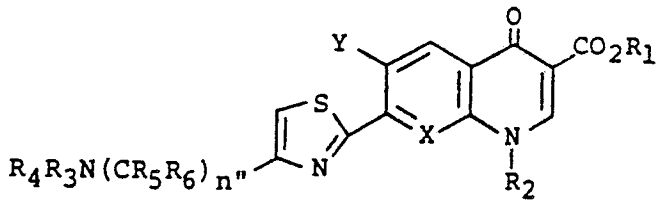

Ic

qui consiste:

a) à faire réagir un composé de formule:

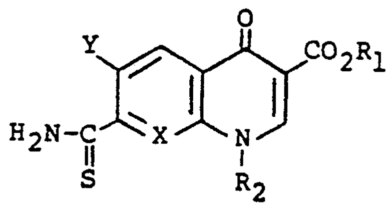

IX

avec un composé de formule:

$$Hal—CH_2\overset{\overset{O}{\|}}{C}(CR_5R_6)_{n''}-Hal$$

dans laquelle:

n'' est égal à 1 ou 2; et

Hal est un halogène;

b) à déplacer l'atome d'halogène du composé résultant de formule:

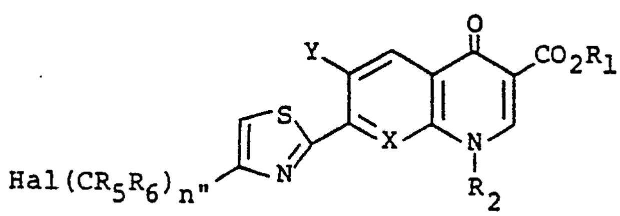

X

par un groupe amino de formule $R_3R_4N$— ou par un ion azide;

c) à réduire le groupe azide résultant en un composé dans lequel $R_3$ et $R_4$ sont des atomes d'hydrogène et, le cas échéant, à alkyler le groupe amino par un halogénure d'alkyle dans lequel le radical alkyle comprend de 1 à 3 atomes de carbone, et éventuellement à convertir le produit résultant en un sel de base ou d'addition d'acide phamaceutiquement acceptable en dérivant par des méthodes connues, dans lesquels $R_1$ à $R_6$, X, Y et n'' sont tels que définis dans la revendication 1.

4. Un procédé de préparation d'un composé de formule:

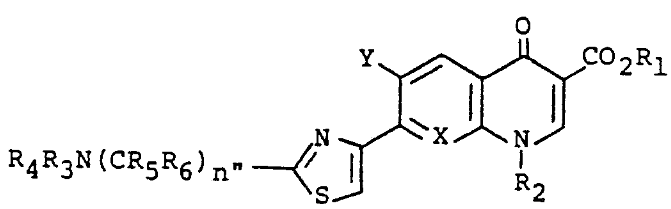

Id

qui consiste à faire réagir un composé de formule:

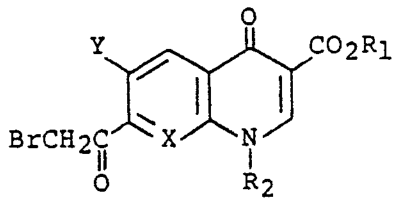

XI

avec un thioamide de formule:

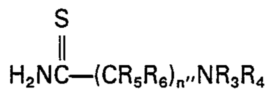

et, le cas échéant, à convertir le produit résultant en un sel de base ou d'addition d'acide pharmaceutiquement acceptable en dérivant par des méthodes connues, dans lesquels $R_1$ à $R_6$, X, Y et n'' sont tels que définis dans la revendication 1.

5. Un procédé ainsi que revendiqué dans l'une quelconque des revendications précédentes, dans lequel Y est un atome de fluor.

6. Un procédé ainsi que revendiqué dans l'une quelconque des revendications 1 à 5, dans lequel $R_2$ est un radical éthyle, vinyle ou 2-fluoroéthyle.

7. Un procédé ainsi que revendiqué dans l'une quelconque des revendications précédentes, dans lequel X est CH ou CF.

8. Un procédé ainsi que revendiqué dans l'une quelconque des revendications précédentes, dans lequel $R_1$ est un atome d'hydrogène ou qui consiste en un sel de base ou d'addition d'acide pharmaceutiquement acceptable en dérivant.

9. Un procédé ainsi que revendiqué dans la revendication 1, dans lequel Z est

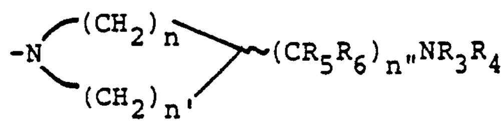

ou

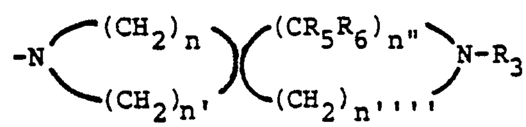

10. Un procédé ainsi que revendiqué dans la revendication 9, dans lequel Z est

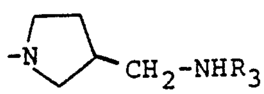

X étant C—F et $R_3$ est un atome d'hydrogène, un radical méthyle ou éthyle.

11. Un procédé ainsi que revendiqué dans la revendication 9, dans lequel Z est

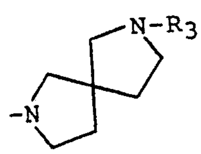

X est C—F et $R_3$ est un atome d'hydrogène, un radical méthyle ou éthyle.

12. Un procédé ainsi que revendiqué dans les revendications 10 ou 11, de production de:

acide 7-[3-(aminométhyl)-1-pyrrolidinyl]-1-éthyl-6,8-difluoro-1,4-dihydro-4-oxo-3-quinolinecarboxylique, ou

acide 7-[3-(aminométhyl)-1-pyrrolidinyl]-6,8-difluoro-1-(2-fluoroéthyl)-1,4-dihydro-4-oxo-3-quinolinecarboxylique, ou

acide 7-[3-(aminométhyl)-1-pyrrolidinyl]-6,8-difluoro-1-éthényl-1,4-dihydro-4-oxo-3-quinolinecarboxylique, ou

acide 1-éthyl-7-[3-[(éthylamino)méthyl]-1-pyrrolidinyl]-6,8-difluoro-1,4-dihydro-4-oxo-3-quinolinecarboxylique, ou

acide 7-[3-[(éthylamino)méthyl]-1-pyrrolidinyl]-6,8-difluoro-1-(2-fluoroéthyl)-1,4-dihydro-4-oxo-3-quinolinecarboxylique, ou

acide 7-[3-[(éthylamino)méthyl]-1-pyrrolidinyl]-6,8-difluoro-1-éthényl-1,4-dihydro-4-oxo-3-quinolinecarboxylique, ou

acide 1-éthyl-7-[3-[[(1-méthyléthyl)amino]méthyl]-1-pyrrolidinyl]-6,8-difluoro-1,4-dihydro-4-oxo-3-quinolinecarboxylique, ou

acide 1-éthyl-6,8-difluoro-1,4-dihydro-7-(7-méthyl-2,7-diazaspiro[4,4]non-2-yl)-4-oxo-3-quinolinecarboxylique.

13. Un procédé de préparation d'une composition pharmaceutique qui consiste à associer une quantité efficace du point de vue antibactérien d'un composé préparé par un procédé, ainsi que revendiqué dans l'une quelconque des revendications précédentes, à un support pharmaceutiquement acceptable.